# EUROPEAN PATENT APPLICATION

(11) **EP 1 900 824 A1**
(43) Date of publication of application: **19.03.2008**
(21) Application number: 06019274.7
(22) Date of filing: 14.09.2006
(51) Int. Cl.: C12Q 1/68

(54) **Gene expression signature for the prognosis, diagnosis and therapy of prostate cancer and uses thereof**

(71) Applicant: Deutsches Krebsforschungszentrum Stiftung des Öffentlichen Rechts, 69120 Heidelberg (DE)
(72) Inventor: The designation of the inventor has not yet been filed

(57) **Abstract**

Provided is a method for monitoring the presence and/or progression of prostate tumor in an individual, by measuring the expression level of marker genes out of a novel signature of 39 genes. The invention further discloses a data base and and array comprising said identified marker genes for prognosing the tumor progression in an individial suffering from prostate cancer.

SEQUENCE LISTING

<110>  Deutsches Krebsforschungszentrum

<120>  Gene expression signature for the improved prognosis, diagnosis
       and therapy of prostate cancer and uses thereof

<130>  P-710

<160>  39

<170>  PatentIn version 3.3

<210>  1
<211>  2208
<212>  DNA
<213>  Homo sapiens

<400>  1
aacgcacttg gcgcgcggcg cgggctgcag acggctgcga ggcgctgggc acaggtgtcc      60

tgatggcaaa tttcaagggc cacgcgcttc cagggagttt cttcctgatc attgggctgt     120

gttggtcagt gaagtacccg ctgaagtact ttagccacac gcggaagaac agcccactac     180

attactatca gcgtctcgag atcgtcgaag ccgcaattag gactttgttt tccgtcactg     240

ggatcctggc agagcagttt gttccggatg ggccccacct gcacctctac catgagaacc     300

actggataaa gttaatgaat tggcagcaca gcaccatgta cctattcttt gcagtctcag     360

gaattgttga catgctcacc tatctggtca gccacgttcc cttgggggtg gacagactgg     420

ttatggctgt ggcagtattc atggaaggtt tcctcttcta ctaccacgtc cacaaccggc     480

ctccgctgga ccagcacatc cactcactcc tgctgtatgc tctgttcgga gggtgtgtta     540

gtatctccct agaggtgatc ttccgggacc acattgtgct ggaacttttc cgaaccagtc     600

tcatcattct tcagggaacc tggttctggc agattgggtt tgtgctgttc ccaccttttg     660

gaacacccga atgggaccag aaggatgatg ccaacctcat gttcatcacc atgtgcttct     720

gctggcacta cctggctgcc ctcagcattg tggccgtcaa ctattctctt gtttactgcc     780

ttttgactcg gatgaagaga cacggaaggg gagaaatcat tggaattcag aagctgaatt     840

cagatgacac ttaccagacc gccctcttga gtggctcaga tgaggaatga gccgagatgc     900

ggagggcgca gatgtcccac tgcacagctg gaatgaatgg agttcatccc ctccacctga     960

atgcctgctg tggtctgatc ttaagggtct atatatttgc acctcctcat tcaacacagg    1020

gctggaggtt ctacaacagg aaatcaggcc tacagcatcc tgtgtatctt gcagttggga    1080

tttttaaaca tactataaag tctgtgttgg tatagtaccc ttcataagga aaaatgaagt    1140

aatgcctata agtagcaggc ctttgtgcct cagtgtcaag agaaatcaag agatgctaaa    1200

agctttacaa tggaagtggc ctcatggatg aatccggggt atgagcccag gagaacgtgc    1260

tgcttttggt aacttatccc tttttctctt aagaaagcag gtactttctt attagaaata    1320

tgttagaatg tgtaagcaaa cgacagtgcc tttagaatta caattctaac ttacatattt    1380

tttgaaagta aaataattca caagctttgg tattttaaaa ttattgttaa acatatcata    1440

actaatcata ccagggtact gcaataccac tgtttataag tgacaaaatt aggccaaagg    1500

tgattttttt ttaaatcagg aagctggtta ctggctctac tgagagttgg agccctgatg 1560

ttctgattct tcaaagtcac cctaaaagaa gatctgacag gaaagctgta taatgagata 1620

gaaaaacgtc aggtatggaa ggctttcagt tttaatatgg ctgaaagcaa aggataacga 1680

attcagaatt agtaatgtaa aatcttgata ccctaatctt gcttctggat ctgttctttt 1740

tttaaaaaaa cttccttcac cgcgcctata atcctagcac tttgggaggc cgaggcaggc 1800

agatcacggg gtcaggagat caagaccatc ctggctaaca tggtgaaacc ccgtctctac 1860

tgaaaataca aaaaattagc cgggtgtggt ggcgggcgcc tgtagttcca gctactcggg 1920

aggctgaggc aagagaatgg catgaacccg gtaggggagc ttgcagtgag cccagatcat 1980

gccactgtac tccagcctag gtgacagagc aagactctgt ctcaaaaaca agcaaacaga 2040

cttccttcaa caaatattta ttaaatatcc actttgcaac agcactgaaa tggctgtaag 2100

gactcctgag atatgtgtcc agcaaggagt ttacagtcaa acaggagaga catgcctgta 2160

gttacatcca gtgtgatggg tgctgagagg caagtacaaa ccacgatg 2208

<210> 2
<211> 1098
<212> DNA
<213> Homo sapiens

<400> 2
ggctggcggc cggcgggaga ggcggccggc ctggactggc ccgagaggga tcccggttcc 60

cagaacagac ctaggaggcg gcctcgaggg cggacggcag ggagggccag catgccccga 120

ctgctgcacc ccgccctgcc gctgctcctg ggcgccacgc tgaccttccg ggcgctccgg 180

cgcgcgctct gtcgcctgcc cctacccgtg cacgtgcgcg ccgaccccct gcgcacctgg 240

cgctggcaca acctgctcgt ctccttcgct cactccattg tgtcggggat ctgggcactg 300

ctgtgtgtat ggcagactcc tgacatgtta gtggagattg agacggcgtg gtcactttct 360

ggctatttgc tcgtttgctt ctctgcgggg tatttcatcc acgatacggt ggacatcgtg 420

gctagcggac agacgcgagc ctcttgggaa taccttgtcc atcacgtcat ggccatgggt 480

gccttcttct ccggcatctt ttggagcagc tttgtcggtg ggggtgtctt aacactactg 540

gtggaagtca gcaacatctt cctcaccatt cgcatgatga tgaaaatcag taatgcccag 600

gatcatctcc tctaccgggt taacaagtat gtgaacctgg tcatgtactt tctcttccgc 660

ctggcccctc aggcctacct cacccatttc ttcttgcgtt atgtgaacca gaggaccctg 720

ggcaccttcc tgctgggtat cctgctcatg ctggacgtga tgatcataat ctacttttcc 780

cgcctcctcc gctctgactt ctgccctgag catgtcccca agaagcaaca caaagacaag 840

ttcttgactg agtgaggggc acagagcctg ggacaacaaa aacggacaag gccagaaaca 900

gcttcatatg gacactggga cttagcccca agcctgggtg tcctctgagg ccagcctctc 960

caccttctga gcctgcgccc acactattga aaacactaat gaaagtaaaa aaaaaaaaaa 1020

aaaaaaaaaa aaaaaaaaaa aaaaaaaaaa aaaaaaaaaa aaaaaaaaaa aaaaaaaaaa 1080

aaaaaaaaaa aaaaaaaa 1098

<210> 3
<211> 1912
<212> DNA
<213> Homo sapiens

<400> 3
aggggctggg cggggctcgg gctcctgctc cggctcagct gcggcggccg caggttccaa 60

agcgggtccg agccgccgcc gcgcgcgcgc cgcgcactgc agccccaggc cccggccccc 120

cacccacgtc tgcgttgctg ccccgcctgg gccaggcccc aaaggcaagg acaaagcagc 180

tgtcagggaa cctccgccgg agtcgaattt acgtgcagct gccggcaacc acaggttcca 240

agatggtttg cgggggcttc gcgtgttcca agaactgcct gtgcgccctc aacctgcttt 300

acaccttggt tagtctgctg ctaattggaa ttgctgcgtg gggcattggc ttcgggctga 360

tttccagtct ccgagtggtc ggcgtggtca ttgcagtggg catcttcttg ttcctgattg 420

ctttagtggg tctgattgga gctgtaaaac atcatcaggt gttgctattt ttttatatga 480

ttattctgtt acttgtattt attgttcagt tttctgtatc ttgcgcttgt ttagccctga 540

accaggagca acagggtcag cttctggagg ttggttggaa caatacggca agtgctcgaa 600

atgacatcca gagaaatcta aactgctgtg ggttccgaag tgttaaccca aatgacacct 660

gtctggctag ctgtgttaaa agtgaccact cgtgctcgcc atgtgctcca atcataggag 720

aatatgctgg agaggttttg agatttgttg gtggcattgg cctgttcttc agttttacag 780

agatcctggg tgtttggctg acctacagat acaggaacca gaaagacccc cgcgcgaatc 840

ctagtgcatt cctttgatga gaaaacaagg aagatttcct ttcgtattat gatcttgttc 900

actttctgta attttctgtt aagctccatt tgccagttta aggaaggaaa cactatctgg 960

aaaagtacct tattgatagt ggaattatat atttttactc tatgtttctc tacatgtttt 1020

tttctttccg ttgctgaaaa atatttgaaa cttgtggtct ctgaagctcg gtggcacctg 1080

gaatttactg tattcattgt cgggcactgt ccactgtggc ctttcttagc atttttacct 1140

gcagaaaaac tttgtatggt accactgtgt tggttatatg gtgaatctga acgtacatct 1200

cactggtata attatatgta gcactgtgct gtgtagatag ttcctactgg aaaaagagtg 1260

gaaatttatt aaaatcagaa agtatgagat cctgttatgt taagggaaat ccaaattccc 1320

aatttttttt ggtcttttta ggaaagattg ttgtggtaaa aagtgttagt ataaaaatga 1380

taatttactt gtagtctttt atgattacac caatgtattc tagaaatagt tatgtcttag 1440

gaaattgtgg tttaattttt gacttttaca ggtaagtgca aaggagaagt ggtttcatga 1500

aatgttctaa tgtataataa catttacctt cagcctccat cagaatggaa cgagttttga 1560

gtaatcagga agtatatcta tatgatcttg atattgtttt ataataattt gaagtctaaa 1620

agactgcatt tttaaacaag ttagtattaa tgcgttggcc cacgtagcaa aaagatattt 1680

gattatctta aaaattgtta aataccgttt tcatgaaatt tctcagtatt gtaacagcaa 1740

cttgtcaaac ctaagcatat ttgaatatga tctcccataa tttgaaattg aaatcgtatt 1800

gtgtggctct gtatattctg ttaaaaaatt aaaggacaga aacctttctt tgtgtatgca 1860

tgtttgaatt aaaagaaagt aatggaagaa ttgatcgatg aaaaaaaaaa aa 1912

<210> 4
<211> 5265
<212> DNA
<213> Homo sapiens

<400> 4
ggcgccggct gcgtcccggc ctcctcctcc tcttcctccg ccctccttgg ctgggaaagt 60

gggaggcggc ggcggcggcg tggtgtgtgt gcgcgcgtga ggggggcagc agagaggagt 120

ctcccggtgc cgccgcggcg tcagagacac tgcgagcggc gagcgcggtg gggccgcatc 180

tgcatcagcc gccgcagccg ctgcggggcc gcgaacaaag aggaggagcc gaggcgcgag 240

agcaaagtct gaaatggatg ttacatgagt cattttaagg gatgcacaca actatgaaca 300

tttctgaaga ttttttctca gtaaagtaga taaagatgga tgaatcagcc ttgttggatc 360

ttttggagtg tccggtgtgt ctagagcgcc ttgatgcttc tgcgaaggtc ttgccttgcc 420

agcatacgtt ttgcaagcga tgtttgctgg ggatcgtagg ttctcgaaat gaactcagat 480

gtcccgagtg caggactctt gttggctcgg gtgtcgagga gcttcccagt aacatcttgc 540

tggtcagact tctggatggc atcaaacaga ggccttggaa acctggtcct ggtgggggaa 600

gtgggaccaa ctgcacaaat gcattaaggt ctcagagcag cactgtggct aattgtagct 660

caaaagatct gcagagctcc cagggcggac agcagcctcg ggtgcaatcc tggagccccc 720

cagtgagggg tatacctcag ttaccatgtg ccaaagcatt atacaactat gaaggaaaag 780

agcctggaga ccttaaattc agcaaaggtg acatcatcat tttgcgaaga caagtggatg 840

aaaattggta ccatggggaa gtcaatggaa tccatggctt tttccccacc aactttgtgc 900

agattattaa accgttacct cagcccccac ctcagtgcaa agcactttat gactttgaag 960

tgaaagacaa ggaagcagac aaagattgcc ttccatttgc aaaggatgat gttctgactg 1020

tgatccgaag agtggatgaa aactgggctg aaggaatgct ggcagacaaa ataggaatat 1080

ttccaatttc atatgttgag tttaactcgg ctgctaagca gctgatagaa tgggataagc 1140

ctcctgtgcc aggagttgat gctggagaat gttcctcggc agcagcccag agcagcactg 1200

ccccaaagca ctccgacacc aagaagaaca ccaaaaagcg gcactccttc acttccctca 1260

ctatggccaa caagtcctcc caggcatccc agaaccgcca ctccatggag atcagccccc 1320

ctgtcctcat cagctccagc aaccccactg ctgctgcacg gatcagcgag ctgtctgggc 1380

tctcctgcag tgccccttct caggttcata taagtaccac cgggttaatt gtgaccccgc 1440

ccccaagcag cccagtgaca actggcccct cgtttacttt cccatcagat gttccctacc 1500

aagctgccct tggaactttg aatcctcctc ttccaccacc ccctctcctg gctgccactg 1560

tccttgcctc cacaccacca ggcgccaccg ccgctgctgc tgctgctgga atgggaccga 1620

ggcccatggc aggatccact gaccagattg cacatttacg gccgcagact cgccccagtg 1680

tgtatgttgc tatatatcca tacactcctc ggaaagagga tgaactagag ctgagaaaag 1740
gggagatgtt tttagtgttt gagcgctgcc aggatggctg gttcaaaggg acatccatgc 1800
ataccagcaa gataggggtt ttccctggca attatgtggc accagtcaca agggcggtga 1860
caaatgcttc ccaagctaaa gtccctatgt ctacagctgg ccagacaagt cggggagtga 1920
ccatggtcag tccttccacg gcaggagggc ctgcccagaa gctccaggga aatggcgtgg 1980
ctgggagtcc cagtgttgtc cccgcagctg tggtatcagc agctcacatc cagacaagtc 2040
ctcaggctaa ggtcttgttg cacatgacgg ggcaaatgac agtcaaccag gcccgcaatg 2100
ctgtgaggac agttgcagcg cacaaccagg aacgccccac ggcagcagtg acacccatcc 2160
aggtacagaa tgccgccggc ctcagccctg catctgtggg cctgtcccat cactcgctgg 2220
cctccccaca acctgcgcct ctgatgccag gctcagccac gcacactgct gccatcagta 2280
tcagtcgagc cagtgcccct ctggcctgtg cagcagctgc tccactgact tccccaagca 2340
tcaccagtgc ttctctggag gctgagccca gtggccggat agtgaccgtt ctccctggac 2400
tccccacatc tcctgacagt gcttcatcag cttgtgggaa cagttcagca accaaaccag 2460
acaaggatag caaaaaagaa aaaaagggtt tgttgaagtt gctttctggc gcctccacta 2520
aacggaagcc ccgcgtgtct cctccagcat cgcccaccct agaagtggag ctgggcagtg 2580
cagagcttcc tctccaggga gcggtggggc ccgaactgcc accaggaggt ggccatggca 2640
gggcaggctc ctgccctgtg gacggggacg gaccggtcac gactgcagtg gcaggagcag 2700
ccctggccca ggatgctttt cataggaagg caagttccct ggactccgca gttcccatcg 2760
ctccacctcc tcgccaggcc tgttcctccc tgggtcctgt cttgaatgag tctagacctg 2820
tcgtttgtga aaggcacagg gtggtggttt cctatcctcc tcagagtgag gcagaacttg 2880
aacttaaaga aggagatatt gtgtttgttc ataaaaaacg agaggatggc tggttcaaag 2940
gcacattaca acgtaatggg aaaactggcc ttttcccagg aagctttgtg gaaaacatat 3000
gaggagactg acactgaaga agcttaaaat cacttcacac aacaaagtag cacaaagcag 3060
tttaacagaa agagcacatt tgtggacttc cagatggtca ggagatgagc aaaggattgg 3120
tatgtgactc tgatgcccca gcacagttac cccagcgagc agaatgaaga agatgtttgt 3180
gtgggttttg ttagtctgga ttcggatgta taaggtgtgc cttgtactgt ctgatttact 3240
acacagagaa actttttttt ttttttaaga tatatgacta aaatggacaa ttgtttacaa 3300
ggcttaacta atttatttgc ttttttaaac ttgaactttt cgtataatag atacgttctt 3360
tggattatga ttttaagaaa ttattaattt atgaaatgat aggtaaggag aagctggatt 3420
atctcctgtt gagagcaaga gattcgtttt gacatagagt gaatgcattt tcccctctcc 3480
tcctccctgc taccattata ttttggggtt atgttttgct tctttaagat agaaatccca 3540
gttctctaat ttggttttct tctttgggaa accaaacata caaatgaatc agtatcaatt 3600
agggcctggg gtagagagac agaaacttga gagaagagaa gttagtgatt ccctctcttt 3660
ctagtttggt aggaatcacc ctgaagacct agtcctcaat ttaattgtgt gggtttttaa 3720

ttttcctaga atgaagtgac tgaaacaatg agaaagaata cagcacaacc cttgaacaaa 3780

atgtatttag aaatatattt agttttatag cagaagcagc tcaattgttt ggttggaaag 3840

taggggaaat tgaagttgta gtcactgtct gagaatggct atgaagcgtc atttcacatt 3900

ttaccccaac tgacctgcat gcccaggaca caagtaaaac atttgtgaga tagtggtggt 3960

aagtgatgca ctcgtgttaa gtcaaaggct ataagaaaca ctgtgaaaag ttcatattca 4020

tccattgtga ttctttcccc acgtcttgca tgtattactg gattcccaca gtaatataga 4080

ctgtgcatgg tgtgtatatt tcattgcgat ttcctgttaa gatgagtttg tactcagaat 4140

tgaccaattc aggaggtgta aaaataaaca gtgttctctt ctctacccca aagccactac 4200

tgaccaaggt ctcttcagtg cactcgctcc ctctctggct aaggcatgca ttagccacta 4260

cacaagtcat tagtgaaagt ggtcttttat gtcctcccag cagacagaca tcaaggatga 4320

gttaaccagg agactactcc tgtgactgtg gagctctgga aggcttggtg ggagtgaatt 4380

tgcccacacc ttacaattgt ggcaggatcc agaagagcct gtctttttat atccattcct 4440

tgatgtcatt ggcctctccc accgatttca ttacggtgcc acgcagtcat ggatctgggt 4500

agtccggaaa acaaaaggag ggaagacagc ctggtaatga ataagatcct taccacagtt 4560

ttctcatggg aaatacataa taaacccttt catctttttt tttttccttt aagaattaaa 4620

actgggaaat agaaacatga actgaaaagt cttgcaatga caagaggttt catggtctta 4680

aaaagatact ttatgtggtt gaagatgaaa tcattcctaa attaaccttt tttttaaaaa 4740

aaaacaatgt atattatgtt cctgtgtgtt gaatttaaaa aaaaaatact ttacttggat 4800

attcatgtaa tatataaagg tttggtgaaa tgaactttag ttaggaaaaa gctggcatca 4860

gctttcatct gtgtaagttg acaccaatgt gtcataatat tctttatttt gggaaattag 4920

tgtattttat aaaaatttta aaaagaaaaa agactactac aggttaagat aattttttta 4980

cctgtctttt ctccatattt taagctatgt gattgaagta cctctgttca tagtttcctg 5040

gtataaagtt ggttaaaatt tcatctgtta atagatcatt aggtaatata atgtatgggt 5100

tttctattgg ttttttgcag acagtagagg gagattttgt aacaagggct tgttacacag 5160

tgatatggta atgataaaat tgcaatttat cactcctttt catgttaata atttgaggac 5220

tggataaaag gtttcaagat taaaatttga tgttcaaacc tttgt 5265

<210> 5
<211> 3270
<212> DNA
<213> Homo sapiens

<400> 5
ggccgcgggc accagagtgc cgagcccagg acgcccccgg cccaggccct tggggtggac 60

aagtccttca cttctcgccg gagtgtgtgg aggagcgatg ggcagaacca gcacttccct 120

caggcactag acctgtcacg agtgaactta gttccctcct atactccttc actctaccct 180

aagaacacag atctatttga gatgattgag aagatgcagg gaagcaggat ggatgaacaa 240

cgctgctcct tcccgccgcc cctcaaaaca gaggaggact acattccata cccgagcgtg 300

cacgaggtct tggggcgaga aggacccttc cccctcatcc tgctgcccca gtttgggggc 360
tactggattg agggcaccaa ccacgaaatc accagcatcc ccgagacaga gccactgcag 420
tcgcccacaa ccaaggtgaa gctcgagtgc aaccccacag cccgcatcta ccggaagcac 480
tttctcggca aggagcattt caattactac tcactggaca ctgccctcgg ccaccttgtc 540
ttctcactca agtacgatgt catcggggac caagagcacc tgcggctgct gctcaggacc 600
aagtgccgga cataccatga tgtcatcccc atctcctgcc tcaccgagtt ccctaatgtt 660
gtccagatgg caaagttggt gtgtgaagac gtcaatgtgg atcggttcta tcctgtgctc 720
taccccaagg cttcccggct catcgtcacc tttgacgagc atgtcatcag caataacttc 780
aagtttggcg tcatttatca gaagcttggg cagacctccg aggaagaact cttcagcacc 840
aatgaggaaa gtcccgcttt cgtggagttc cttgaatttc ttggccagaa ggtcaaactg 900
caggacttta aggggttccg aggaggcctg gacgtgaccc acgggcagac ggggaccgaa 960
tctgtgtact gcaacttccg caacaaggag atcatgtttc acgtgtccac caagctgcca 1020
tacacggaag gggacgccca gcagttgcag cggaagcggc acatcgggaa cgacatcgtg 1080
gctgtggtct tccaggatga aaacactcct ttcgtgcccg acatgatcgc gtccaacttc 1140
ctgcatgcct acgtcgtggt gcaggctgag ggcgggggcc ctgatggccc cctctacaag 1200
gtctctgtca ctgcaagaga tgatgtgccc ttctttggac ccccctcccc ggaccccgct 1260
gtgttcagga aggggcctga gttccaggaa tttttgctga caaagctgat caatgctgaa 1320
tatgcctgct acaaggcaga gaagtttgcc aaactggagg agcggacgcg ggccgccctc 1380
ctggagacgc tctatgagga actacacatc cacagccagt ccatgatggg cttgggcggc 1440
gacgaggaca agatggagaa tggcagtggg ggcggcggct tctttgagtc tttcaagcgg 1500
gtcatccgga gccgcagcca gtccatggat gccatggggc tgagcaacaa gaagcccaac 1560
accgtgtcca ccagccacag cgggagcttc gcgcccaaca accccgacct ggccaaggcg 1620
gctggaatat cactgattgt ccctgggaag agccccacga ggaagaagtc gggcccgttc 1680
ggctcccgcc gcagcagcgc cattggcatc gagaacatac aggaggtgca ggagaagagg 1740
gagagccctc cggctggtca gaagacccca gacagcgggc acgtctcaca ggagcccaag 1800
tcggagaact catccactca gagctcccca gagatgccca cgaccaagaa cagagcggag 1860
accgcagcgc agagagcaga ggcgctcaag gacttctccc gctcctcgtc cagtgccagc 1920
agcttcgcca gcgtggtgga ggagacggag ggtgtggacg gagaggacac aggcctggag 1980
agcgtgtcat cctcaggaac accccacaag cgggactcct tcatctatag cacgtggctg 2040
gaggacagtg tcagcaccac tagtgggggc agctccccag gcccctctcg atcaccccac 2100
ccagacgccg gcaagttggg ggaccctgcg tgtcccgaga tcaagatcca gctggaagca 2160
tctgagcagc acatgcccca gctgggctgt tagccgggcc accccctctg aaggtgaaac 2220
tgagcagatg aggccacaga agcacaaggg gaaggtgccg tgtcaagccc aggcagacga 2280
gacctctgcc ctgaagacca acaccagccc gtgggctgcc ccctgcctcc ccaccctccc 2340

catggcccac ccatctgggc tgtctctgca gggcagagcc gtccagacct gggatcaggg 2400

aagctgctgg catcgtcccc acccccagcc tgggggtctg cgctggggca gggattgctc 2460

agtggaagca ggactggggg tctggcttgc cccctccctg ggcctccatc acccctgagc 2520

atccctctgg actcagaggg aacaaggtgg gagagagagt ttgagacagc tccgtgtgga 2580

gagcttagcc cctggaggca gcacaaggag gatgtgatat gtgggggagt gagcactggg 2640

ttgggagccg ggtcctggtt tccaatttgg gttctgctgt gtgactctgg gcaagtcact 2700

ctccctctct gggcatgtct gctacaaatg gacaagatta tttcagaggt cactgaagac 2760

tgtgattaca tgcacctgcc ttagaaggta ggattttctt cccagggacc tcctatcacc 2820

ctaccctgct tcttgaggtc cctggagccc caggtgggct gaggggcagg gagccggctg 2880

tgcccagtat gcctcctgga ccctccagtt ctgccacagg tctgccgatg ccctgtccac 2940

tgcctacaca tgacagacaa gtaaccccct catgggggat ggggacctac ctggctcctc 3000

agccagcacc cagcttaacc cctgccatcc catgctgggc cctccaggcc aagagtctca 3060

gctggccgag agtccaggcc ttgcctcccc cgaccgccat ggagggggca gcccggcaca 3120

gctgctggga gcccttgtgt gtctggtcac actttttagg cgtcacgcca aaggccagcc 3180

tcctggcccc aatacccatt ttggaagccc ctgtggccgt gtggatgtcg gtaacagttg 3240

tataaaataa attctattta tcgctattgt 3270

<210> 6
<211> 1750
<212> DNA
<213> Homo sapiens

<400> 6
tggtaactca ggcgccgggc gcactgtccg tagctgctgg ttttccacgc tggttttagc 60

tcccggcgtc tgcaaaatga agattgagga ggtgaagagc actacgaaga cgcagcgcat 120

cgcctcccac agccacgtga aagggctggg gctggacgag agcggcttgg ccaagcaggc 180

ggcctcaggg cttgtgggcc aggagaacgc gcgagaggca tgtggcgtca tagtagaatt 240

aatcaaaagc aagaaaatgg ctggaagagc tgtcttgttg gcaggacctc ctggaactgg 300

caagacagct ctggctctgg ctattgctca ggagctgggt agtaaggtcc ccttctgccc 360

aatggtgggg agtgaagttt actcaactga gatcaagaag acagaggtgc tgatggagaa 420

cttccgcagg gccattgggc tgcgaataaa ggagaccaag gaagtttatg aaggtgaagt 480

cacagagcta actccgtgtg agacagagaa tcccatggga ggatatggca aaaccattag 540

ccatgtgatc ataggactca aaacagccaa aggaaccaaa cagttgaaac tggaccccag 600

catttttgaa agtttgcaga aagagcgagt agaagctgga gatgtgattt acattgaagc 660

caacagtggg gccgtgaaga ggcagggcag gtgtgatacc tatgccacag aattcgacct 720

tgaagctgaa gagtatgtcc ccttgccaaa aggggatgtg cacaaaaaga aagaaatcat 780

ccaagatgtg accttgcatg acttggatgt ggctaatgcg cggccccagg ggggacaaga 840

tatcctgtcc atgatgggcc agctaatgaa gccaaagaag acagaaatca cagacaaact 900

tcgaggggag attaataagg tggtgaacaa gtacatcgac cagggcattg ctgagctggt 960

cccgggtgtg ctgtttgttg atgaggtcca catgctggac attgagtgct tcacctacct 1020

gcaccgcgcc ctggagtctt ctatcgctcc catcgtcatc tttgcatcca accgaggcaa 1080

ctgtgtcatc agaggcactg aggacatcac atcccctcac ggcatccctc ttgaccttct 1140

ggaccgagtg atgataatcc ggaccatgct gtatactcca caggaaatga aacagatcat 1200

taaaatccgt gcccagacgg aaggaatcaa catcagtgag gaggcactga accacctggg 1260

ggagattggc accaagacca cactgaggta ctcagtgcag ctgctgaccc cggccaactt 1320

gcttgctaaa atcaacggga aggacagcat tgagaaagag catgtcgaag agatcagtga 1380

acttttctat gatgccaagt cctccgccaa aatcctggct gaccagcagg ataagtacat 1440

gaagtgagat ggctgaggtt ttcagcagca agagactccc caggtgtgcc tggcctgggt 1500

ccagcctgtg ggcgcttgcc cctgggcttg gggctgccgt ccccactcag gcgtgggctg 1560

cagcgctgtc agttcagtgt ggaaagcatt tctttttaag ttatcgtaac tgttcctgtg 1620

gttgctttga aagaaccctt ccttacctgg tgtgttttct ataaatcttc ataggttatt 1680

ttgattcttc tctctctctc tctaagtttt ttaaaaataa acttttcaga acagttaaaa 1740

aaaaaaaaaa 1750

<210> 7
<211> 570
<212> DNA
<213> Homo sapiens

<400> 7
cacctgcacc ccgcccgggc atagcaccat gcctgcttgt cgcctaggcc cgctagccgc 60

cgccctcctc ctcagcctgc tgctgttcgg cttcacccta gtctcaggca caggagcaga 120

gaagactggc gtgtgccccg agctccaggc tgaccagaac tgcacgcaag agtgcgtctc 180

ggacagcgaa tgcgccgaca acctcaagtg ctgcagcgcg ggctgtgcca ccttctgctc 240

tctgcccaat gataaggagg gttcctgccc ccaggtgaac attaactttc cccagctcgg 300

cctctgtcgg gaccagtgcc aggtggacag ccagtgtcct ggccagatga aatgctgccg 360

caatggctgt gggaaggtgt cctgtgtcac tcccaatttc tgagctccag ccaccaccag 420

gctgagcagt gaggagagaa agtttctgcc tggccctgca tctggttcca gcccacctgc 480

cctccccttt ttcgggactc tgtattccct cttgggctga ccacagcttc tccctttccc 540

aaccaataaa gtaaccactt tcagcaaaaa 570

<210> 8
<211> 2398
<212> DNA
<213> Homo sapiens

<400> 8
cggagggggc tcagtccgca gccgccgccg ccaccgccgc gcctcggcct cggtgcaggc 60

agcggccgcc gccgccgaga cagctgcgcg ggcgagcatc cccacgcagc accttggaag 120
ttgttttcaa ccatatccag cctttgccga atacatccta tctgccacac atccagcgtg 180
aggtccctcc agctacaagg tgggcaccat ggcggagaag tttgactgcc actactgcag 240
ggatcccttg caggggaaga agtatgtgca aaaggatggc caccactgct gcctgaaatg 300
ctttgacaag ttctgtgcca acacctgtgt ggaatgccgc aagcccatcg gtgcggactc 360
caaggaggtg cactataaga accgcttctg gcatgacacc tgcttccgct gtgccaagtg 420
ccttcacccc ttggccaatg agacctttgt ggccaaggac aacaagatcc tgtgcaacaa 480
gtgcaccact cgggaggact cccccaagtg caaggggtgc ttcaaggcca ttgtggcagg 540
agatcaaaac gtggagtaca aggggaccgt ctggcacaaa gactgcttca cctgtagtaa 600
ctgcaagcaa gtcatcggga ctggaagctt cttccctaaa ggggaggact tctactgcgt 660
gacttgccat gagaccaagt ttgccaagca ttgcgtgaag tgcaacaagg ccatcacatc 720
tggaggaatc acttaccagg atcagccctg gcatgccgat tgctttgtgt gtgttacctg 780
ctctaagaag ctggctgggc agcgtttcac cgctgtggag gaccagtatt actgcgtgga 840
ttgctacaag aactttgtgg ccaagaagtg tgctggatgc aagaacccca tcactgggtt 900
tggtaaaggc tccagtgtgg tggcctatga aggacaatcc tggcacgact actgcttcca 960
ctgcaaaaaa tgctccgtga atctggccaa caagcgcttt gttttccacc aggagcaagt 1020
gtattgtccc gactgtgcca aaaagctgta aactgacagg ggctcctgtc ctgtaaaatg 1080
gcatttgaat ctcgttcttt gtgtccttac tttctgccct ataccatcaa taggggaaga 1140
gtggtccttc ccttctttaa agttctcctt ccgtcttttc tcccatttta cagtattact 1200
caaataaggg cacacagtga tcatattagc atttagcaaa aagcaaccct gcagcaaagt 1260
gaatttctgt ccggctgcaa tttaaaaatg aaaacttagg tagattgact cttctgcatg 1320
tttctcatag agcagaaaag tgctaatcat ttagccactt agtgatgtaa gcaagaagca 1380
taggagataa aacccccact gagatgcctc tcatgcctca gctgggaccc accgtgtaga 1440
cacacgacat gcaagagttg cagcggctgc tccaactcac tgctcaccct cttctgtgag 1500
caggaaaaga accctactga catgcatggt ttaacttcct catcagaact ctgcccttcc 1560
ttctgttctt ttgtgctttc aaataactaa cacgaacttc cagaaaatta acatttgaac 1620
ttagctgtaa ttctaaactg acctttcccc gtactaacgt ttggtttccc cgtgtggcat 1680
gttttctgag cgttcctact ttaaagcatg gaacatgcag gtgatttggg aagtgtagaa 1740
agacctgaga aaacgagcct gtttcagagg aacatcgtca caacgaatac ttctggaagc 1800
ttaacaaaac taaccctgct gtccttttta ttgtttttaa ttaatatttt tgttttaatt 1860
gatagcaaaa tagtttatgg gtttggaaac ttgcatgaaa atattttagc cccctcagat 1920
gttcctgcag tgctgaaatt catcctacgg aagtaaccgc aaaactctag agggggagtt 1980
gagcaggcgc cagggctgtc atcaacatgg atatgacatt tcacaacagt gactagttga 2040
atcccttgta acgtagtagt tgtctgctct ttgtccatgt gttaatgagg actgcaaagt 2100

cccttctgtt gtgattccta ggacttttcc tcaagaggaa atctggattt ccacctaccg 2160

cttacctgaa atgcaggatc acctacttac tgtattctac attattatat gacatagtat 2220

aatgagacaa tatcaaaagt aaacatgtaa tgacaataca tactaacatt cttgtaggag 2280

tggttagaga agctgatgcc tcatttctac attctgtcat tagctattat catctaacgt 2340

ttcagtgtat ccttacagaa ataaagcagc atatgaaaaa aaaaaaaaaa aaaaaaaa 2398

<210> 9
<211> 3721
<212> DNA
<213> Homo sapiens

<400> 9
tcccgctccc cactcccggg atgtgtctcc gccgtacgac gggctatggc caccacgact 60

tccgggttcc gtcatttcgt tctcccgccg ccgacccgcg ccgccaaact gaggctcttc 120

aataagccag gcagcagcca acctgccaac acctactgac actcactcat ctcccagaga 180

gagaaagaga gcgagagaga gcgagcgcga gagagcgagc gcgagtgaga gcgagcgagc 240

gagcgagaaa gagagagagg gagagacaaa atacctacca ggaaaggggg ggaggaagtc 300

caatttttgc aaactattca tttttttttc ttgatttttc tcactgcttt ctttgaacaa 360

tactttaaag agagaggatc gtattataga taccgcgggg gcaaagctaa aaaagggggg 420

aggggggagg aaaaaattca agaagcagaa acccctcgcg gagttttact ggaagaaaaa 480

aacgggtctg aaagattcct cctcttcatc atcatcaacc atcattcatt cactaccttg 540

acattccggg ctttgattga cagctggagt ggcaaaaagc catgaaacac gacagttcgg 600

ttacatgtgg gctgctgacg ggccgctcgt aaccttcagt tcgggggctt gacaattttt 660

ttcttctttt tcttcttttt cttttctttc tttttttttc caactgaggg gaagagaaga 720

gaaagagggg gaaaggagga ccgaagagga ggaggagggg aggggggagg aggaggaggt 780

ggaggaggag gaggaagatc aggaggagga ggaagaagag gaaaaaagag aaaaagaaga 840

aatatcacag aaaaaaaaat tcttcgttgt ctagactggg ctttttttcc cccctaaaaa 900

atagcatatt ggagaattgg gagaagtctc tttggtttgg aaaaaaaaaa aaggaatctt 960

cagcctagat cactttctta tccggactgg gatattaaat atacgacaca tccaggagtt 1020

tattggagcg cagactgatg gcgcaaaggt acgatgagct gccccattac ggcgggatgg 1080

acggagtagg ggttcccgct tccatgtacg gagaccctca cgcgccgcgg ccgatccccc 1140

cggttcacca cctgaaccac gggccgccgc tccacgccac acagcactac ggcgcgcacg 1200

ccccgcaccc caatgtcatg ccggccagta tgggatccgc tgtcaacgac gccttgaagc 1260

gggacaagga cgcgatctat gggcacccgt tgtttcctct gttagctctg gtctttgaga 1320

agtgcgagct ggcgacctgc actccccggg aacctggagt ggctggcgga gacgtctgct 1380

cctccgactc cttcaacgag gacatcgcgg tcttcgccaa gcaggttcgc gccgaaaagc 1440

cacttttttc ctcaaatcca gagctggaca atttgatgat acaagcaata caagtactaa 1500

ggtttcatct tttggagtta gaaaaggtcc acgaactgtg cgataacttc tgccaccgat 1560

acattagctg tttgaagggg aaaatgccca tcgacctcgt cattgatgaa agagacggca 1620
gctccaagtc agatcatgaa gaactttcag gctcctccac aaatctcgct gaccataacc 1680
cttcttcttg gcgagaccac gatgatgcaa cctcaaccca ctcagcaggc accccagggc 1740
cctccagtgg gggccatgct tcccagagcg gagacaacag cagtgagcaa ggggatggtt 1800
tagacaacag tgtagcttca cctggtacag gtgacgatga tgatccggat aaggacaaaa 1860
aacgccagaa gaaaagaggc attttcccca aagtagcaac aaatatcatg agagcatggc 1920
tcttccagca tctcacacat ccgtaccctt ccgaagagca gaagaaacag ttagcgcaag 1980
acacaggact tacaattctc caagtaaaca actggtttat taatgccaga agaagaatag 2040
tacagcccat gattgaccag tcaaatcgag caggttttct tcttgatcct tcagtgagcc 2100
aaggagcagc atatagtcca gagggtcagc ccatggggag ctttgtgttg gatggtcagc 2160
aacacatggg gatccggcct gcaggaccta tgagtggaat gggcatgaat atgggcatgg 2220
atgggcaatg gcactacatg taaccttcat catgtaaagc aatcgcaaag caagggggaa 2280
gtttgcagag catgccaggg gactacgttt ctcagggtgg tcctatggga atgagtatgg 2340
cacagccaag ttacactcct ccccagatga ccccacaccc tactcaatta agacatggac 2400
ccccaatgca ttcatatttg ccaagccatc cccaccaccc agccatgatg atgcacggag 2460
gaccccctac ccaccctgga atgactatgt cagcacagag ccccacaatg ttaaattctg 2520
tagatcccaa tgttggcgga caggttatgg acattcatgc ccaatagtat aagggaactc 2580
aagggaaaag gaaacacacg caaaaactat tttaagactt tctgaacttt gaccagatgt 2640
tgacacttaa tatgaaattc cagacagctg tgattatttt ttacttttgt catttttcat 2700
caagcaacag aggaccaatg caacaagaac acaaatgtga aatcatgggc tgactgagac 2760
aattctgtcc atgtaaagat cctctggaaa aagactccga gagttataac tactgtagta 2820
taaatatagg aactaagtta aacttgtaca tttctgttga tcacgccgtt atgttgcctc 2880
aaatagtttt agaagagaaa aaaaaatata tccttgtttt ccacactatg tgtgttgttc 2940
ccaaaagaat gactgttttg gttcatcagt gaattcacca tccaggagag actgtggtat 3000
atattttaaa cctgttgggc caatgagaaa agaaccacac tggagatcat gatgaacttt 3060
tggctgaacc tcatcactcg aactccagct tcaagaatgt gttttcatgc ccggcctttg 3120
ttcctccata aatgtgtcct ttagtttcaa acagatcttt atagttcgtg cttcataagc 3180
caattcttat tattattttt gggggactct tcttcaaaga gcttgccaat gaagatttaa 3240
agacagagca ggagcttctt ccaggagttc tgagccttgg ttgtggacaa aacaatctta 3300
agttgggcag ctttcctcaa cacaaaaaaa agttattaat ggtcattgaa ccataactag 3360
gactttatca gaaactcaaa gcttggggga taaaaaggag caagagaata ctgtaacaaa 3420
cttcgtacag agttcggtct attaattgtt tcatgttaga tattctatgt gtttacctca 3480
attgaaaaaa aaaagaatgt ttttgctagt atcagatctg ctgtggaatt ggtattgtat 3540
gtccatgaat tcttcttttc tcagcacgtg ttcctcacta gaagaaaatg ctgttacctt 3600

taagctttgt caaatttaca ttaaaatact tgtatgagga ctgtgacgtt atgttaaaaa 3660

aaaaaaggtg ttaagtcaca aaaagcggta ataaatattt catttttgaa aaaaaaaaaa 3720

a 3721

<210> 10
<211> 3684
<212> DNA
<213> Homo sapiens

<400> 10
cctgctgccg cctgggcccc gccgagcgga gctagcgccg cgcgcagagc acacgctcgc 60

gctccagctc ccctcctgcg cggttcatga ctgtgtcccc tgaccgcagc ctctgcgagc 120

ccccgccgca ggaccacggc ccgctccccg ccgccgcgag ggccccgagc gaaggaagga 180

agggaggcgc gctgtgcgcc ccgcggagcc cgcgaacccc gctcgctgcc ggctgcccag 240

cctggctggc accatgctgc ccgcgcgctg cgcccgcctg ctcacgcccc acttgctgct 300

ggtgttggtg cagctgtccc ctgctcgcgg ccaccgcacc acaggcccca ggtttctaat 360

aagtgaccgt gacccacagt gcaacctcca ctgctccagg actcaaccca aacccatctg 420

tgcctctgat ggcaggtcct acgagtccat gtgtgagtac cagcgagcca agtgccgaga 480

cccgaccctg ggcgtggtgc atcgaggtag atgcaaagat gctggccaga gcaagtgtcg 540

cctggagcgg gctcaagccc tggagcaagc caagaagcct caggaagctg tgtttgtccc 600

agagtgtggc gaggatggct cctttaccca ggtgcagtgc catacttaca ctgggtactg 660

ctggtgtgtc accccggatg ggaagcccat cagtggctct tctgtgcaga ataaaactcc 720

tgtatgttca ggttcagtca ccgacaagcc cttgagccag ggtaactcag gaaggaaaga 780

tgacgggtct aagccgacac ccacgatgga gacccagccg gtgttcgatg gagatgaaat 840

cacagcccca actctatgga ttaaacactt ggtgatcaag gactccaaac tgaacaacac 900

caacataaga aattcagaga aagtctattc gtgtgaccag gagaggcaga gtgccctgga 960

agaggcccag cagaatcccc gtgagggtat tgtcatccct gaatgtgccc ctgggggact 1020

ctataagcca gtgcaatgcc accagtccac tggctactgc tggtgtgtgc tggtggacac 1080

agggcgcccg ctgcctggga cctccacacg ctacgtgatg cccagttgtg agagcgacgc 1140

cagggccaag actacagagg cggatgaccc cttcaaggac agggagctac caggctgtcc 1200

agaagggaag aaaatggagt ttatcaccag cctactggat gctctcacca ctgacatggt 1260

tcaggccatt aactcagcag cgcccactgg aggtgggagg ttctcagagc cagaccccag 1320

ccacaccctg gaggagcggg tagtgcactg gtatttcagc cagctggaca gcaatagcag 1380

caacgacatt aacaagcggg agatgaagcc cttcaagcgc tacgtgaaga agaaagccaa 1440

gcccaagaaa tgtgcccggc gtttcaccga ctactgtgac ctgaacaaag acaaggtcat 1500

ttcactgcct gagctgaagg gctgcctggg tgttagcaaa gaagtaggac gcctcgtcta 1560

aggagcagaa aacccaaggg caggtggaga gtccagggag gcaggatgga tcaccagaca 1620

cctaaccttc agcgttgccc atggccctgc cacatcccgt gtaacataag tggtgcccac 1680
catgtttgca cttttaataa ctcttacttg cgtgttttgt ttttggtttc attttaaaac 1740
accaatatct aataccacag tgggaaaagg aaagggaaga aagactttat tctctctctt 1800
attgtaagtt tttggatctg ctactgacaa cttttagagg gttttggggg ggtgggggag 1860
ggtgttgttg gggctgagaa gaaagagatt tatatgctgt atataaatat atatgtaaat 1920
tgtatagttc ttttgtacag gcattggcat tgctgtttgt ttatttctct ccctctgcct 1980
gctgtgggtg gtgggcactc tggacacata gtccagcttt ctaaaatcca ggactctatc 2040
ctgggcctac taaacttctg tttggagact gacccttgtg tataaagacg ggagtcctgc 2100
aattgtactg cggactccac gagttctttt ctggtgggag gactatattg ccccatgcca 2160
ttagttgtca aaattgataa gtcacttggc tctcggcctt gtccagggag gttgggctaa 2220
ggagagatgg aaactgccct gggagaggaa gggagtccag atcccatgaa tagcccacac 2280
aggtaccggc tctcagaggg tccgtgcatt cctgctctcc ggacccccaa agggcccagc 2340
attggtgggt gcaccagtat cttagtgacc ctcggagcaa attatccaca aaggatttgc 2400
attacgtcac tcgaaacgtt ttcatccatg cttagcatct actctgtata acgcatgaga 2460
ggggaggcaa agaagaaaaa gacacacaga agggccttta aaaaagtaga tatttaatat 2520
ctaagcaggg gaggggacag gacagaaagc ctgcactgag gggtgcggtg ccaacaggga 2580
aactcttcac ctccctgcaa acctaccagt gaggctccca gagacgcagc tgtctcagtg 2640
ccaggggcag attgggtgtg acctctccac tcctccatct cctgctgttg tcctagtggc 2700
tatcacaggc ctgggtgggt gggttggggg aggtgtcagt caccttgttg gtaacactaa 2760
agttgttttg ttggtttttt aaaaacccaa tactgaggtt cttcctgttc cctcaagttt 2820
tcttatgggc ttccaggctt taagctaatt ccagaagtaa aactgatctt gggtttccta 2880
ttctgcctcc cctagaaggg caggggtgat aacccagcta cagggaaatc ccggcccagc 2940
tttccacagg catcacaggc atcttccgcg gattctaggg tgggctgccc agccttctgg 3000
tctgaggcgc agctccctct gcccaggtgc tgtgcctatt caagtggcct tcaggcagag 3060
cagcaagtgg cccttagcgc cccttcccat aagcagctgt ggtggcagtg agggaggttg 3120
ggtagccctg gactggtccc ctcctcagat cacccttgca aatctggcct catcttgtat 3180
tccaacccga catccctaaa agtacctcca cccgttccgg gtctggaagg cgttggcacc 3240
acaagcactg tccctgtggg aggagcacaa ccttctcggg acaggatctg atggggtctt 3300
gggctaaagg aggtccctgc tgtcctggag aaagtcctag aggttatctc aggaatgact 3360
ggtggccctg ccccaacgtg gaaaggtggg aaggaagcct tctcccatta gccccaatga 3420
gagaactcaa cgtgccggag ctgagtgggc cttgcacgag acactggccc cactttcagg 3480
cctggaggaa gcatgcacac atggagacgg cgcctgcctg tagatgtttg gatcttcgag 3540
atctccccag gcatcttgtc tcccacagga tcgtgtgtgt aggtggtgtt gtgtggtttt 3600
cctttgtgaa ggagagaggg aaactatttg tagcttgttt tataaaaaat aaaaaatggg 3660

taaatcttga aaaaaaaaaa aaaa                                          3684

<210>  11
<211>  1735
<212>  DNA
<213>  Homo sapiens

<400>  11
agggtacggg ccgggaccgc cgcagcccgg ggcgggggca cggcaaccgc gaggcctggg      60

ggcgcccgcc ccccgcgccc cacgcccggt gccagcgagc cgaggcgtgc atctccttat     120

atggtcaaat gacacggcgg ggtttctcga gggcgggagc tgcgcagcgc tccactcggc     180

cggcagcgga gccgcagcca ccagccgccc gcgccctcca gccccgtccg ggagtccccg     240

gcccgctgcg gtgccgtgag tacctccaac cccctgcgcc ccggagggag gccgaggggc     300

ttagccacca gggctcggaa gtgggggccg aatccggtgc gagacccaag gagaggggag     360

cagagccgga gttggggaga ctggttgctg aaaagccagg agtcaaaatg actgagcgct     420

ttgactgcca ccattgcaac gaatctctct ttggcaagaa gtacatcctg cgggaggaga     480

gcccctactg cgtggtgtgc tttgagaccc tgttcgccaa cacctgcgag gagtgtggga     540

agcccatcgg ctgtgactgc aaggacttgt cttacaagga ccggcactgg catgaagcct     600

gtttccactg ctcgcagtgc agaaactcac tggtggacaa gccctttgct gccaaggagg     660

accagctgct ctgtacagac tgctattcca acgagtactc atccaagtgc caggaatgca     720

agaagaccat catgccaggt acccgcaaga tggagtacaa gggcagcagc tggcatgaga     780

cctgcttcat ctgccaccgc tgccagcagc caattggaac caagagtttc atccccaaag     840

acaatcagaa tttctgtgtg ccctgctatg agaaacaaca tgccatgcag tgcgttcagt     900

gcaaaaagcc catcaccacg ggaggggtca cttaccggga gcagccctgg cacaaggagt     960

gcttcgtgtg caccgcctgc aggaagcagc tgtctgggca gcgcttcaca gctcgcgatg    1020

actttgccta ctgcctgaac tgcttctgtg acttgtatgc caagaagtgt gctgggtgca    1080

ccaaccccat cagcggactt ggtggcacaa aatacatctc ctttgaggaa cggcagtggc    1140

ataacgactg ctttaactgt aagaagtgct ccctctcact ggtggggcgt ggcttcctca    1200

cagagaggga cgacatcctg tgccccgact gtgggaaaga catctgaatt caacacagag    1260

aagttgctgc ttgtgatctc acacacagat ttttatgttt tctttctcac ccaggcaatc    1320

ttgccttctg gtttcttcca gccacattga gactttcttc tagtgctttt cagtgatact    1380

cacgtttgct taaacccttt agtgctttgt gatagttcag tcccagggaa agagaaaact    1440

cgccctaggc cctaggtggg aagatggttt gaaatttttg taatcgagta aggcacaccc    1500

aaatgtaaaa atccttttga atgatgcctt tataaatctt tctctcactg tctatttaag    1560

tgcaattaac atatgtcacg aacttgaaag ttttctaaac tcaataaggt aatgaccagt    1620

tgttatttac agctctgtaa cctcccgttg cgtcaagtct aaaccaagat tatgtgactt    1680

gcaataaagt tattcagaac agaaaaaaaa aaaaaaaaaa aaaaaaaaaa aaaaa         1735

<210> 12
<211> 5603
<212> DNA
<213> Homo sapiens

<400> 12
ggagtgggcc aggccgccag ccccgccagc cccgccagcc ccgccagccc cgcgatggct 60

tgggccgcgc tcctcggcct cctggccgca ctgttgctgc tgctgctact gagccgccgc 120

cgcacgcggc gacctggtga gcctcccctg gacctgggca gcatcccctg gttggggtat 180

gccttggact ttggaaaaga tgctgccagc ttcctcacga ggatgaagga gaagcacggt 240

gacatcttta ctatactggt tgggggcagg tatgtcaccg ttctcctgga cccacactcc 300

tacgacgcgg tggtgtggga gcctcgcacc aggctcgact tccatgccta tgccatcttc 360

ctcatggaga ggatttttga tgtgcagctt ccacattaca gccccagtga tgaaaaggcc 420

aggatgaaac tgactcttct ccacagagag ctccaggcac tcacagaagc catgtatacc 480

aacctccatg cagtgctgtt gggcgatgct acagaagcag gcagtggctg gcacgagatg 540

ggtctcctcg acttctccta cagcttcctg ctcagagccg gctacctgac tctttacgga 600

attgaggcgc tgccacgcac ccatgaaagc caggcccagg accgcgtcca ctcagctgat 660

gtcttccaca cctttcgcca gctcgaccgg ctgctcccca aactggcccg tggctccctg 720

tcagtggggg acaaggacca catgtgcagt gtcaaaagtc gcctgtggaa gctgctatcc 780

ccagccaggc tggccaggcg ggcccaccgg agcaaatggc tggagagtta cctgctgcac 840

ctggaggaga tgggtgtgtc agaggagatg caggcacggg ccctggtgct gcagctgtgg 900

gccacacagg ggaatatggg tcccgctgcc ttctggctcc tgctcttcct tctcaagaat 960

cctgaagccc tggctgctgt ccgcggagag ctcgagagta tcctttggca agcggagcag 1020

cctgtctcgc agacgaccac tctcccacag aaggttctag acagcacacc tgtgcttgat 1080

agcgtgctga gtgagagcct caggcttaca gctgccccct tcatcacccg cgaggttgtg 1140

gtggacctgg ccatgcccat ggcagacggg cgagaattca acctgcgacg tggtgaccgc 1200

ctcctcctct tccccttcct gagcccccag agagacccag aaatctacac agacccagag 1260

gtatttaaat acaaccgatt cctgaaccct gacggatcag agaagaaaga cttttacaag 1320

gatgggaaac ggctgaagaa ttacaacatg ccctgggggg cggggcacaa tcactgcctg 1380

gggaggagtt atgcggtcaa cagcatcaaa caatttgtgt tccttgtgct ggtgcacttg 1440

gacttggagc tgatcaacgc agatgtggag atccctgagt ttgacctcag caggtacggc 1500

ttcggtctga tgcagccgga acacgacgtg cccgtccgct accgcatccg cccatgacac 1560

agggagcaga tggatccacg tgctcgcctc tgcccagcct gccccagcct gccccagcct 1620

cccagctttc tgtgtgcaca gttggcccgg gtgcaggtgc tagcattacc acttccctgc 1680

ttttctccca gaaggctggg tccaggggag ggaaaagcta agagggtgaa caaagaaaag 1740

acattgaaag ctctatggat tatccactgc aaagttttct ttccaaaatc aggctttgtc 1800

tgctcccaat tcacctcgtt actctcacct cgtgatatcc acaaatgcta ttcagataag 1860

gcagaactag gagtcttcac tgctctgccc ccaactcccg gaggtgtcac cttcctagtt 1920
cttatgagct agcatggccc gggccttatc cagtcaaagc ggatgctggc cacagaaagg 1980
ccactcagga tgtcctttgt gtccattgat gtcattcagc agtcagtccc ccaataatcc 2040
ttaaactagc taaaaccaaa ggagtccctt agaagatctg cttccctggg gccccatttg 2100
ccagattgcc ccattgctca cactacttga gaaaatgcag gagagcttcc cccaaggctg 2160
atgcattccc ggtgcagaac aggggcaccc tccaaacact gggctctgag gagtggagtt 2220
ctctgttcta gagtgacagg caccagatgg gatgggcttt ctcagtgtca gcactcaggt 2280
agggagctaa ggaagacaca gcccagacaa gatggctgga aggagccagc caggactcct 2340
tagactgatc aagccaaaaa agaaggtgcc gatttcatgc attctagtgc agaagcccca 2400
actgtgatca cgatccagtc tgcagacgtg ttttgtttgg acttcactta aaaaaatgcc 2460
ttagttgtta tcatctttgg gagagttcat tcaaaatgtc cagcttctct tgaaaacttg 2520
gtatatctgg ccacactggg ctcacattcc caagggtaac tcttggccag agctgagtgg 2580
cagccgcctc ccttatgcag gacatgtgct ctcggcttca ccagggttct gaccgggtct 2640
gcttctgcat tcacagcgcc tcctggacct gaaggcatct gagtgtgaga ccctgttcta 2700
actcttagaa gtgacattgt aagaggtggt ggggaccagc taattggtcc aacccagcct 2760
gagtgcacca ccctttgaac aaatgtatca gtgatgaaaa tttgcctttg ccccggcttg 2820
cctgtaatcc cagcactttg ggaggccgag gtgggcggat cacttgaggt cgggagttca 2880
agaccagcct ggccaacatg gcgaaacccc gtctctacta aacataaaaa aattagtcag 2940
gtgtggcggt gcgtgcctgt aatcccagct attcaggagg ctgaggcacc agaattgctt 3000
gaacccagga ggtggaggtt gcagtgaact gagactgcgc cacggcactc cagcctgggc 3060
gacagagcaa gactctgtct caataaataa ataattaatt aaaataaaaa acagcttaaa 3120
gagaaaaatg gcctgcaaac cttttttatg atgctatttt tattaatata aagtcctgtt 3180
tattgagacc ctttaaatgc ctgcgagaga ccctacagac agtatgtcta ctcctcacag 3240
catctctatg aagagaagga gggttgtgcc cacttcatag atgagaaaac tgagaggtga 3300
ggtgacttgc ctggggccac atagctcata agctgtagaa ctctgcaggc agatttactg 3360
tcccaggagc aaatgctgga tgagcaactc ctgttctttg ggctcaaggg gactggtgat 3420
gggacaattc ttctcgactt caggagctga cagagccaga ggcacctaaa cttgggtaca 3480
tcttgtaaga cacataatga ggtccctcta gctttagctg gagggagata aaagaacccc 3540
agacctctga atgtcccaga ggctagtctc ttctcagagc agcactgggg tttgggggct 3600
tccctgggcc tcagcctcca ggcaccccca ggattcccag agagacgctg tgattggcag 3660
gaggcaggat atccccaggg aaacccatct tcacgcctgg gtgaccccca ctgccgcctc 3720
cttcttaact ctgcaggaaa tcagagctgg cagcctccag tgggaggaca gagccggttt 3780
ccgtggcaac cctcagctgc ctcatcgtgg ctgggaaggg aaggaagcaa gccggcagaa 3840
atagctatgg aaggtcttgc gcaggctgca accctggtgt gctgggcgaa aacccttatg 3900

actcccccct tccaaatcag gctgggttgt cactgagact agattctcac ctgccttcaa 3960

agaagggcca attcccttta aaggtcgcac ctccttggaa ccacagtcat tagtgaatta 4020

cactcaagga aaagatgtgc tcccaccagg cagctccagc tgttacctga gatactgaag 4080

tgcagctcag gagacgatta tttaaacctg cccttgtttt aatcgttatt tttctcttta 4140

aaaaaaatag aagctataaa gaaaagaggg agagatgagt gggttagcta cctgctatgc 4200

gctagttagg aagttacctg gatgccattg tatttcttca tcccttgctt aagaatcaaa 4260

attactggac atatgttagg aatactcttt ctttctttct ttctttttaa gctcagtggc 4320

aagaatgaaa tactcttttt tttttttttt ttttgagatg gagtctcgct ctgctgccca 4380

ggctagagtg cagtggcgtg atctcggctc actgcaagct ctgcctcccg tgttcacacc 4440

attctcctgc ctcagcctcc tgagtagctg ggactacagg cacccgccac cacacccggc 4500

taatttttgg atttttagta gagatgggat ttcaccgtat tagccaggat ggtcttgatc 4560

tcctgagctc gtgatctgcc cgcctcggcc tcccaaagtg ctgggattac aggtgtgagc 4620

caccgcgccc agccaagaat aaaatactct taagttgatc taatgaagtg tttccttacc 4680

attgtgatta ttgttactat tatttgctat attttaatat tgttgtttac caaatattct 4740

cctttaaaca gactcgcttt ttaaactttt tttttttttt ttgagacgga gtttcacgct 4800

tattgcccag gctggagtgc aatggcacga tctcagctca ccacaacctc cacctcctgg 4860

gtttaagcaa tgcttctgcc tcagcttccc aagtagctga gattacaggc gcacaccacc 4920

acgcccaact gatttttgta tttttagtag agacggggtt tccccatgtt ggtcaggctg 4980

gtctcgaact cctgacctcg tgatttgcct gcctgggcct cccaaagtgc taggattaca 5040

ggcatgagcc accatgcccg gcctaaactt tgtttttaaa atgaactttt tttcccccca 5100

attgctgcca atagtggata acatgtatca ctcactgcca aaaatagaaa gtgaccatga 5160

aaaataaatt cgctggggaa gggggctcca tgctggtgtg gccaaggctg agagctctct 5220

cttctctgtt acaaaacgag ataagcaagt gttagaattg ccttaaggcc acactggcat 5280

ctccctgacc ttctccaggg acagaagcag gagtaagttt ctcatcccat gggcgaccag 5340

ggccatctcc tcccaccagt ggcccccact cacagggagc tggcaatgcc ctacctgcct 5400

gttctccaga tggagaaaca ggctctgaga tttcacaggt cttgcccaaa gtcattgatt 5460

ttgatgatta aaaagaataa acacagtgtt tcctgagtag cagtgattgt tatgccttgc 5520

tattttaata aagattctat tttcgtataa cattgtcaag tggaaacatg ctgaaatcta 5580

ttaaaccatc tttgtttgtg gaa 5603

<210> 13
<211> 3037
<212> DNA
<213> Homo sapiens

<400> 13
ctcctcacag gtgtgtctct agtcctcgtg gttgcctgcc ccactccctg ccgagacgcc 60

tgccagaaag gtcacctatc ctgaacccca gcaagcctga aacagctcag ccaagcaccc 120

tgcgatggaa gctgcagatg cctccaggag caacgggtcg agcccagaag ccagggatgc 180
ccggagcccg tcgggcccca gtggcagcct ggagaatggc accaaggctg acggcaagga 240
tgccaagacc accaacgggc acggcgggga ggcagctgag ggcaagagcc tgggcagcgc 300
cctgaagcca ggggaaggta ggagcgccct gttcgcgggc aatgagtggc ggcgacccat 360
catccagttt gtcgagtccg gggacgacaa gaactccaac tacttcagca tggactctat 420
ggaaggcaag aggtcgccgt acgcagggct ccagctgggg gctgccaaga agccacccgt 480
tacctttgcc gaaaagggcg agctgcgcaa gtccattttc tcggagtccc ggaagcccac 540
ggtgtccatc atggagcccg gggagacccg gcggaacagc tacccccggg ccgacacggg 600
ccttttttca cggtccaagt ccggctccga ggaggtgctg tgcgactcct gcatcggcaa 660
caagcagaag gcggtcaagt cctgcctggt gtgccaggcc tccttctgcg agctgcatct 720
caagccccac ctggagggcg ccgccttccg agaccaccag ctgctcgagc ccatccggga 780
ctttgaggcc cgcaagtgtc ccgtgcatgg caagacgatg gagctcttct gccagaccga 840
ccagacctgc atctgctacc tttgcatgtt ccaggagcac aagaatcata gcaccgtgac 900
agtggaggag gccaaggccg agaaggagac ggagctgtca ttgcaaaagg agcagctgca 960
gctcaagatc attgagattg aggatgaagc tgagaagtgg cagaaggaga aggaccgcat 1020
caagagcttc accaccaatg agaaggccat cctggagcag aacttccggg acctggtgcg 1080
ggacctggag aagcaaaagg aggaagtgag ggctgcgctg gagcagcggg agcaggatgc 1140
tgtggaccaa gtgaaggtga tcatggatgc tctggatgag agagccaagg tgctgcatga 1200
ggacaagcag acccgggagc agctgcatag catcagcgac tctgtgttgt ttctgcagga 1260
atttggtgca ttgatgagca attactctct ccccccaccc ctgcccacct atcatgtcct 1320
gctggagggg gagggcctgg gacagtcact aggcaacttc aaggacgacc tgctcaatgt 1380
atgcatgcgc cacgttgaga agatgtgcaa ggcggacctg agccgtaact tcattgagag 1440
gaaccacatg gagaacggtg gtgaccatcg ctatgtgaac aactacacga acagcttcgg 1500
gggtgagtgg agtgcaccgg acaccatgaa gagatactcc atgtacctga cacccaaagg 1560
tggggtccgg acatcatacc agccctcgtc tcctggccgc ttcaccaagg agaccaccca 1620
gaagaatttc aacaatctct atggcaccaa aggtaactac acctcccggg tctgggagta 1680
ctcctccagc attcagaact ctgacaatga cctgcccgtc gtccaaggca gctcctcctt 1740
ctccctgaaa ggctatccct ccctcatgcg gagccaaagc cccaaggccc agccccagac 1800
ttggaaatct ggcaagcaga ctatgctgtc tcactaccgg ccattctacg tcaacaaagg 1860
caacgggatt gggtccaacg aagccccatg agctcctggc ggaaggaacg aggcgccaca 1920
cccctgctct tcctcctgac cctgctgctc ttgccttcta agctactgtg cttgtctggg 1980
tgggagggag cctggtcctg cacctgccct ctgcagccct ctgccagcct cttgggggca 2040
gttccggcct ctccgacttc cccactggcc acactccatt cagactcctt tcctgccttg 2100
tgacctcaga tggtcaccat cattcctgtg ctcagaggcc aacccatcac aggggtgaga 2160

taggttgggg cctgccctaa cccgccagcc tcctcctctc gggctggatc tgggggctag 2220

cagtgagtac ccgcatggta tcagcctgcc tctcccgccc acgccctgct gtctccaggc 2280

ctatagacgt ttctctccaa ggccctatcc cccaatgttg tcagcagatg cctggacagc 2340

acagccaccc atctcccatt cacatggccc acctcctgct tcccagagga ctggccctac 2400

gtgctctctc tcgtcctacc tatcaatgcc cagcatggca gaacctgcag cccttggcca 2460

ctgcagatgg aaacctctca gtgtcttgac atcaccctac ccaggcggtg ggtctccacc 2520

acagccactt tgagtctgtg gtccctggag ggtggcttct cctgactggc aggatgacct 2580

tagccaagat attcctctgt tccctctgct gagataaaga attcccttaa catgatataa 2640

tccacccatg caaatagcta ctggcccagc taccatttac catttgccta cagaatttca 2700

ttcagtctac actttggcat tctctctggc gatggagtgt ggctgggctg accgcaaaag 2760

gtgccttaca cactgccccc accctcagcc gttgccccat cagaggctgc ctcctccttc 2820

tgattacccc ccatgttgca tatcagggtg ctcaaggatt ggagaggaga caaaaccagg 2880

agcagcacag tggggacatc tcccgtctca acagccccag gcctatgggg gctctggaag 2940

gatgggccag cttgcagggg ttggggaggg agacatccag cttgggcttt cccctttgga 3000

ataaaccatt ggtctgtcaa aaaaaaaaaa aaaaaaa 3037

<210> 14
<211> 2056
<212> DNA
<213> Homo sapiens

<400> 14
ccacagccca gctgttccct ccgcggattc tccggggctg gttcatcacc tccgaatatt 60

cctgtgacag gagacgcttg caaaacccgc ctccagcctc cagcagcaaa taaatagaag 120

gcttgcagcc cagaaggagc cagaagaagt ttctaggcgc gcgtgccctg ggtttattaa 180

gctcctggct ccgctctaga cctcagcggt tctggctgcc agcctgggca gcctgggaag 240

cctgggagga cggtggcttg ccggtctgtc gtgaggcagt gcggacgggg accctctggg 300

attctgctgg atctgccccg ggggttacct ttgggggctg ggaccccagt cgaggggaca 360

caaccgtccc tggcagtggt tggttctgct tctccctgca gaaaagcagc attttcggaa 420

gctgaagaat aagctagccc agccacacca ccttgttgtg tgaccttggg caggtggttc 480

tgtctctctg agcctctgtt tctctctgag ctgagcagcc accatggctg acggtcagat 540

gcccttctcc tgccactacc caagccgcct gcgccgagac cccttccggg actctcccct 600

ctcctctcgc ctgctggatg atggctttgg catggacccc ttcccagacg acttgacagc 660

ctcttggccc gactgggctc tgcctcgtct ctcctccgcc tggccaggca ccctaaggtc 720

gggcatggtg ccccggggcc ccactgccac cgccaggttt ggggtgcctg ccgagggcag 780

gaccccccca cccttccctg gggagccctg gaaagtgtgt gtgaatgtgc acagcttcaa 840

gccagaggag ttgatggtga agaccaaaga tggatacgtg gaggtgtctg gcaaacatga 900

agagaaacag caagaaggtg gcattgtttc taagaacttc acaaagaaaa tccagcttcc 960

tgcagaggtg gatcctgtga cagtatttgc ctcactttcc ccagagggtc tgctgatcat 1020

cgaagctccc caggtccctc cttactcaac atttggagag agcagtttca acaacgagct 1080

tccccaggac agccaggaag tcacctgtac ctgagatgcc agtactggcc catccttgtt 1140

ttgtccccaa ccctagggct tctctgattc caggatacat tactttagct gaactcagat 1200

ttagtgcaag taaaatgtta gagggtgcgg gggtgaggac tgaccacaga ttccctggat 1260

agtgtagtgg tagatttctc cacaggatag cgcaattggc aaatcatgct tggttgtgtt 1320

aggccaaaat actagttttg ctttctttac cttttctatc ttgatgaaaa tgttgcacat 1380

tctatagttg caaaacacat aaaaggggac ttaacatttc acgttgtatc ttacttgcag 1440

tgaatgcaag ggttactttt ctctggggac ctcccccatc acccaggttc ctactctggg 1500

ctcccgattc ccatggctcc caaaccatgc cgcatggttt ggttaatgaa acccagtagc 1560

taaccccact gtgcttccac atgcctggcc taaaatgggt gatatacagg tcttatatcc 1620

ccatatggaa tttatccatc aaccacataa aaacaaacag tgccttctgc cctctgccca 1680

gatgtgtcca gcacgttctc aaagtttcca cattagcact ccctaaggac gctgggagcc 1740

tgtcagttta tgatctgacc taggtccccc ctttcttctg tcccctgtgt ttaagtcggg 1800

atttttacag agggagctgt ctccagacag ctccatcagg aaccaagcaa aggccagata 1860

gcctgacaga taggctagtg gtattgtgta tatgggcggg acgtgtgtgt cattattatt 1920

tgagttatgc tgttgtttag gggtaaataa cagtaaataa ttaataataa taataataat 1980

aataaaggag ctgacgttct taaaaaaaaa aaaaaaaaaa aaaaaaaaaa aaaaaaaaaa 2040

aaaaaaaaaa aaaaaa 2056

<210> 15
<211> 1989
<212> DNA
<213> Homo sapiens

<400> 15
agcttcgggg gcgagcgctc gtgtgtgtga gtgcgcgccg gccagcgcgc cttctgcggc 60

aggcggacag atcctcggcg cggcagggcc ggggcaagct ggacgcagca tgatgcgcgc 120

agtgtgggag gcgctggcgg cgctggcggc ggtggcgtgc ctggtgggcg cggtgcgcgg 180

cgggcccggg ctcagcatgt tcgcgggcca ggcggcgcag cccgatccct gctcggacga 240

gaacggccac ccgcgccgct gcatcccgga ctttgtcaat gcggccttcg gcaaggacgt 300

gcgcgtgtcc agcacctgcg gccggccccc ggcgcgctac tgcgtggtga gcgagcgcgg 360

cgaggagcgg ctgcgctcgt gccacctctg caacgcgtcc gaccccaaga aggcgcaccc 420

gcccgccttc ctcaccgacc tcaacaaccc gcacaacctg acgtgctggc agtccgagaa 480

ctacctgcag ttcccgcaca acgtcacgct cacactgtcc ctcggcaaga agttcgaagt 540

gacctacgtg agcctgcagt tctgctcgcc gcggcccgag tccatggcca tctacaagtc 600

catggactac gggcgcacgt gggtgccctt ccagttctac tccacgcagt gccgcaagat 660

gtacaaccgg ccgcaccgcg cgcccatcac caagcagaac gagcaggagg ccgtgtgcac 720

cgactcgcac accgacatgc gcccgctctc gggcggcctc atcgccttca gcacgctgga 780

cgggcggccc tcggcgcacg acttcgacaa ctcgcccgtg ctgcaggact gggtcacggc 840

cacagacatc cgcgtggcct tcagccgcct gcacacgttc ggcgacgaga acgaggacga 900

ctcggagctg gcgcgcgact cgtacttcta cgcggtgtcc gacctgcagg tgggcggccg 960

gtgcaagtgc aacggccacg cggcccgctg cgtgcgcgac cgcaccgaca gcctggtgtg 1020

cgactgcagg cacaacacgg ccggcccgga gtgcgaccgc tgcaagccct tccactacga 1080

ccggccctgg cagcgcgcca cagcccgcga agccaacgag tgcgtggcct gtaactgcaa 1140

cctgcatgcc cggcgctgcc gcttcaacat ggagctctac aagctttcgg ggcgcaagag 1200

cggaggtgtc tgcctcaact gtcgccacaa caccgccggc cgccactgcc attactgcaa 1260

ggagggctac taccgcgaca tgggcaagcc catcacccac cggaaggcct gcaaagcctg 1320

tgattgccac cctgtgggtg ctgctggcaa aacctgcaac caaaccaccg gccagtgtcc 1380

ctgcaaggac ggcgtgacgg gtatcacctg caaccgctgc gccaaaggct accagcagag 1440

ccgctctccc atcgccccct gcataaagat ccctgtagcg ccgccgacga ctgcagccag 1500

cagcgtggag gagcctgaag actgcgattc ctactgcaag gcctccaagg ggaagctgaa 1560

gattaacatg aaaaagtact gcaagaagga ctatgccgtc cagatccaca tcctgaaggc 1620

ggacaaggcg ggggactggt ggaagttcac ggtgaacatc atctccgtgt ataagcaggg 1680

cacgagccgc atccgccgcg gtgaccagag cctgtggatc cgctcgcggg acatcgcctg 1740

caagtgtccc aaaatcaagc ccctcaagaa gtacctgctg ctgggcaacg cggaggactc 1800

tccggaccag agcggcatcg tggccgataa aagcagcctg gtgatccagt ggcgggacac 1860

gtgggcgcgg cggctgcgca agttccagca gcgtgagaag aagggcaagt gcaagaaggc 1920

ctagcgccga ggcagcgggc gggcgggccg ggcgggcccg agggcggggc gagcgagacg 1980

gcgcttggc 1989

<210> 16
<211> 3278
<212> DNA
<213> Homo sapiens

<400> 16
ggcccgaaga tggcggcggt ggctggatct ggggctgccg cggctccgag ctcactgctc 60

ctcgtggtgg gcagcgagtt cgggagcccg gggctcctca cctacgtcct ggaggagctc 120

gaaagaggca tccggtcttg ggatgtcgat cctggcgtct gcaaccttga tgaacagctc 180

aaggtctttg tgtcccgaca ctctgccacc ttctccagca ttgtgaaagg ccagcggagc 240

ctgcaccacc gtggagacaa cctggagacc ctggtcctcc tgaacccatc agacaagtcc 300

ctgtatgatg agctccggaa ccttctgttg gaccctgcct ctcacaagct actggtgttg 360

gctgggccct gcctggagga gacgggggag ctgctgctac agacaggggg cttctcgcct 420

caccacttcc tccaggtcct gaaggacaga gagatccggg acatcctggc caccacgccc 480
ccacctgtgc agccgcccat actcaccatc acctgcccca ccttcggtga ctgggctcag 540
ctggcacccg ctgtgcctgg ccttcagggg gcgctccggc tccagctgcg gctgaacccc 600
ccggcgcagc tgcccaactc tgagggcctg tgcgaattcc tggagtacgt ggctgagtct 660
ctggagccac cgtccccctt cgagctgctg gagcccccga cctccggggg cttcctcagg 720
ctgggccggc cctgctgcta catcttccct ggaggcctcg gggatgccgc cttcttcgcc 780
gtcaatggct tcactgtgct ggtcaacggt ggctcaaacc ccaagtccag tttctggaag 840
ctggtgcggc acctggaccg cgtggatgcc gtgctggtga cccaccctgg cgccgacagc 900
ctccccggcc tcaacagcct gctgcggcgc aaactggcgg agcgctccga ggtggctgct 960
ggtgggggct cctgggacga caggctgcgc aggctcatct cccccaacct gggggtcgtg 1020
ttcttcaacg cctgcgaggc cgcgtcgcgg ctggcgcgcg gcgaggatga ggcggagctg 1080
gcgctgagcc tcctggcgca gctgggcatc acgcctctgc cactcagccg cggccccgtg 1140
ccagccaaac ccaccgtgct cttcgagaag atgggcgtgg gccggctgga catgtatgtg 1200
ctgcacccgc cctccgccgg cgccgagcgc acgctggcct ctgtgtgcgc cctgctggtg 1260
tggcaccccg ccggccccgg cgagaaggtg gtgcgcgtgc tgttccccgg ttgcaccccg 1320
cccgcctgcc tcctggacgg cctggtccgc ctgcagcact tgaggttcct gcgagagccc 1380
gtggtgacgc cccaggacct ggaggggccg gggcgagccg agagcaaaga gagcgtgggc 1440
tcccgggaca gctcgaagag agagggcctc ctggccaccc accctagacc tggccaggag 1500
cgccctgggg tggcccgcaa ggagccagca cgggctgagg ccccacgcaa gactgagaaa 1560
gaagccaaga cccccccgga gttgaagaaa gaccccaaac cgagtgtctc ccggacccag 1620
ccgcgggagg tgcgccgggc agcctcttct gtgcccaacc tcaagaagac gaatgcccag 1680
gcggcaccca agccccgcaa agcgcccagc acgtcccact ctggcttccc gccggtggca 1740
aatggacccc gcagcccgcc cagcctccga tgtggagaag ccagcccccc cagtgcagcc 1800
tgcggctctc cggcctccca gctggtggcc acgcccagcc tggagctggg gccgatccca 1860
gccggggagg agaaggcact ggagctgcct ttggccgcca gctcaatccc aaggccacgc 1920
acaccctccc ctgagtccca ccggagcccc gcagagggca gcgagcggct gtcgctgagc 1980
ccactgcggg gcggggaggc cgggccagac gcctcaccca cagtgaccac acccacggtg 2040
accacgccct cactacccgc agaggtgggc tccccgcact cgaccgaggt ggacgagtcc 2100
ctgtcggtgt cctttgagca ggtgctgccg ccatccgccc ccaccagtga ggctgggctg 2160
agcctcccgc tgcgtggccc ccgggcgcgg cgctcggctt ccccacacga tgtggacctg 2220
tgcctggtgt caccctgtga atttgagcat cgcaaggcgg tgccaatggc accggcacct 2280
gcgtcccccg gcagctcgaa tgacagcagt gcccggtcac aggaacgggc aggtgggctg 2340
ggggccgagg agacgccacc cacatcggtc agcgagtccc tgcccaccct gtctgactcg 2400
gatcccgtgc ccctggcccc cggtgcggca gactcagacg aagacacaga gggctttgga 2460

gtccctcgcc acgacccttt gcctgacccc ctcaaggtcc ccccaccact gcctgaccca 2520

tccagcatct gcatggtgga ccccgagatg ctgcccccca agacagcacg gcaaacggag 2580

aacgtcagcc gcacccggaa gcccctggcc cgccccaact cacgcgctgc cgcccccaaa 2640

gccactccag tggctgctgc caaaaccaag gggcttgctg gtggggaccg tgccagccga 2700

ccactcagtg cccggagtga gcccagtgag aagggaggcc gggcacccct gtccagaaag 2760

tcctcaaccc ccaagactgc cactcgaggc ccgtcggggt cagccagcag ccggcccggg 2820

gtgtcagcca ccccacccaa gtccccggtc tacctggacc tggcctacct gcccagcggg 2880

agcagcgccc acctggtgga tgaggagttc ttccagcgcg tgcgcgcgct ctgctacgtc 2940

atcagtggcc aggaccagcg caaggaggaa ggcatgcggg ccgtcctgga cgcgctactg 3000

gccagcaagc agcattggga ccgtgacctg caggtgaccc tgatccccac tttcgactcg 3060

gtggccatgc atacgtggta cgcagagacg cacgcccggc accaggcgct gggcatcacg 3120

gtgttgggca gcaacagcat ggtgtccatg caggatgacg ccttcccggc ctgcaaggtg 3180

gagttctagc cccatcgccg acacgccccc cactcagccc agcccgcctg tccctagatt 3240

cagccacatc agaaataaac tgtgactaca cttggcaa 3278

<210> 17
<211> 431
<212> DNA
<213> Homo sapiens

<400> 17
ccacgccgtc cgggtgggcc tagcagtcgc tccatttatc gcttgagatc tccagcctta 60

ccgcggctcg aaatggaccc caactgctcc tgcaccactg gtgtctcctg cgcctgcacc 120

ggctcctgca agtgcaaaga gtgcaaatgc acctcctgca agaagagctg ctgctcctgc 180

tgccccgtgg gctgtgccaa gtgtgcccac ggctgtgtct gcaaagggac gttggagaac 240

tgcagctgct gtgcctgatg tgggaacagc tcttctccca gatgttaata gaacaagctg 300

cacaacctgg attttttttc aatacgatac tgagccattt gctgcatttc tttttatgtt 360

aaatatgtga gtgacaataa aacaattttg acttgaaaaa aaaaaaaaaa aaaaaaaaaa 420

aaaaaaaaaa a 431

<210> 18
<211> 2859
<212> DNA
<213> Homo sapiens

<400> 18
   ctttccgcgg cattctttgg gcgtgagtca tgcaggtttg cagccagccc caaagggggt 60
   gtgtgcgcga gcagagcgct ataaatacgg cgcctcccag tgcccacaac gcggcgtcgc 120
   caggaggagc gcgcgggcac agggtgccgc tgaccgaggc gtgcaaagac tccagaattg 180
   gaggcatgat gaagactctg ctgctgtttg tggggctgct gctgacctgg gagagtgggc 240
   aggtcctggg ggaccagacg gtctcagaca atgagctcca ggaaatgtcc aatcagggaa 300
   gtaagtacgt caataaggaa attcaaaatg ctgtcaacgg ggtgaaacag ataaagactc 360
   tcatagaaaa aacaaacgaa gagcgcaaga cactgctcag caacctagaa gaagccaaga 420
   agaagaaaga ggatgcccta aatgagacca gggaatcaga gacaaagctg aaggagctcc 480
   caggagtgtg caatgagacc atgatggccc tctgggaaga gtgtaagccc tgcctgaaac 540
   agacctgcat gaagttctac gcacgcgtct gcagaagtgg ctcaggcctg gttggccgcc 600
   agcttgagga gttcctgaac cagagctcgc ccttctactt ctggatgaat ggtgaccgca 660
   tcgactccct gctggagaac gaccggcagc agacgcacat gctggatgtc atgcaggacc 720
   acttcagccg cgcgtccagc atcatagacg agctcttcca ggacaggttc ttcacccggg 780
   agccccagga tacctaccac tacctgccct tcagcctgcc ccaccggagg cctcacttct 840
   tctttcccaa gtcccgcatc gtccgcagct tgatgccctt ctctccgtac gagcccctga 900
   acttccacgc catgttccag cccttccttg agatgataca cgaggctcag caggccatgg 960
   acatccactt ccatagcccg gccttccagc acccgccaac agaattcata cgagaaggcg 1020
   acgatgaccg gactgtgtgc cgggagatcc gccacaactc cacgggctgc ctgcggatga 1080
   aggaccagtg tgacaagtgc cgggagatct tgtctgtgga ctgttccacc aacaacccct 1140
   cccaggctaa gctgcggcgg gagctcgacg aatccctcca ggtcgctgag aggttgacca 1200
   ggaaatacaa cgagctgcta aagtcctacc agtggaagat gctcaacacc tcctccttgc 1260
   tggagcagct gaacgagcag tttaactggg tgtcccggct ggcaaacctc acgcaaggcg 1320
   aagaccagta ctatctgcgg gtcaccacgg tggcttccca cacttctgac tcggacgttc 1380
   cttccggtgt cactgaggtg gtcgtgaagc tctttgactc tgatcccatc actgtgacgg 1440
   tccctgtaga agtctccagg aagaacccta aatttatgga gaccgtggcg gagaaagcgc 1500
   tgcaggaata ccgcaaaaag caccgggagg agtgagatgt ggatgttgct tttgcaccta 1560
   cgggggcatc tgagtccagc tccccccaag atgagctgca gccccccaga gagagctctg 1620
   cacgtcacca agtaaccagg ccccagcctc caggccccca actccgccca gcctctcccc 1680
   gctctggatc ctgcactcta acactcgact ctgctgctca tgggaagaac agaattgctc 1740
   ctgcatgcaa ctaattcaat aaaactgtct tgtgagctga tcgcttggag ggtcctcttt 1800
   ttatgttgag ttgctgcttc ccggcatgcc ttcattttgc tatggggggc aggcaggggg 1860
   gatggaaaat aagtagaaac aaaaaagcag tggctaagat ggtataggga ctgtcatacc 1920
   agtgaagaat aaaagggtga agaataaaag ggatatgatg acaaggttga tccacttcaa 1980
   gaattgcttg ctttcaggaa gagagatgtg tttcaacaag ccaactaaaa tatattgctg 2040
   caaatggaag cttttctgtt ctattataaa actgtcgatg tattctgacc aaggtgcgac 2100
   aatctcctaa aggaatacac tgaaagttaa ggagaagaat cagtaagtgt aaggtgtact 2160
   tggtattata atgcataatt gatgttttcg ttatgaaaac atttggtgcc cagaagtcca 2220
   aattatcagt tttatttgta agagctattg cttttgcagc ggttttattt gtaaaagctg 2280
   ttgatttcga gttgtaagag ctcagcatcc caggggcatc ttcttgactg tggcatttcc 2340
   tgtccaccgc cggtttatat gatcttcata cctttccctg gaccacaggc gtttctcggc 2400
   ttttagtctg aaccatagct gggctgcagt accctacgct gccagcaggt ggccatgact 2460
   acccgtggta ccaatctcag tcttaaagct caggcttttc gttcattaac attctctgat 2520
   agaattctgg tcatcagatg tactgcaatg gaacaaaact catctggctg catcccaggt 2580
   gtgtagcaaa gtccacatgt aaatttatag cttagaatat tcttaagtca ctgtcccttg 2640
   tctctctttg aagttataaa caacaaactt aaagcttagc ttatgtccaa ggtaagtatt 2700
   ttagcatggc tgtcaaggaa attcagagta aagtcagtgt gattcactta atgatataca 2760
   ttaattagaa ttatggggtc agaggtattt gcttaagtga tcataattgt aaagtatatg 2820
   tcacattgtc acattaatgt caaaaaaaaa aaaaaaaaa 2859

<210> 19
<211> 454
<212> DNA
<213> Homo sapiens

<400> 19
tctgtcccgc tgcgtgtttt cctcttgatc gggaactcct gcttctcctt gcctcgaaat 60

ggaccccaac tgctcctgct cgcctgttgg ctcctgtgcc tgtgccggct cctgcaaatg 120

caaagagtgc aaatgcacct cctgcaagaa gagctgctgc tcctgctgcc ctgtgggctg 180

tgccaagtgt gcccagggct gcatctgcaa agggacgtca gacaagtgca gctgctgtgc 240

ctgatgccag gacagctgtg ctctcagatg taaatagagc aacctatata aacctggatt 300

tttttttttt tttttttgta caaccctgac ccgtttgcta catctttttt tctatgaaat 360

atgtgaatgg caataaattc atctagacta aaaaaaaaaa aaaaaaaaaa aaaaaaaaaa 420

aaaaaaaaaa aaaaaaaaaa aaaaaaaaaa aaaa 454

<210> 20
<211> 3580
<212> DNA
<213> Homo sapiens

<400> 20
agttctggcc gctgtcccgg tgcgcacgga cgtggctcga gtttcctctg ctctccgctc 60

tcgcccgcta gctctcctcc cttccgctcc tgcttctctc cgggtctccc gctccagctc 120

cagccccacc cggccggtcc cgcacggctc cgggtagcca tggaggaccc cacgctctat 180

attgtcgagc ggccgcttcc cgggtacccc gacgccgagg ccccggagcc ttcctccgct 240

ggggctcagg cagcggagga gccgtcgggg gccggctcag aagagctgat caagtcggac 300

caggtgaacg gcgtgctggt gctgagcctc ctggacaaaa tcatcggggc cgtagaccag 360

atccagctga ctcaagcaca gctggaggag cggcaggcgg agatggaggg cgcagtgcag 420

agcatccagg gcgagctgag caagctgggc aaggcgcacg ccaccacgag caatacggtg 480

agcaagctgc tggagaaggt gcgcaaggtc agcgtcaacg tgaagaccgt gcgcggcagc 540

ctggagcgcc aggcggggca gatcaagaag ctggaggtca acgaggccga gctgctgcgg 600

cgccgcaact ttaaagtcat gatctaccag gatgaagtga agctgccggc caaactgagc 660
atcagcaaat cgctgaaaga gtcggaggcg ctgccagaga aggagggcga ggagctgggc 720
gagggcgagc ggcccgagga ggacgcagcg gcgctggagc tttcgtcgga cgaggcggtg 780
gaggttgagg aggttattga ggagtcccgc gcagagcgta tcaagcgcag cggcctgcgg 840
cgcgtggacg acttcaagaa ggccttctcc aaggagaaga tggagaagac caaggtgcgt 900
acccgcgaga acctggagaa gacgcgcctc aagaccaagg aaaacctgga gaagacgcgg 960
cacaccctgg agaagcgcat gaacaagctg ggcacgcgcc tggtgcccgc cgagcggcgc 1020
gagaaactga agacgtcgcg agacaagttg cgcaaatcct tcacgcccga ccacgtggtg 1080
tacgcgcgct ccaagaccgc ggtctacaag gtgccaccct tcaccttcca cgtcaagaag 1140
atccgcgagg gccaggtgga agtgctcaag gccaccgaga tggtggaggt gggcgccgac 1200
gacgacgagg gcggcgcgga gcgcggggag gccggcgacc tgcggcgcgg gagcagcccc 1260
gacgtgcacg cgctgctgga gatcaccgag gagtcggacg ccgtgctggt ggacaagagc 1320
gacagcgact gagccgcccc cgctgccacc caccccattc ctcgctcctt ccgaacttcc 1380
tctttcgcat tctctctcgg ctcgagctgg ctgagatttt tctaaattga aaacacgccc 1440
ccctccccac acctccagga actccactcc cagtcttaga gctgttagga cccgatgggg 1500
aggcagcccc cgcagtggac agcccccgct tggacacagt ccgagtggaa tgggaaggga 1560
atggtcaatc cctgtcctgg ttgtccaagt cgggatctca gaggaaattg cagtgattcc 1620
acggttaggc ccccctgggg gggctgcctt cccctcagcc tctccccaca ccacccaccc 1680
agctgctgtc attccgctca ctgagctctt cttcattctc accctgatcc ctgggggact 1740
caaagccaaa actgcccaaa gaggaaagat tgaatcctaa aggggatcct tgcccccatg 1800
ggaggccccc tactagaagg acgtgaaagc agcttttggg ggaaactgag gcagtgggga 1860
agacagagca gaatgagccc tcaccctggc tgggggtcca gcacaggctg tatctgcaga 1920
gggtcccaga ggaacgctgg agccaagaga agccctggga aggaggggtg gggaacgaca 1980
tgcatgtgag ggatggcaca ctgatgtgtt tatgcacctg tacacaggag cgcatggcca 2040
tggctttgga aaggagaatg gaaaaataga agaaggtcgg ccgggcttgg tggcttaagc 2100
ctgtaacccc agcactttgg gaggccgagg tgggcggatc acctgaggtc aggagttcgg 2160
gaccagcctg gcaaacaccc catctctact aagcgaaaac ccatctctac taaaattaca 2220
aaaattagct gggcatggtt gcgcatgcct gtaatcccag ctactttgga ggctgaggtg 2280
gggagaattg cttgaacctg ggaggtggag gttgcagtga gccaaggtcg cgacactgca 2340
ctccagcctg ggtgacagag tgagactcca tctcaacaga aggaaaaaaa aggaaaatag 2400
gagaaggtgg aaatgggtga agagagaagt cccctcacta gctgcatgag aaatctatct 2460
tactgtggtt ctccatgggc agcaggacca tttttcagaa tcaagaggga ggacagtgtg 2520
agaaggcgat gatccaaaga agacagagag gtcagcccca cccgatccct caaatgggct 2580
cttggaggca cccccagggg cagcccattt ctcaaagtcc agaaaattag ggtcccagaa 2640

gggggcagca gcaggctggg agttaggagg gagagcaggg tgccggccct gccaccaagt 2700
tgagagctgg aggggaggtg gggagagaac atcacagagc agccagccct ggttcactcc 2760
tggcagtttc ttctcaagct ccttccctag gagcatggtg gcacgtgcct gtggtctcag 2820
ctacttggga ggctgaggcg ggaggatcgc cagagcccag gagtttgagg ctgcagtgag 2880
ctatgagggt gcctctgtgc tccatcctag gcaacagagt gagacgctgt ttaaaaagga 2940
aaaaatcctt ccctagagct agtatcctaa agctgcagag ctagcccaga cctcattggt 3000
ttccttgtcc ttggggtgct tttcctgaat ctttgggggt gaagggagtg ttgctcccag 3060
tccagaggcc tgattctgtt cggactgggt tctcaagaca cgaccaggtt ctcaagacac 3120
gagtcccctt gttcctcccc attaaagggg gtttgtcaga agcaagaaca gcccctctcc 3180
ccagtcacag cctgaaggga ggccccgaga gcttcctcct tccccccacc tgctccttac 3240
cttctctgcc ctgcttttta gaactgcagt tcattgtttt aagggattgg gggagggagc 3300
ctggggacac aaacctttta tacaatacaa agctttgctt tttttttttt ttttcttcct 3360
tttccctttc tcggttctct tctctcctct gaatggctga agacccctct gccgagggag 3420
gttggggatt gtgggacaag gtcccttggt gctgatggcc tgaaggggcc tgagctgtgg 3480
gcagatgcag ttttctgtgg gcttggggaa cctctcacgt tgctgtgtcc tggtgagcag 3540
cccgaccaat aaacctgctt ttccaaaaaa aaaaaaaaaa 3580

<210> 21
<211> 5066
<212> DNA
<213> Homo sapiens

<400> 21
gcaccattca ctccacctga tctcggggcg ctgtgcgctg aggaaggcgc gggcgagccg 60
gagcagaaga aggagggagg gagccagccg ctgcagccac caccgccacc atgtcctacc 120
aaggcaagaa gaacatcccg cggatcacga gtgaccgtct ccttatcaag ggaggcagaa 180
tcgtcaatga tgatcagtcc ttttatgctg atatttacat ggaagatggc ttaataaaac 240
aaattggaga caatctgatt gttcctggag gagtgaagac cattgaagcc aatgggaaga 300
tggtgatccc tggaggcatc gatgtccata ctcacttcca gatgccatat aagggaatga 360
ccacagtaga tgacttcttc caagggacaa aggcggcctt agcaggtggc accaccatga 420
tcattgacca tgtggtgcct gagcctgagt ccagcctgac tgaggcctat gagaaatgga 480
gagagtgggc tgatgggaag agttgctgtg actatgccct gcatgtggac atcacccact 540
ggaatgacag cgtcaagcag gaagtgcaga acctcatcaa ggacaaaggg gttaactcct 600
tcatggttta tatggcttat aaggatttgt atcaagtatc taacacagag ctctatgaga 660
tcttcacctg cctgggagag ctgggggcca ttgctcaagt tcatgctgag aatggggata 720
tcattgccca ggagcaaacc cgcatgttgg aaatggggat aactggccca gaaggccatg 780
tactgagcag gccagaagag ctggaagctg aggctgtgtt ccgtgccatc accattgcca 840
gccaaaccaa ttgccctctc tacgtcacaa aggtcatgag caagagtgca gctgacctca 900

tctcacaagc caggaaaaaa ggaaatgtag tctttggtga gcccatcact gccagcctcg 960
gcatagatgg aacccattat tggagcaaga actgggccaa ggcggctgca tttgtgacat 1020
ccccacccct gagccctgac ccaactactc cggactacat caactccttg ctggccagcg 1080
gggatctgca gctatctggg agtgcccact gcaccttcag cactgcccag aaagcaattg 1140
ggaaggacaa cttcacagcc attcctgagg gcaccaatgg tgtggaggag cggatgtctg 1200
tcatctggga caaggctgtg gccacaggga aaatggacga aaaccagttc gtggctgtga 1260
caagcacaaa cgctgccaag atcttcaacc tgtatccccg caagggaaga atatctgtgg 1320
gttctgacag cgacctcgtc atctgggatc cagatgctgt gaagatcgtc tctgccaaga 1380
accaccagtc tgcggcagag tacaacatct ttgaagggat ggagctgcgc ggggctcctc 1440
tggttgtcat ctgccagggc aagatcatgc tggaagatgg caacctgcac gtgacccagg 1500
gggctggccg cttcataccc tgcagcccgt tctccgacta tgtctacaag cgcattaaag 1560
cacggaggaa gatggcagac ctgcatgccg tcccaagggg catgtacgat gggcctgtgt 1620
ttgacctgac caccaccccc aaaggtggca cccccgcagg ctctgctcgg ggctctccta 1680
ctcggccgaa cccacctgtg aggaatcttc atcagtcggg atttagcctg tcaggcaccc 1740
aagtggatga gggggttcgc tcagccagca agcgcatcgt ggcgccccca ggcggccgtt 1800
ctaatatcac atctctgagt taagcaagcc ttcctcaaag agaggggcag aagcaagaag 1860
agattgtttt gaagccaaaa tggtacaccg atatttaaga aggaaagcga atccaaacgg 1920
ttgtgatcta aagaatcaat aagcctcaag ccttatgttt ctccaatgtt acgctcgctt 1980
gcctagcttt acgaatattg ctttgttttc tgtttatgca tagccttgat ttgtttgact 2040
cccctccccc catttacatg catgcaatca gacaggccac taaggtaaaa gagtctgctc 2100
tatcatagtg ttgagagcgt gtgtagtgct gcatcttatg acaaggggac agacaagctg 2160
ggacgtcagg gaaatgaaca aaagggacgc aggttatttg gggtgagtgg gtggtgggag 2220
cctggagcaa ggtggagggt gcagaggggc tggggtaggg catgtaggag ggaggtgggt 2280
gggtcaggtg agtggaaggg gtgttgtata ttgtgttgat gacgtacgtt atttccatgg 2340
aagatagccg ctgtggcagc tgtcacatca ccacagctcc ctagggtctg ccgagaaggc 2400
aggcagtctt tgggttctgt tctttgtcac gtcccctaca agtaaatttt gtttctttga 2460
acgtttatta aaatgccaag acccaaccat ttcttccacc tgcttgattg tgccagtgtt 2520
tgctcaggcc tctttcttag tgttgctttc aaatccttct ctttcctggg ttgggaaggc 2580
caggcaggga cagagcaaat gacacttctc ttcctcttgc cctccctgcc tctttggtgc 2640
tcttaaaagc cagcagctga gaacatagca caggcccacg tggtgagggc acccacagct 2700
taaagacgct tccttctaaa cacggcgagg tcacctctca ctcttctgtc tttgcaaacc 2760
gagaagagtg gcatgcttct ggcatcccaa gtcaggattt tagctcagat gaggcagaat 2820
gaagggcctc tcttacaggc agtttgtgtt tgattctctc gatcctggca catccatgat 2880
aaataggagt ttttgaaagt tggttttatt aggtgttccc taatttttac cgtaataggt 2940

catctcagct tatatgaaag tcaagtgggg aactgggaaa gccaaagtca gtcttgagca 3000
gagggagcac attttgtgga cctggttcca cctttccatt ccaaaccacc tgtttcccct 3060
tccattagca gaaactctgg gggaactttg tgtctcagtc ctagaatctc cccaagtgag 3120
tggaagtgac atgatgcagt cttcctcatg gggcacctga aagaaattag tgtgggtgct 3180
tcgatctacc ttgtctgtca gagttgaata tctctttccc tatcatgctg cttctgaaaa 3240
ttcagttttg gagcaagtcc tgtgagcaag ataagaatct atagaaccaa gatgctcatt 3300
ttcagaagaa atatgttcaa cctgggatca gacttccatg ctctggggaa tccaagtggt 3360
agcacctgta accctgtgta ctaagtgctt tgaagagaag agcaggcctc agacaccttt 3420
taattgctta ggagaaacca ttgtctctga ctgcaggttt gaataagttg aagaccagag 3480
aaaagtacac actgggctac aaaggaattt ggagatagcc aaggaacagg atttccccta 3540
gcaagctacc ttctgttcaa atcatgaaaa aagactattt ccccttagaa tagggaagct 3600
tgctatttta aagctcttgt agtgcttttc ttttaaggga gatgtagtaa aagggaaaat 3660
gtagctctta gtttacactt caaagatgtg ggggtctttc agagaactaa gaataacagt 3720
tttatgtgca gagagagttt gccagatctg aagcatatac ctcattgact aggctgttac 3780
tttgggatag gttgcagtac cagccacagc cagcagatag aggaaaagac acacataaac 3840
tcgcttctga gcgtccactt ctgcactctc tgctctgctg ttactcagcc cctgagtctg 3900
actcatctct gcacaacctc tctgtgccat gaagataagt cttccatggc caaatcggtc 3960
atccgcactg cccttgggac ttccgaagtg aaccattcca ccagaacctt tgattctgca 4020
caagatttcc ttgctctggg aacaaccccc aaatgccctt gggaggaaca acatgagctc 4080
aggaagcctc tctttcttca cttaccatta ctaactctcc aagcatagaa atccctggga 4140
attgcgagaa taactcccac tattttaaaa tttatattca gatttgtttc gtttcataag 4200
acacatcaaa caggcctata caaaaggttt aggaaaagaa aacaatggtg agtcccggcc 4260
ctcttcgaat tcactggcac ctcatgcaag tgtaggaagg cacgctggat cgtctatctg 4320
attccaaagc tgtcctttgc catctcatcc cttggcctgc cccccaaccc tgaggatgcc 4380
cctgccatcc ccccaacctc ctcatattgc ctctgaaccc agatggcaat ccatcccggt 4440
tctctctgag ggccacgggc ttgggtagtg gaaagggtgt ttgggaaatt gttaaatcag 4500
ttacccgtag tagagctatt tcttgtactt ctaagttttc tagaagtgga aggattgtag 4560
tcatcctgaa aatgggttta cttcaaaatc cctcagcctt gttcttcacg actgtctata 4620
ctgagagtgt catgtttcca caaagggctg acacctgagc ctggattttc actcatccct 4680
gagaagccct ttccagtagg gtgggcaatt cccaacttcc ttgccacaag cttcccaggc 4740
tttctcccct ggaaaactcc agcttgagtc ccagatacac tcatgggctg ccctgggcag 4800
ccagcattca ttgtaagttc cctctttgaa aactggtgtg tgggtgttca gttctgtgtc 4860
tggtgggtat ggacagacag taatctcctg tgatctgtgc tagctgtgag gcagctctgg 4920
aacgtgaaga gctgtttggt ttgaaccgtg aacaaaactg tgttttgagt ttagctgaca 4980

ttaaagaaaa aagttcatca cgtgactgtt aatgtaaacc tggttattaa aataactatg 5040

aaattaccaa aaaaaaaaaa aaaaaa 5066

<210> 22
<211> 2574
<212> DNA
<213> Homo sapiens

<400> 22
cctgtttaga cacatggaca acaatcccag cgctacaagg cacacagtcc gcttcttcgt 60

cctcagggtt gccagcgctt cctggaagtc ctgaagctct cgcagtgcag tgagttcatg 120

caccttcttg ccaagcctca gtctttggga tctggggagg ccgcctggtt ttcctccctc 180

cttctgcacg tctgctgggg tctcttcctc tccaggcctt gccgtccccc tggcctctct 240

tcccagctca cacatgaaga tgcacttgca aagggctctg gtggtcctgg ccctgctgaa 300

ctttgccacg gtcagcctct ctctgtccac ttgcaccacc ttggacttcg gccacatcaa 360

gaagaagagg gtggaagcca ttaggggaca gatcttgagc aagctcaggc tcaccagccc 420

ccctgagcca acggtgatga cccacgtccc ctatcaggtc ctggcccttt acaacagcac 480

ccgggagctg ctggaggaga tgcatgggga gagggaggaa ggctgcaccc aggaaaacac 540

cgagtcggaa tactatgcca aagaaatcca taaattcgac atgatccagg ggctggcgga 600

gcacaacgaa ctggctgtct gccctaaagg aattacctcc aaggttttcc gcttcaatgt 660

gtcctcagtg gagaaaaata gaaccaacct attccgagca gaattccggg tcttgcgggt 720

gcccaacccc agctctaagc ggaatgagca gaggatcgag ctcttccaga tccttcggcc 780

agatgagcac attgccaaac agcgctatat cggtggcaag aatctgccca cacggggcac 840

tgccgagtgg ctgtcctttg atgtcactga cactgtgcgt gagtggctgt tgagaagaga 900

gtccaactta ggtctagaaa tcagcattca ctgtccatgt cacacctttc agcccaatgg 960

agatatcctg gaaaacattc acgaggtgat ggaaatcaaa ttcaaaggcg tggacaatga 1020

ggatgaccat ggccgtggag atctggggcg cctcaagaag cagaaggatc accacaaccc 1080

tcatctaatc ctcatgatga ttcccccaca ccggctcgac aacccgggcc aggggggtca 1140

gaggaagaag cgggctttgg acaccaatta ctgcttccgc aacttggagg agaactgctg 1200

tgtgcgcccc ctctacattg acttccgaca ggatctgggc tggaagtggg tccatgaacc 1260

taagggctac tatgccaact tctgctcagg cccttgccca tacctccgca gtgcagacac 1320

aacccacagc acggtgctgg gactgtacaa cactctgaac cctgaagcat ctgcctcgcc 1380

ttgctgcgtg ccccaggacc tggagcccct gaccatcctg tactatgttg ggaggacccc 1440

caaagtggag cagctctcca acatggtggt gaagtcttgt aaatgtagct gagaccccac 1500

gtgcgacaga gagaggggag agagaaccac cactgcctga ctgcccgctc ctcgggaaac 1560

acacaagcaa caaacctcac tgagaggcct ggagcccaca accttcggct ccgggcaaat 1620

ggctgagatg gaggtttcct tttggaacat ttctttcttg ctggctctga gaatcacggt 1680

ggtaaagaaa gtgtgggttt ggttagagga aggctgaact cttcagaaca cacagacttt 1740

ctgtgacgca gacagagggg atggggatag aggaaaggga tggtaagttg agatgttgtg 1800

tggcaatggg atttgggcta ccctaaaggg agaaggaagg gcagagaatg gctgggtcag 1860

ggccagactg gaagacactt cagatctgag gttggatttg ctcattgctg taccacatct 1920

gctctaggga atctggatta tgttatacaa ggcaagcatt ttttttttta aagacaggtt 1980

acgaagacaa agtcccagaa ttgtatctca tactgtctgg gattaagggc aaatctatta 2040

cttttgcaaa ctgtcctcta catcaattaa catcgtgggt cactacaggg agaaaatcca 2100

ggtcatgcag ttcctggccc atcaactgta ttgggccttt tggatatgct gaacgcagaa 2160

gaaagggtgg aaatcaaccc tctcctgtct gccctctggg tccctcctct cacctctccc 2220

tcgatcatat ttccccttgg acacttggtt agacgccttc caggtcagga tgcacatttc 2280

tggattgtgg ttccatgcag ccttggggca ttatgggtct tcccccactt cccctccaag 2340

accctgtgtt catttggtgt tcctggaagc aggtgctaca acatgtgagg cattcgggga 2400

agctgcacat gtgccacaca gtgacttggc cccagacgca tagactgagg tataaagaca 2460

agtatgaata ttactctcaa aatctttgta taaataaata tttttggggc atcctggatg 2520

atttcatctt ctggaatatt gtttctagaa cagtaaaagc cttattctaa ggtg 2574

<210> 23
<211> 3046
<212> DNA
<213> Homo sapiens

<400> 23
agcatcctcc tgggcgccgc tttcgcgcgg cggcggcggc tgcggcgggg tctttctttg 60

cttaaatacc tcgttggcca gaagcgctgg taccgggggc gggttgggtc gggtcgggca 120

gtgctgcaca cctgggtttc cttgcctaga gctgtgtgtt cggggtcctt tggtccagtc 180

ggaggctgcg gagcggcggg ggttgcctgc gctgtccgcc cgggcatcct cccggtgatg 240

gaagcagccg ccgccgccgc tgcggggtcg cgctgtgccc catccaccgc tgccagagag 300

gtgggaaaat tcgccgcacg gaggccgaaa gcgagagggg ctgcgccgct atgccgggag 360

ctgagtccca tataagccgc ccccagccat cgcccccagc cggcttcgtt cccctgagcg 420

agacaggaag ctgcggtccc gagaaagcgg aggagacgtc gctggagccg ggaggcgccg 480

ggttcggcgg agcgcggagc ggggctctgg gccgcgtgaa agtttttctt cccgagccgc 540

agggcgcccg ctgcccggaa actgcccagg gataagtcgg ccgactcccc agacccctcg 600

aaggtgcggg gacccccagc ggaagcgaga gggagcgaaa tcgaggaacg agtgacagcc 660

ggacagtccg ccgggcggtg atccggggcc gctcccgggc gcgccctcgg ctccaggtcc 720

tacccggagc cgctgccatg ggagagccag ccttgggcgc tggggaccag ccgccgcgcc 780

cgcctcggag tcgcggcccg agtcccggcg ccagcagcca gcccgctgcg tccccttccc 840

gggctgcagg gctgcctccg ccgcgccgcc ggcccggatt gtgcctgtga tgagccgcag 900

cccgcagcga gctctgcccc cgggcgcgct ccctcggctg ctccaggctg cgcctgcagc 960

cgcgccgcgt gccctgctcc cgcagtggcc ccggcgccca ggacgccgct ggcccgcgtc 1020
ccctctcgga atgaaggtgt tccgtaggaa ggcgctggtg ttgtgcgcgg gctatgcact 1080
gctgctggtg ctcactatgc tcaacctcct ggactacaag tggcacaagg agccgctgca 1140
gcagtgcaac cccgatgggc cgctgggtgc cgcagcgggg gcagccggag gcagctgggg 1200
gcgcccaggg ccgcctccgg ccgggccgcc ccgtgctcat gcccgtttgg acctccgcac 1260
tccttaccgc cctcccgctg ccgccgtcgg ggcggctcct gcagccgcgg cagggatggc 1320
gggggttgcg gcccctccag gcaatggcac tcggggcacc gggggcgtcg gggacaagcg 1380
gcagctggtg tacgtgttca ccacgtggcg ctctggctcg tcgttcttcg gcgagctatt 1440
caaccagaat cccgaggtgt tctttctcta cgagccagtg tggcatgtat ggcaaaaact 1500
gtatccgggg gacgccgttt ccctgcaggg ggcagcgcgg gacatgctga gcgctcttta 1560
ccgctgcgac ctctctgtct tccagttgta tagccccgcg ggcagcgggg ggcgcaacct 1620
caccacgctg ggcatcttcg gcgcagccac caacaaggtg gtgtgctcgt caccactctg 1680
ccccgcctac cgcaaggagg tcgtggggtt ggtggacgac cgcgtgtgca agaagtgccc 1740
gccacagcgc ctggcgcgtt tcgaggagga gtgccgcaag taccgcacac tagtcataaa 1800
gggtgtgcgc gtcttcgacg tggcggtctt ggcgccactg ctgcgagacc cggccctgga 1860
cctcaaggtc atccacttgg tgcgtgatcc ccgcgcggtg gcgagttcac ggatccgctc 1920
gcgccacggc ctcatccgtg agagcctaca ggtggtgcgc agccgagacc cgcgagctca 1980
ccgcatgccc ttcttggagg ccgcgggcca caagcttggc gccaagaagg agggcgtggg 2040
cggccccgca gactaccacg ctctgggcgc tatggaggtc atctgcaata gtatggctaa 2100
gacgctgcag acagccctgc agccccctga ctggctgcag ggccactacc tggtggtgcg 2160
gtacgaggac ctggtgggag accccgtcaa gacactacgg agagtgtacg atttgtgggg 2220
actgttggtg agccccgaaa tggagcagtt tgccctgaac atgaccagtg gctcgggctc 2280
ctcctccaag cctttcgtgg tatctgcacg caatgccacg caggccgcca atgcctggcg 2340
gaccgccctc accttccagc agatcaaaca ggtggaggag ttttgctacc agcccatggc 2400
cgtcctgggc tatgagcggg tcaacagccc tgaggaggtc aaagacctca gcaagaccct 2460
gcttcggaag ccccgtctct aaaaggggtt cccaggagac ctgattccct gtggtgatac 2520
ctataaagag gatcgtagtg tgtttaaata aacacagtcc agactcaaac ggaggaagcc 2580
cacatattct attatagata tataaataat cacacacaca cttgctgtca atgttttgag 2640
tcagtgcatt tcaaggaaca gccacaaaat acacacccct aagaaaaggc aagacttgaa 2700
cgttctgacc aggtgcccct cttcttcttt gccttctctt gtcctctttc tcctatttct 2760
taccctgtcc tccacctgcc ttccattttg aagtgggatg ttaatgaaat caagttccag 2820
taacccaaat cttgtttaca aaatattcgt ggtatctgtg aacatgttaa gagtaatttg 2880
gatgtggggg tgggggtgga gaaaggggaa gtggtccaga aacaaaaagc cccattgggc 2940
atgataagcc gaggaggcat tcttctaaag cagacttttg tgtaaaaagc aaaggttaca 3000

tgtgagtatt aataaagaag ataataaata atattctttt taaaaa 3046

<210> 24
<211> 2704
<212> DNA
<213> Homo sapiens

<400> 24
gggagaaacg ttctcactcg ctctctgctc gctgcgggcg ctccccgccc tctgctgcca 60

gaaccttggg gatgtgccta gacccggcgc agcacacgtc cgggccaacc gcgagcagaa 120

caaacctttg gcgggcggcc aggaggctcc ctcccagcca ccgcccccct ccagcgcctt 180

tttttccccc catacaatac aagatcttcc ttcctcagtt cccttaaagc acagcccagg 240

gaaacctcct cacagttttc atccagccac gggccagcat gtctgggggc aaatacgtag 300

actcggaggg acatctctac accgttccca tccgggaaca gggcaacatc tacaagccca 360

acaacaaggc catggcagac gagctgagcg agaagcaagt gtacgacgcg cacaccaagg 420

agatcgacct ggtcaaccgc gaccctaaac acctcaacga tgacgtggtc aagattgact 480

ttgaagatgt gattgcagaa ccagaaggga cacacagttt tgacggcatt tggaaggcca 540

gcttcaccac cttcactgtg acgaaatact ggttttaccg cttgctgtct gccctctttg 600

gcatcccgat ggcactcatc tggggcattt acttcgccat tctctctttc ctgcacatct 660

gggcagttgt accatgcatt aagagcttcc tgattgagat tcagtgcatc agccgtgtct 720

attccatcta cgtccacacc gtctgtgacc cactctttga agctgttggg aaaatattca 780

gcaatgtccg catcaacttg cagaaagaaa tataaatgac atttcaagga tagaagtata 840

cctgattttt tttcctttta attttcctgg tgccaatttc aagttccaag ttgctaatac 900

agcaacaatt tatgaattga attatcttgg ttgaaaataa aaagatcact ttctcagttt 960

tcataagtat tatgtctctt ctgagctatt tcatctattt ttggcagtct gaatttttaa 1020

aacccattta aatttttttc cttacctttt tatttgcatg tggatcaacc atcgctttat 1080

tggctgagat atgaacatat tgttgaaagg taatttgaga gaaatatgaa gaactgagga 1140

ggaaaaaaaa aaaaaagaaa agaaccaaca acctcaactg cctactccaa aatgttggtc 1200

attttatgtt aagggaagaa ttccagggta tggccatgga gtgtacaagt atgtgggcag 1260

attttcagca aactcttttc ccactgttta aggagttagt ggattactgc cattcacttc 1320

ataatccagt aggatccagt gatccttaca agttagaaaa cataatcttc tgccttctca 1380

tgatccaact aatgccttac tcttcttgaa attttaacct atgatatttt ctgtgcctga 1440

atatttgtta tgtagataac aagacctcag tgccttcctg tttttcacat tttccttttc 1500

aaatagggtc taactcagca actcgcttta ggtcagcagc ctccctgaag accaaaatta 1560

gaatatccat gacctagttt tccatgcgtg tttctgactc tgagctacag agtctggtga 1620

agctcacttc tgggcttcat ctggcaacat ctttatccgt agtgggtatg gttgacacta 1680

gcccaatgaa atgaattaaa gtggaccaat agggctgagc tctctgtggg ctggcagtcc 1740

tggaagccag ctttccctgc ctctcatcaa ctgaatgagg tcagcatgtc tattcagctt 1800

cgtttatttt caagaataat cacgctttcc tgaatccaaa ctaatccatc accggggtgg 1860

tttagtggct caacattgtg ttcccatttc agctgatcag tgggcctcca aggaggggct 1920

gtaaaatgga ggccattgtg tgagcctatc agagttgctg caaacctgac ccctgctcag 1980

taaagcactt gcaaccgtct gttatgctgt gacacatggc ccctccccct gccaggagct 2040

ttggacctaa tccaagcatc cctttgccca gaaagaagat gggggaggag gcagtaataa 2100

aaagattgaa gtattttgct ggaataagtt caaattcttc tgaactcaaa ctgaggaatt 2160

tcacctgtaa acctgagtcg tacagaaagc tgcctggtat atccaaaagc tttttattcc 2220

tcctgctcat attgtgattc tgcctttggg gacttttctt aaaccttcag ttatgatttt 2280

tttttcatac acttattgga actctgcttg atttttgcct cttccagtct tcctgacact 2340

ttaattacca acctgttacc tactttgact ttttgcattt aaaacagaca ctggcatgga 2400

tatagtttta cttttaaact gtgtacataa ctgaaaatgt gctatactgc atacttttta 2460

aatgtaaaga tatttttatc tttatatgaa gaaaatcact taggaaatgg ctttgtgatt 2520

caatctgtaa actgtgtatt ccaagacatg tctgttctac atagatgctt agtccctcat 2580

gcaaatcaat tactggtcca aaagattgct gaaattttat atgcttactg atatatttta 2640

caatttttta tcatgcatgt cctgtaaagg ttacaagcct gcacaataaa aatgtttaac 2700

ggtt 2704

<210> 25
<211> 2254
<212> DNA
<213> Homo sapiens

<400> 25
aatcgaaagt agactctttt ctgaagcatt tcctgggatc agcctgacca cgctccatac 60

tgggagaggc ttctgggtca aaggaccagt ctgcagaggg atcctgtggc tggaagcgag 120

gaggctccac acggccgttg cagctaccgc agccaggatc tgggcatcca ggcacggcca 180

tgacccctcc gaggctcttc tgggtgtggc tgctggttgc aggaacccaa ggcgtgaacg 240

atggtgacat gcggctggcc gatgggggcg ccaccaacca gggccgcgtg gagatcttct 300

acagaggcca gtggggcact gtgtgtgaca acctgtggga cctgactgat gccagcgtcg 360

tctgccgggc cctgggcttc gagaacgcca cccaggctct gggcagagct gccttcgggc 420

aaggatcagg ccccatcatg ctggacgagg tccagtgcac gggaaccgag gcctcactgg 480

ccgactgcaa gtccctgggc tggctgaaga gcaactgcag gcacgagaga gacgctggtg 540

tggtctgcac caatgaaacc aggagcaccc acaccctgga cctctccagg gagctctcgg 600

aggcccttgg ccagatcttt gacagccagc ggggctgcga cctgtccatc agcgtgaatg 660

tgcagggcga ggacgccctg ggcttctgtg gccacacggt catcctgact gccaacctgg 720

aggcccaggc cctgtggaag gagccgggca gcaatgtcac catgagtgtg gatgctgagt 780

gtgtgcccat ggtcagggac cttctcaggt acttctactc ccgaaggatt gacatcaccc 840

tgtcgtcagt caagtgcttc cacaagctgg cctctgccta tggggccagg cagctgcagg 900
gctactgcgc aagcctcttt gccatcctcc tcccccagga cccctcgttc cagatgcccc 960
tggacctgta tgcctatgca gtggccacag gggacgccct gctggagaag ctctgcctac 1020
agttcctggc ctggaacttc gaggccttga cgcaggccga ggcctggccc agtgtcccca 1080
cagacctgct ccaactgctg ctgcccagga gcgacctggc ggtgcccagc gagctggccc 1140
tactgaaggc cgtggacacc tggagctggg gggagcgtgc ctcccatgag gaggtggagg 1200
gcttggtgga gaagatccgc ttccccatga tgctccctga ggagctcttt gagctgcagt 1260
tcaacctgtc cctgtactgg agccacgagg ccctgttcca gaagaagact ctgcaggccc 1320
tggaattcca cactgtgccc ttccagttgc tggcccggta caaaggcctg aacctcaccg 1380
aggataccta caagccccgg atttacacct cgcccacctg gagtgccttt gtgacagaca 1440
gttcctggag tgcacggaag tcacaactgg tctatcagtc cagacggggg cctttggtca 1500
aatattcttc tgattacttc caagccccct ctgactacag atactacccc taccagtcct 1560
tccagactcc acaacacccc agcttcctct tccaggacaa gagggtgtcc tggtccctgg 1620
tctacctccc caccatccag agctgctgga actacggctt ctcctgctcc tcggacgagc 1680
tccctgtcct gggcctcacc aagtctggcg gctcagatcg caccattgcc tacgaaaaca 1740
aagccctgat gctctgcgaa gggctcttcg tggcagacgt caccgatttc gagggctgga 1800
aggctgcgat tcccagtgcc ctggacacca acagctcgaa gagcacctcc tccttcccct 1860
gcccggcagg gcacttcaac ggcttccgca cggtcatccg ccccttctac ctgaccaact 1920
cctcaggtgt ggactagacg cgtggccaag ggtggtgaga accggagaac cccaggacgc 1980
cctcactgca ggctcccctc ctcggcttcc ttcctctctg caatgacctt caacaaccgg 2040
ccaccagatg tcgccctact cacctgaggc tcagcttcaa gaaattactg gaaggcttcc 2100
actagggtcc accaggagtt ctcccaccac ctcaccagtt tccaggtggt aagcaccagg 2160
aggccctcga ggttgctctg gatcccccca cagcccctgg tcagtctgcc cttgtcactg 2220
gtctgaggtc attaaaatta cattgaggtt ccta 2254

<210> 26
<211> 2769
<212> DNA
<213> Homo sapiens

<400> 26
cgacgccaag gggagggggc tgacctgtgc ttggtccacc ccaggtaggg gctgagagag 60
gcttgaggtg gaagtggggg tcgggcactc tgacctggtc gaggaggggc tagggtttga 120
accggggaca gagtctaggt gagctggggc ttgggagcta ttagcgtaga ggatccgggt 180
tcggttgctc tggcgagggc tccagcatca caggcggcgg ctgcgggcgc agagcggaga 240
tgcagcggct tggggccacc ctgctgtgcc tgctgctggc ggcggcggtc cccacggccc 300
ccgcgcccgc tccgacggcg acctcggctc cagtcaagcc cggcccggct ctcagctacc 360

cgcaggagga ggccaccctc aatgagatgt tccgcgaggt tgaggaactg atggaggaca 420
cgcagcacaa attgcgcagc gcggtggaag agatggaggc agaagaagct gctgctaaag 480
catcatcaga agtgaacctg gcaaacttac ctcccagcta tcacaatgag accaacacag 540
acacgaaggt tggaaataat accatccatg tgcaccgaga aattcacaag ataaccaaca 600
accagactgg acaaatggtc ttttcagaga cagttatcac atctgtggga gacgaagaag 660
gcagaaggag ccacgagtgc atcatcgacg aggactgtgg gcccagcatg tactgccagt 720
ttgccagctt ccagtacacc tgccagccat gccggggcca gaggatgctc tgcacccggg 780
acagtgagtg ctgtggagac cagctgtgtg tctggggtca ctgcaccaaa atggccacca 840
ggggcagcaa tgggaccatc tgtgacaacc agagggactg ccagccgggg ctgtgctgtg 900
ccttccagag aggcctgctg ttccctgtgt gcacacccct gcccgtggag ggcgagcttt 960
gccatgaccc cgccagccgg cttctggacc tcatcacctg ggagctagag cctgatggag 1020
ccttggaccg atgcccttgt gccagtggcc tcctctgcca gccccacagc cacagcctgg 1080
tgtatgtgtg caagccgacc ttcgtgggga gccgtgacca agatggggag atcctgctgc 1140
ccagagaggt ccccgatgag tatgaagttg gcagcttcat ggaggaggtg cgccaggagc 1200
tggaggacct ggagaggagc ctgactgaag agatggcgct gagggagcct gcggctgccg 1260
ccgctgcact gctgggaggg gaagagattt agatctggac caggctgtgg gtagatgtgc 1320
aatagaaata gctaatttat ttccccaggt gtgtgcttta ggcgtgggct gaccaggctt 1380
cttcctacat cttcttccca gtaagtttcc cctctggctt gacagcatga ggtgttgtgc 1440
atttgttcag ctcccccagg ctgttctcca ggcttcacag tctggtgctt gggagagtca 1500
ggcagggtta aactgcagga gcagtttgcc acccctgtcc agattattgg ctgctttgcc 1560
tctaccagtt ggcagacagc cgtttgttct acatggcttt gataattgtt tgaggggagg 1620
agatggaaac aatgtggagt ctccctctga ttggttttgg ggaaatgtgg agaagagtgc 1680
cctgctttgc aaacatcaac ctggcaaaaa tgcaacaaat gaattttcca cgcagttctt 1740
tccatgggca taggtaagct gtgccttcag ctgttgcaga tgaaatgttc tgttcaccct 1800
gcattacatg tgtttattca tccagcagtg ttgctcagct cctacctctg tgccagggca 1860
gcattttcat atccaagatc aattccctct ctcagcacag cctggggagg gggtcattgt 1920
tctcctcgtc catcagggat ctcagaggct cagagactgc aagctgcttg cccaagtcac 1980
acagctagtg aagaccagag cagtttcatc tggttgtgac tctaagctca gtgctctctc 2040
cactacccca caccagcctt ggtgccacca aaagtgctcc ccaaaaggaa ggagaatggg 2100
atttttcttt tgaggcatgc acatctggaa ttaaggtcaa actaattctc acatccctct 2160
aaaagtaaac tactgttagg aacagcagtg ttctcacagt gtggggcagc cgtccttcta 2220
atgaagacaa tgatattgac actgtccctc tttggcagtt gcattagtaa ctttgaaagg 2280
tatatgactg agcgtagcat acaggttaac ctgcagaaac agtacttagg taattgtagg 2340
gcgaggatta taaatgaaat ttgcaaaatc acttagcagc aactgaagac aattatcaac 2400

cacgtggaga aaatcaaacc gagcagggct gtgtgaaaca tggttgtaat atgcgactgc 2460

gaacactgaa ctctacgcca ctccacaaat gatgttttca ggtgtcatgg actgttgcca 2520

ccatgtattc atccagagtt cttaaagttt aaagttgcac atgattgtat aagcatgctt 2580

tctttgagtt ttaaattatg tataaacata agttgcattt agaaatcaag cataaatcac 2640

ttcaactgct cttctgtagt tcttggattt cttttccctt ttgactttga ataaatgtaa 2700

aatcctttca gccagaaaaa gtaaaataga aacaacctgt attaaaaatc ttccatagaa 2760

aaaaaaaaa 2769

<210> 27
<211> 2808
<212> DNA
<213> Homo sapiens

<400> 27
cggcatgaga ggccagcctg ccagggaaat ccaggaatct gcaacaaaaa cgatgacagt 60

ctgaaatact ctctggtgcc aacctccaaa ttctcgtctg tcacttcaga cccccactag 120

ttgacagagc agcagaatat caactccagt agacttgaat gtgcctctgg gcaaagaagc 180

agagctaacg aggaaaggga tttaaagagt ttttcttggg tgtttgtcaa acttttattc 240

cctgtctgtg tgcagagggg attcaacttc aattttctgc agtggctctg ggtccagccc 300

cttacttaaa gatctggaaa gcatgaagac tgggcctttt ttcctatgtc tcttgggaac 360

tgcagctgca atcccgacaa atgcaagatt attatctgat cattccaaac caactgctga 420

aacggtagca cctgacaaca ctgcaatccc cagtttatgg gctgaagctg aagaaaatga 480

aaaagaaaca gcagtatcca cagaagacga ttcccaccat aaggctgaaa aatcatcagt 540

actaaagtca aaagaggaaa gccatgaaca gtcagcagaa cagggcaaga gttctagcca 600

agagctggga ttgaaggatc aagaggacag tgatggtcac ttaagtgtga atttggagta 660

tgcaccaact gaaggtacat tggacataaa agaagatatg attgagcctc aggagaaaaa 720

actctcagag aacactgatt ttttggctcc tggtgttagt tccttcacag attctaacca 780

acaagaaagt atcacaaaga gagaggaaaa ccaagaacaa cctagaaatt attcacatca 840

tcagttgaac aggagcagta aacatagcca aggcctaagg gatcaaggaa accaagagca 900

ggatccaaat atttccaatg gagaagagga agaagaaaaa gagccaggtg aagttggtac 960

ccacaatgat aaccaagaaa gaaagacaga attgcccagg gagcatgcta acagcaagca 1020

ggaggaagac aatacccaat ctgatgatat tttggaagag tctgatcaac caactcaagt 1080

aagcaagatg caggaggatg aatttgatca gggtaaccaa gaacaagaag ataactccaa 1140

tgcagaaatg gaagaggaaa atgcatcgaa cgtcaataag cacattcaag aaactgaatg 1200

gcagagtcaa gagggtaaaa ctggcctaga agctatcagc aaccacaaag agacagaaga 1260

aaagactgtt tctgaggctc tgctcatgga acctactgat gatggtaata ccacgcccag 1320

aaatcatgga gttgatgatg atggcgatga tgatggcgat gatggcggca ctgatggccc 1380

caggcacagt gcaagtgatg actacttcat cccaagccag gcctttctgg aggccgagag 1440

agctcaatcc attgcctatc acctcaaaat tgaggagcaa agagaaaaag tacatgaaaa 1500

tgaaaatata ggtaccactg agcctggaga gcaccaagag gccaagaaag cagagaactc 1560

atcaaatgag gaggaaacgt caagtgaagg caacatgagg gtgcatgctg tggattcttg 1620

catgagcttc cagtgtaaaa gaggccacat ctgtaaggca gaccaacagg gaaaacctca 1680

ctgtgtctgc caggatccag tgacttgtcc tccaacaaaa ccccttgatc aagtttgtgg 1740

cactgacaat cagacctatg ctagttcctg tcatctattc gctactaaat gcagactgga 1800

ggggaccaaa aaggggcatc aactccagct ggattatttt ggagcctgca aatctattcc 1860

tacttgtacg gactttgaag tgattcagtt tcctctacgg atgagagact ggctcaagaa 1920

tatcctcatg cagctttatg aagccaactc tgaacatgct ggttatctaa atgagaagca 1980

gagaaataaa gtcaagaaaa tttacctgga tgaaaagagg cttttggctg gggaccatcc 2040

cattgatctt ctcttaaggg actttaagaa aaactaccac atgtatgtgt atcctgtgca 2100

ctggcagttt agtgaacttg accaacaccc tatggataga gtcttgacac attctgaact 2160

tgctcctctg cgagcatctc tggtgcccat ggaacactgc ataacccgtt tctttgagga 2220

gtgtgacccc aacaaggata agcacatcac cctgaaggag tggggccact gctttggaat 2280

taaagaagag gacatagatg aaaatctctt gttttgaacg aagattttaa agaactcaac 2340

tttccagcat cctcctctgt tctaaccact tcagaaatat atgcagctgt gatacttgta 2400

gatttatatt tagcaaaatg ttagcatgta tgacaagaca atgagagtaa ttgcttgaca 2460

acaacctatg caccaggtat ttaacattaa ctttggaaac aaaaatgtac aattaagtaa 2520

agtcaacata tgcaaaatac tgtacattgt gaacagaagt ttaattcata gtaatttcac 2580

tctctgcatt gacttatgag ataattaatg attaaactat taatgataaa aataatgcat 2640

ttgtattgtt cataatatca tgtgcacttc aagaaaatgg aatgctactc ttttgtggtt 2700

tacgtgtatt attttcaata tcttaatacc ctaataaaga gtccataaaa atccaaaaaa 2760

aaaaaaaaaa aaaaaaaaaa aaaaaaaaaa aaaaaaaaaa aaaaaaaa 2808

<210> 28
<211> 404
<212> DNA
<213> Homo sapiens

<400> 28
gcctgccctg acttctcata tcttgcctag gaactccagg cttgtcttgg ctccaaatgg 60

atcccaactg ctcctgcacc acaggtggct cctgtgcctg cgccggctcc tgcaagtgca 120

aagagtgcaa atgtacctcc tgcaagaagt gctgctgctc ttgctgcccc gtgggctgtg 180

ccaagtgtgc ccagggctgt gtctgcaaag gctcatcaga gaagtgccgc tgctgtgcct 240

gatgttggga gagccctgct cccagacata aatagagcaa ccagtactaa cctggatttt 300

tttttttaac taccctgacc ggtttgctac attctttttt ctattcaata tgtgaaagac 360

aataaaacac ttttgacttg aaaaaaaaaa aaaaaaaaaa aaaa 404

<210> 29
<211> 4124
<212> DNA
<213> Homo sapiens

<400> 29
gcacagactc aaagccccgc gggcgagctc agcagcccgg agcgaccgcg gccccgccgc 60
ctcccccgcg agtcccggcg atgcggcccg gcctgtgagc ggccggcgac cctgggacgc 120
cccgccgcac aactacctca acgccgtgcc gcccccctcc cgcccccagg gaatctctgg 180
agattggttc accttttgtg gtcctgaccc cttctgtcct cactgtacct tgaagtccta 240
gagtccaata aaatcgctgc ctcccagctg tttggatcac agagaggttt ttgcactgcc 300
atagggcgcc cccgtgaggc gcttcgcccc ccaccatgtt ccagcgcctc tccagcctct 360
tcttcagcac cccctcgccc cccgaagacc ccgactgccc ccgcgccttc gtgtcggagg 420
aggatgaagt ggacggctgg ctcatcattg acctgccgga cagctacgcg gctccaccca 480
gccccggggc cgcccctgcc cccgcgggcc gccctccgcc cgcgccctcc ttgatggacg 540
agagctggtt tgttacccct cccgcctgtt ttacggcaga ggggcctgga ctcggtcccg 600
cccgcctcca gagcagtccc ctggaggacc tcctcatcga gcaccccagc atgtccgttt 660
acgtcaccgg cagcaccata gtgctagagc ccgggtcccc ttccccgctc ccggacgcgg 720
ccctgcctga cggcgacctc agcgaagggg aattgacgcc cgcccgccgc gagccgcggg 780
ccgcgcgcca cgccgctcct ctcccagcgc gggcggcgct gctggagaag gcgggccagg 840
tgcggcggct gcagcgggcc cggcagcggg cagagcgcca cgcgctgagc gccaaggcgg 900
tgcagcggca gaaccgagcc cgcgagagcc gtccgcgccg gtccaagaac cagagcagct 960
tcatctacca gccgtgccag cgccagttca actactgagc gtccaccggc cgcgccacga 1020
accccttgcc gatcccgatc cctgtcgggc tcctccgact cctcgggctg gacaccgaaa 1080
cctcccttct taaagcgtgt gaggttgggt gatagccgtt ccttccccga caccctcaat 1140
ttccccatct ctgatcctct aatctgcctc tgaacccatt cacccttcac cctcactcct 1200
ggtccccata cccagcatct aatcatccat gccccctact cctggcccct ccatcctttc 1260
ttctctggtc cccatccctg tctctccctt tcacccttgc cctccagtcc tctacctctg 1320
gcctgcccct atttctgaaa gcttcttcca gtccctgatc tggctcattc cccaccttca 1380
actcccacct tacatgtctc acactatccc atggttggca ttacactcac tcctgttccc 1440
ttattcttca ttcccagtaa ttccctacca aatggtgggg accctgagcc cagctctgac 1500
caggtagagc ctgtgcagcc tgggctgctg tcattgccct ccagtaaggg ctcagggttt 1560
tgcttttagt ctccccttcc ttctgccttg ggggcggtac tctgtggagc tgcttaggcc 1620
tggaaaggta cagtatgtag aagaggactg tgagacgtga gttagaggga gaagatggag 1680
ggaatctagg gaacgaggca gcctattgga gatgcggaca ggacagacac gttgagaagc 1740
tgcagggagc agggcaccaa gggagtgtgc actgtgcttg ctcagagagg ccaaaccctg 1800
ctcccaggct gaagcctgga gtctgccccc acttcccttt tctataatcc acccttctgc 1860

aggccctgaa atctgaaggg cttagttagt accttgccac tctaccccca acacgtcacc 1920
cgagtagaag ctgggcaagg ccctaccatc ctggccgtct gttcacaagc ccaaggtgct 1980
agaactagct taggagacat gcaggccaca gggcttctag gcagggaaag ggcacacacc 2040
ctaggtcagc gtgcagagca gtgatgctgg aggacacacc atggggtgaa gccatcccaa 2100
ggctgacagc tcagtcttca ccttgcctct ggccttgtat ttcacaccct gctcagtatg 2160
gctagccagc agtcctgagt aggagtccag gactcccact gtctcaatct gcaaattgtt 2220
cctatccagg ttcagggcct tcagggggcc tcttttcatt ttctcccaca ggcctctctt 2280
tctttctagg ttgctgggga gaaatgggta ccctatgatc cccctcccct tctcctccag 2340
taaatacctg gaagagggaa cctgaatccc tgggggagac agaaggggca ggggccacca 2400
gcctcccctt cttgtggtga gactgaattt tgggctcaga caccagcaac agcctcttgg 2460
gatgccctga gttgcttccc atttcctctt tctagccgtc cttttctagt gtgtgctcac 2520
tcttcctagg aacttttaag acttcttggt acctatgaac ataggtcctc ccctcacccc 2580
aactctaggt ttccaggcct cacagccaag ctgaagcttg gagaaaactc tgcattccca 2640
tgagggcaaa ggcagctgcc ctccctgacc ctatagcccc aggcctcatg gggggtatgt 2700
ggggaaggga tggggtatcc ccatggatgc tgggatgagg acagaggaag acctgatggg 2760
gtctcctatt ccagggaata agccaaatta acactaaaaa cggatcaaag ctcccacgcc 2820
agtccactag ggccccagta gttgacagcc ttgctcctct cccaagttct ccttcagaac 2880
ctagttgctt ttattcttcc agctaccact tgggcacttc acagccagcc tagggtcttc 2940
ggcacctcca agagctgaat ctccctccaa cccttcttgc ctactcctca ctgccagctg 3000
ggacctaggc tcagtcctgt gtggtgccca tgatccttct ggtgggggaa gagtttaagt 3060
tatagggcat ttggctcaaa ttttaaaagg ccttttgttt acctatattt ctggaggctc 3120
ctgtattcta gaacccaatc tctcacctgc ttggttgcaa ggctcatatt tttttgtacc 3180
tttcctatag attctgtagc atttgagtgt ggcaatattt taattgtgta tagatttcta 3240
agaaccaaca ctactcagtc tcctgctagt ctgactcctg aagcatcagc ccttgtcata 3300
ctgtattgac tgtgtacgtg cctttcacct tgagcatgct tcaggatttt tttttaaacc 3360
acagaacttg aatacatgag ggaaccagag ttcaaagtcc tatgcaacct taggaggggg 3420
ttagagagtc tgttttgatt gatgttttct gaggccctag aggagtttgt atcaatttgt 3480
gagtattaat gtcagtacta ccagcacttt gccaaaactg tcagagggac ccgtttctag 3540
agtgagtccc agttacatca aacagtgact tccagttatt ccccagtaag tctgagtggt 3600
tccttcaagc tgggtgtctt tccagccttt gccagtctag ccccagcagg gcaccgtgta 3660
tgaatgcagt ttggtgctgt tttagagtat gcctgctccc cagccccctg cctggaaccc 3720
tctgagcaac ttgctctgac ctataatgtc ttaggtgcaa cacggacccc accagagctc 3780
ttggataccc ccctagatcc atgtggcttt atgtgagggg actgaatgca gacacaccat 3840
agcccccttc tactactttc cctctcgccc tgccacctag ttccacatgg aaccaacaag 3900

ttgagtgcat ccctgttggg tgttttgtgt tgagactggc tgaaatgagg agactttgac 3960

catgtgacgt gtcaacagac tcaaggagac aaccacctca actgggtcat gtggcatgcc 4020

tgtgtatgtg tgtaacagaa ttctgattgt tagactgtaa tgctattcct ctatgggaga 4080

aaaaaattaa tataaagaaa aacaaataaa aatatattta aagc 4124

<210> 30
<211> 1454
<212> DNA
<213> Homo sapiens

<400> 30
agcggagata agattcagag gttgaggatg gggtgtcctg gtggactgaa ggtagcctac 60

tagctgtaca tgggtgacaa cttgaaactt cagaaccctg aagtttaaaa aattctaaag 120

gtgcctgtca tctcagagag tgacgtaagt gttctttctt tatttggggg aagtccagga 180

gaacatatta cagacatgtt taatccacat gcattagatg ttcctgctgt aatttttgac 240

aatggttcag gactctgcaa agcaggcctg tctggagaga ttggaccccg ccatgtcatc 300

agctccgtct tgggacattg taaattcaat gtgcctttag caagacttaa tcagaagtac 360

ttcgtggggc aagaagccct gtacaagtat gaggccctac atttgcacta ccccattgag 420

cgtggactgg taacaggatg ggatgacatg gagaaactct ggaaacatct ctttgagcgg 480

gagcttggag taaaacccag ccaacagcct gtacttatga ccgagccctc tttgaatcct 540

agggaaattc gagaaaagct agcagaaatg atgtttgaga ccttcagtgt gcctggtttc 600

tacctgtcta atcatgcggt ggcagcgctc tatgcctctg cctgtgtcac aggcctggtg 660

gtggacagtg gagatggggt cacttgcact gtccccatct ttgagggtta ctccctgcct 720

cacgcagtca ccaaactctg tatggcaggg agggacatca cagagcacct cacccggctc 780

ctctttgcta gcgggtttaa cttcccttgc atactcaaca aggccgtggt aaataacatc 840

aaagagaagt tgtgctacat cgccttggag ccagagaaag agctacgcaa gagccgggga 900

gaggtcctgg gagcatacag actgccagat ggacatgtca tccactttgg ggatgagctg 960

taccaagtgc ccgaggttct ttttgcacct gaccagctgg gcatccacag cccaggactc 1020

tcaaaaatgg tctccagcag catcatgaag tgtgacactg acatccagaa taaactttat 1080

gcagacattg tactctccgg gggcaccact ctcctccctg ggctggagga aaggctcatg 1140

aaggaagtgg aacagctggc ttccaaaggt actcccatca agatcacagc ttctcctgat 1200

agatgcttct ctgcatggat tggtgcatcc atcatgacct ctatgagcag tttcaagcag 1260

atgtgggtca cctcggcaga cttcaaggag tatgggacat ctgtggttca aagaaggtgc 1320

ttttaaagat ccttgagcag aggagacatc ttgaagtgtc agattacagg agtaccagtg 1380

ggagatggca ttttcttctg ggcttcagca tgatgttcaa taaaagtttt gccattccaa 1440

aaaaaaaaaa aaaa 1454

<210> 31

<211>  3942
<212>  DNA
<213>  Homo sapiens

<400>  31
gggtgattca gcgcccggcg aggcggaagc ggccgcaaga ggaggagggg agagcccgtc      60

cgcgcctggg ctcccggggt ggcacgagcc cgcggccgga gtgcgaggcg gaggcgagga     120

ggccgcgggg acgggaggcg aggccggccg ggcccccgaa gccatggaga acgcgcacac     180

caagacggtg gaggaggtgc tgggccactt cggcgtcaac gagagtacgg ggctgagcct     240

ggaacaggtc aagaagctta aggagagatg gggctccaac gagttaccgg ctgaagaagg     300

aaaaaccttg ctggaacttg tgattgagca gtttgaagac ttgctagtta ggattttatt     360

actggcagca tgtatatctt ttgttttggc ttggtttgaa gaaggtgaag aaacaattac     420

agcctttgta gaaccttttg taattttact catattagta gccaatgcaa ttgtgggtgt     480

atggcaggaa agaaatgctg aaaatgccat cgaagccctt aaggaatatg agcctgaaat     540

gggcaaagtg tatcgacagg acagaaagag tgtgcagcgg attaaagcta aagacatagt     600

tcctggtgat attgtagaaa ttgctgttgg tgacaaagtt cctgctgata taaggttaac     660

ttccatcaaa tctaccacac taagagttga ccagtcaatt ctcacaggtg aatctgtctc     720

tgtcatcaag cacactgatc ccgtccctga cccacgagct gtcaaccaag ataaaaagaa     780

catgctgttt tctggtacaa acattgctgc tgggaaagct atgggagtgg tggtagcaac     840

tggagttaac accgaaattg gcaagatccg ggatgaaatg gtggcaacag aacaggagag     900

aacacccctt cagcaaaaac tagatgaatt tggggaacag ctttccaaag tcatctccct     960

tatttgcatt gcagtctgga tcataaatat tgggcacttc aatgacccgg ttcatggagg    1020

gtcctggatc agaggtgcta tttactactt taaaattgca gtggccctgg ctgtagcagc    1080

cattcctgaa ggtctgcctg cagtcatcac cacctgcctg gctcttggaa ctcgcagaat    1140

ggcaaagaaa aatgccattg ttcgaagcct cccgtctgtg gaaacccttg gttgtacttc    1200

tgttatctgc tcagacaaga ctggtacact tacaacaaac cagatgtcag tctgcaggat    1260

gttcattctg gacagagtgg aaggtgatac ttgttccctt aatgagttta ccataactgg    1320

atcaacttat gcacctattg gagaagtgca taaagatgat aaaccagtga attgtcacca    1380

gtatgatggt ctggtagaat tagcaacaat ttgtgctctt tgtaatgact ctgctttgga    1440

ttacaatgag gcaaagggtg tgtatgaaaa agttggagaa gctacagaga ctgctctcac    1500

ttgcctagta gagaagatga atgtatttga taccgaattg aagggtcttt ctaaaataga    1560

acgtgcaaat gcctgcaact cagtcattaa acagctgatg aaaaaggaat tcactctaga    1620

gttttcacgt gacagaaagt caatgtcggt ttactgtaca ccaaataaac caagcaggac    1680

atcaatgagc aagatgtttg tgaagggtgc tcctgaaggt gtcattgaca ggtgcaccca    1740

cattcgagtt ggaagtacta aggttcctat gacctctgga gtcaaacaga agatcatgtc    1800

tgtcattcga gagtggggta gtggcagcga cacactgcga tgcctggccc tggccactca    1860

tgacaaccca ctgagaagag aagaaatgca ccttgaggac tctgccaact ttattaaata    1920

tgagaccaat ctgaccttcg ttggctgcgt gggcatgctg gatcctccga gaatcgaggt 1980
ggcctcctcc gtgaagctgt gccggcaagc aggcatccgg gtcatcatga tcactgggga 2040
caacaagggc actgctgtgg ccatctgtcg ccgcatcggc atcttcgggc aggatgagga 2100
cgtgacgtca aaagctttca caggccggga gtttgatgaa ctcaacccct ccgcccagcg 2160
agacgcctgc ctgaacgccc gctgttttgc tcgagttgaa ccctcccaca agtctaaaat 2220
cgtagaattt cttcagtctt ttgatgagat tacagctatg actggcgatg gcgtgaacga 2280
tgctcctgct ctgaagaaag ccgagattgg cattgctatg ggctctggca ctgcggtggc 2340
taaaaccgcc tctgagatgg tcctggcgga tgacaacttc tccaccattg tggctgccgt 2400
tgaggagggg cgggcaatct acaacaacat gaaacagttc atccgctacc tcatctcgtc 2460
caacgtcggg gaagttgtct gtattttcct gacagcagcc cttggatttc ccgaggcttt 2520
gattcctgtt cagctgctct gggtcaatct ggtgacagat ggcctgcctg ccactgcact 2580
ggggttcaac cctcctgatc tggacatcat gaataaacct ccccggaacc caaaggaacc 2640
attgatcagc gggtggctct tttccgttta cttggctatt ggctgttacg tcggcgctgc 2700
taccgtgggt gctgctgcat ggtggttcat tgctgctgac ggtggtccaa gagtgtcctt 2760
ctaccagctg agtcatttcc tacagtgtaa agaggacaac ccggactttg aaggcgtgga 2820
ttgtgcaatc tttgaatccc catacccgat gacaatggcg ctctctgttc tagtaactat 2880
agaaatgtgt aacgccctca acagcttgtc cgaaaaccag tccttgctga ggatgccccc 2940
ctgggagaac atctggctcg tgggctccat ctgcctgtcc atgtcactcc acttcctgat 3000
cctctatgtc gaacccttgc cactcatctt ccagatcaca ccgctgaacg tgacccagtg 3060
gctgatggtg ctgaaaatct ccttgcccgt gattctcatg gatgagacgc tcaagtttgt 3120
ggcccgcaac tacctggaac ctgcaatact ggagtaaccg cttcctaaac cattttgcag 3180
aaatgtaagg gtgttcggtt gcgtgcatgt gcgtttttag caacacatct accaaccctg 3240
tgcatgactg atgttgggga aaaagaaaag taaaaaactt cccaactcac tttgtgttat 3300
gtggaggaaa tgtgtattac caatggggtt gttagctttt aaatcaaaat actgattaca 3360
gatgtacaat ttagcttaat cagaaagcct ctccagagaa gtttggtttc tttgctgcaa 3420
gaggaatgag gctctgtaac cttatctaag aacttggaag ccgtcagcca agtcgccaca 3480
tttctctgca aaatgtcata gcttatataa atgtacagta ttcaattgta atgcatgcct 3540
tcggttgtaa gtagccagat ccctctccag tgacattgga acatgctact ttttaattgg 3600
ccctgtacag tttgcttatt tataaattca ttaaaaacac tacaggtgtt gaatggttaa 3660
aatgtaggcc tccagttcat tttcagttat tttctgagtg tgcagacagc tatttcgcac 3720
tgtattaaat gtaacttatt taatgaaatc agaagcagta gacagatgtt ggtgcaatac 3780
aaatattgtg atgcatttat cttaataaaa tgctaaatgt caatttatca ctgcgcatgt 3840
ttgactttag actgtaaata gagatcagtt tgtttctttc tgtgctggta acaatgagcg 3900
tcgcacagac atggtttcag gtaaataaat ctattctatg at 3942

<210> 32
<211> 367
<212> DNA
<213> Homo sapiens

<400> 32
ctccagtctc acctcggctt gcaatggacc ccaactgctc ctgcgaggct ggtggctcct 60

gcgcctgcgc cggctcctgc aagtgcaaaa agtgcaaatg cacctcctgc aagaagagct 120

gctgctcctg ttgccccctg ggctgtgcca agtgtgccca gggctgcatc tgcaaagggg 180

cgtcagagaa gtgcagctgc tgtgcctgat gtcgggacag ccctgctgtc agatgaaaac 240

agaatgacac gtaaaatccg aggttttttt tttctacaac tccgactcat ttgctacatt 300

cctttttttc tgtgaaatat gtgaataata attaaacact tagacttgaa aaaaaaaaaa 360

aaaaaaa 367

<210> 33
<211> 6903
<212> DNA
<213> Homo sapiens

<400> 33
gggagatttg gacgctccgg cctgggaggt gcgtcagatc cgagctcgcc atccagtttc 60

ctctccacta gtccccccag ttggagatct gggaccaaca aggcaccatg gcgcagaagg 120

gccaactcag tgacgatgag aagttcctct ttgtggacaa aaacttcatc aacagcccag 180

tggcccaggc tgactgggcc gccaagagac tcgtctgggt cccctcggag aagcagggct 240

tcgaggcagc cagcattaag gaggagaagg gggatgaggt ggttgtggag ctggtggaga 300

atggcaagaa ggtcacggtt gggaaagatg acatccagaa gatgaaccca cccaagttct 360

ccaaggtgga ggacatggcg gagctgacgt gcctcaacga agcctccgtg ctacacaacc 420

tgagggagcg gtacttctca gggctaatat atacgtactc tggcctcttc tgcgtggtgg 480

tcaaccccta taaacacctg cccatctact cggagaagat cgtcgacatg tacaagggca 540

agaagaggca cgagatgccg cctcacatct acgccatcgc agacacggcc taccggagca 600

tgcttcaaga tcgggaggac cagtccattc tatgcacagg cgagtctgga gccgggaaaa 660

ccgaaaacac caagaaggtc attcagtacc tggccgtggt ggcctcctcc cacaagggca 720

agaaagacac aagtatcacg caaggcccat cttttgccta cggagagctg gaaaagcagc 780

ttctacaagc aaacccgatt ctggaggctt tcggcaacgc caaaacagtg aagaacgaca 840

actcctcacg attcggcaaa ttcatccgca tcaacttcga cgtcacgggt tacatcgtgg 900

gagccaacat tgagacctat ctgctagaaa aatcacgggc aattcgccaa gccagagacg 960

agaggacatt ccacatcttt tactacatga ttgctggagc caaggagaag atgagaagtg 1020

acttgctttt ggagggcttc aacaactaca ccttcctctc caatggcttt gtgcccatcc 1080

cagcagccca ggatgatgag atgttccagg aaaccgtgga ggccatggca atcatgggtt 1140

tcagcgagga ggagcagcta tccatattga aggtggtatc atcggtcctg cagcttggaa 1200

atatcgtctt caagaaggaa agaaacacag accaggcgtc catgccagat aacacagctg 1260
ctcagaaagt ttgccacctc atgggaatta atgtgacaga tttcaccaga tccatcctca 1320
ctcctcgtat caaggttggg cgagatgtgg tacagaaagc tcagacaaaa gaacaggctg 1380
actttgctgt agaggctttg gccaaggcaa catatgagcg ccttttccgc tggatactca 1440
cccgcgtgaa caaagccctg gacaagaccc atcggcaagg ggcttccttc ctggggatcc 1500
tggatatagc tggatttgag atctttgagg tgaactcctt cgagcagctg tgcatcaact 1560
acaccaacga gaagctgcag cagctcttca accacaccat gttcatcctg gagcaggagg 1620
agtaccagcg cgagggcatc gagtggaact tcatcgactt tgggctggac ctacagccct 1680
gcatcgagct catcgagcga ccgaacaacc ctccaggtgt gctggccctg ctggacgagg 1740
aatgctggtt ccccaaagcc acggacaagt ctttcgtgga gaagctgtgc acggagcagg 1800
gcagccaccc caagttccag aagcccaagc agctcaagga caagactgag ttctccatca 1860
tccattatgc tgggaaggtg gactataatg cgagtgcctg gctgaccaag aatatggacc 1920
cgctgaatga caacgtgact tccctgctca atgcctcctc cgacaagttt gtggccgacc 1980
tgtggaagga cgtggaccgc atcgtgggcc tggaccagat ggccaagatg acggagagct 2040
cgctgcccag cgcctccaag accaagaagg gcatgttccg cacagtgggg cagctgtaca 2100
aggagcagct gggcaagctg atgaccacgc tacgcaacac cacgcccaac ttcgtgcgct 2160
gcatcatccc caaccacgag aagaggtccg gcaagctgga tgcgttcctg gtgctggagc 2220
agctgcggtg caatggggtg ctggaaggca ttcgcatctg ccggcagggc ttccccaacc 2280
ggatcgtctt ccaggagttc cgccaacgct acgagatcct ggcggcgaat gccatcccca 2340
aaggcttcat ggacgggaag caggcctgca ttctcatgat caaagccctg gaacttgacc 2400
ccaacttata caggataggg cagagcaaaa tcttcttccg aactggcgtc ctggcccacc 2460
tagaggagga gcgagatttg aagatcaccg atgtcatcat ggccttccag gcgatgtgtc 2520
gtggctactt ggccagaaag gcttttgcca agaggcagca gcagctgacc gccatgaagg 2580
tgattcagag gaactgcgcc gcctacctca agctgcggaa ctggcagtgg tggaggcttt 2640
tcaccaaagt gaagccactg ctgcaggtga cacggcagga ggaggagatg caggccaagg 2700
aggatgaact gcagaagacc aaggagcggc agcagaaggc agagaatgag cttaaggagc 2760
tggaacagaa gcactcgcag ctgaccgagg agaagaacct gctacaggaa cagctgcagg 2820
cagagacaga gctgtatgca gaggctgagg agatgcgggt gcggctggcg gccaagaagc 2880
aggagctgga ggagatactg catgagatgg aggcccgcct ggaggaggag gaagacaggg 2940
gccagcagct acaggctgaa aggaagaaga tggcccagca gatgctggac cttgaagaac 3000
agctggagga ggaggaagct gccaggcaga agctgcaact tgagaaggtc acggctgagg 3060
ccaagatcaa gaaactggag gatgagatcc tggtcatgga tgatcagaac aataaactat 3120
caaaagaacg aaaactcctt gaggagagga ttagtgactt aacgacaaat cttgcagaag 3180
aggaagaaaa ggccaagaat cttaccaagc tgaaaaacaa gcatgaatct atgatttcag 3240

aactggaagt gcggctaaag aaggaagaga agagccgaca ggagctggag aagctgaaac 3300
ggaagctgga gggtgatgcc agcgacttcc acgagcagat cgctgacctc caggcgcaga 3360
tcgcagagct caagatgcag ctggccaaga aggaggagga gctgcaggcg gccctggcca 3420
ggcttgacga tgaaatcgct cagaagaaca atgccctgaa gaagatccgg gagctggagg 3480
gccacatctc agacctccag gaggacctgg actcagagcg ggccgccagg aacaaggctg 3540
aaaagcagaa gcgagacctc ggcgaggagc tggaggccct aaagacagag ctggaagaca 3600
cactggacag cacagccact cagcaggagc tcagggccaa gagggagcag gaggtgacgg 3660
tgctgaagaa ggccctggat gaagagacgc ggtcccatga ggctcaggtc caggagatga 3720
ggcagaaaca cgcacaggcg gtggaggagc tcacagagca gcttgagcag ttcaagaggg 3780
ccaaggcgaa cctagacaag aataagcaga cgctggagaa agagaacgca gacctggccg 3840
gggagctgcg ggtcctgggc caggccaagc aggaggtgga acataagaag aagaagctgg 3900
aggcgcaggt gcaggagctg cagtccaagt gcagcgatgg ggagcgggcc cgggcggagc 3960
tcaatgacaa agtccacaag ctgcagaatg aagttgagag cgtcacaggg atgcttaacg 4020
aggccgaggg gaaggccatt aagctggcca aggacgtggc gtccctcagt tcccagctcc 4080
aggacaccca ggagctgctt caagaagaaa cccggcagaa gctcaacgtg tctacgaagc 4140
tgcgccagct ggaggaggag cggaacagcc tgcaagacca gctggacgag gagatggagg 4200
ccaagcagaa cctggagcgc cacatctcca ctctcaacat ccagctctcc gactcgaaga 4260
agaagctgca ggactttgcc agcaccgtgg aagctctgga agaggggaag aagaggttcc 4320
agaaggagat cgagaacctc acccagcagt acgaggagaa ggcggccgct tatgataaac 4380
tggaaaagac caagaacagg cttcagcagg agctggacga cctggttgtt gatttggaca 4440
accagcggca actcgtgtcc aacctggaaa agaagcagag gaaatttgat cagttgttag 4500
ccgaggagaa aaacatctct tccaaatacg cggatgagag ggacagagct gaggcagaag 4560
ccagggagaa ggaaaccaag gccctgtccc tggctcgggc ccttgaagag gccttggaag 4620
ccaaagagga actcgagcgg accaacaaaa tgctcaaagc cgaaatggaa gacctggtca 4680
gctccaagga tgacgtgggc aagaacgtcc atgagctgga gaagtccaag cgggccctgg 4740
agacccagat ggaggagatg aagacgcagc tggaagagct ggaggacgag ctgcaagcca 4800
cggaggacgc caaactgcgg ctggaagtca acatgcaggc gctcaagggc cagttcgaaa 4860
gggatctcca agcccgggac gagcagaatg aggagaagag gaggcaactg cagagacagc 4920
ttcacgagta tgagacggaa ctggaagacg agcgaaagca acgtgccctg gcagctgcag 4980
caaagaagaa gctggaaggg gacctgaaag acctggagct tcaggccgac tctgccatca 5040
aggggaggga ggaagccatc aagcagctac gcaaactgca ggctcagatg aaggactttc 5100
aaagagagct ggaagatgcc cgtgcctcca gagatgagat ctttgccaca gccaaagaga 5160
atgagaagaa agccaagagc ttggaagcag acctcatgca gctacaagag gacctcgccg 5220
ccgctgagag ggctcgcaaa caagcggacc tcgagaagga ggaactggca gaggagctgg 5280

ccagtagcct gtcgggaagg aacgcactcc aggacgagaa gcgccgcctg gaggcccgga 5340
tcgcccagct ggaggaggag ctggaggagg agcagggcaa catggaggcc atgagcgacc 5400
gggtccgcaa agccacacag caggccgagc agctcagcaa cgagctggcc acagagcgca 5460
gcacggccca gaagaatgag agtgcccggc agcagctcga gcggcagaac aaggagctcc 5520
ggagcaagct ccacgagatg gagggggccg tcaagtccaa gttcaagtcc accatcgcgg 5580
cgctggaggc caagattgca cagctggagg agcaggtcga gcaggaggcc agagagaaac 5640
aggcggccac caagtcgctg aagcagaaag acaagaagct gaaggaaatc ttgctgcagg 5700
tggaggacga gcgcaagatg gccgagcagt acaaggagca ggcagagaaa ggcaatgcca 5760
gggtcaagca gctcaagagg cagctggagg aggcagagga ggagtcccag cgcatcaacg 5820
ccaaccgcag gaagctgcag cgggagctgg atgaggccac ggagagcaac gaggccatgg 5880
gccgcgaggt gaacgcactc aagagcaagc tcaggcgagg aaacgagacc tctttcgttc 5940
cttctagaag gtctggagga cgtagagtta ttgaaaatgc agatggttct gaggaggaaa 6000
cggacactcg agacgcagac ttcaatggaa ccaaggccag tgaataagca actttctaca 6060
gttttgcacc acggcaagaa aaccaaaaac caaaacaaac aaacaaaaaa aacccaacaa 6120
caacccagaa caaagcaaaa cccagcagac tgtacttagc attgtctaaa tccattctca 6180
aattccaaat atcacagaca cccctcacac aaggaatata aaaaccacca ccctccagcc 6240
tgggcaacgt agtaaaacct catctataca agaatttaaa aataagctgg gcgtggtggt 6300
acacacctgt ggtcccagct actagggagg ctgagccagg aagaacgctc cagcccagga 6360
cttcgaggct gcaatgagct ataattgcat cattgcactc cagcctgggc aacagagacc 6420
ctgtctcaac caccaccacc accaccaccc ctactacccc tgtattcaag gtaaaaattg 6480
aagtttgtat gatgtaagag atgagaaaaa cccaacagga aacacagaca catcctccag 6540
ttctatcaat ggattgtgca gacactgagt ttttagaaaa acatatccac ggtaaccggt 6600
ccctggcaat tctgtttaca tgaaatgggg agaaagtcac cgaaatgggt gccgccggcc 6660
cccactccca attcattccc taacctgcaa acctttccaa cttctcacgt caggcctttg 6720
agaattcttt ccccctctcc tggtttccac acctcagaca cgcacagttc accaagtgcc 6780
ttctgtagtc acatgaattg aaaaggagac gctgctccca cggaggggag caggaatgct 6840
gcactgttta caccctgact gtgcttaaaa acactttcac taataaatgg ttataaatca 6900
caa 6903

<210> 34
<211> 11102
<212> DNA
<213> Homo sapiens

<400> 34
ggcgggagga gtaaagatgg cggcgcgagg gtctccgccc tctgctccgg gctgaagcgc 60
tctgagagag gcggcagcgg caactcgagc cccaacagta atttagtgtt ggtagttttg 120

gcagcagctg ccgaggccgg agcaatggcg gaactggagc acctaggagg gaagcgggca 180
gagtcggcgc gaatgcggcg ggcagagcag cttcggcgct ggcggggctc gctgacagag 240
caggagcctg cggagcgacg aggcgcgggg cggcagccgc tgaccaggcg cgggagcccc 300
agggtccgct tcgaggacgg tgctgtcttt ctggccgcct gctctagcgg ggacaccgac 360
gaggtgagaa agcttctggc aagaggtgct gatatcaaca cggtcaacgt ggacggcttg 420
acagccctgc accaggcatg tattgatgaa aatttggaca tggtgaagtt tctggtggag 480
aacagagcca atgtaaacca gcaagacaac gagggctgga caccccttca tgcagcagct 540
tcctgtggct atctcaacat agcagagtat ttcattaatc acggagccag tgtaggtatt 600
gtcaatagtg aaggtgaagt tccctctgac cttgcagaag agccagccat gaaggatctt 660
cttctggagc aagtaaagaa gcaaggaatt gatctagagc agtcaagaaa agaagaagag 720
cagcagatgt tgcaggatgc ccgccagtgg ctcaacagtg ggaaaataga ggatgtgagg 780
caggctcgct caggggctac agcccttcat gtggctgctg ccaagggcta ctctgaagtc 840
ctcagacttt taattcaggc tggctatgaa ctcaatgttc aggattatga tggctggact 900
cccctccatg ctgctgcaca ctggggagtg aaggaggctt gctccatcct ggcagaagca 960
ctttgtgaca tggatattcg aaataaactg ggccagacac catttgatgt ggctgatgag 1020
ggtctcgtgg agcatttgga gttgctccag aagaagcaga atgtgcttcg aagtgaaaag 1080
gagacacgga ataaactcat tgagtcagat ctgaacagca agattcagag tgggttcttt 1140
aagaacaaag agaagatgct ctatgaggag gagacaccta agtcccaaga aatggaggaa 1200
gaaaataaag aatctagtag ctccagctca gaggaggagg aaggtgaaga tgaagcttct 1260
gagtcagaaa ctgagaagga ggcagataaa aagccagaag cctttgtcaa tcattccaac 1320
tctgaaagca agagtagtat cacagagcag ataccagcac cagctcaaaa taccttctct 1380
gcctcttctg ctaggaggtt ctcttctggc ctttttaaca agccagaaga gcccaaagat 1440
gaatctcctt cttcatggag attgggactg agaaaaactg gcagccacaa catgctgagt 1500
gaggtggcca attccaggga acctataagg gaccgaggct cttccatcta tcgctcctct 1560
tcaagccctc gaatttctgc tctactggac aacaaagata aggagagaga aaacaaaagc 1620
tatattagtt cactagcacc ccggaagctc aacagcacaa gtgatattga agaaaaggag 1680
aacagagaat cagctgttaa tctagtgagg agtggctcct atacccggca gctatggagg 1740
gatgaagcaa aaggaaatga aatcccacag acaattgctc cctccaccta tgtatcaact 1800
tacttgaaaa ggactcctca caaatcccag gccgacacaa cagcagagaa aacagcagac 1860
aatgtctctt ctagcacccc gctctgtgtg atcaccaatc gccctcttcc tagcactgcc 1920
aatggggtta cagctactcc tgtgctctcc attactggaa cagattcctc tgtggaagcc 1980
agggagaaga ggaggtccta tctgactcct gtacgggatg aggaagcaga gtctttacgg 2040
aaagcacgct ccagacaagc tcggcagaca cgaaggtcta ctcaaggtgt caccctaaca 2100
gaccttcaag aagcagaaag gacattcagc cggtcgaggg cagagaggca agctcaggag 2160

cagcctcgtg agaagcccac agacactgaa gggcttgagg ggagccctga gaagcatgag 2220
ccctcagcag ttccagcaac agaagctggg gagggccagc agccctgggg caggagtctg 2280
gatgaagagc ctatctgtca tcgcctgagg tgcccagctc agccagacaa acccaccacg 2340
ccagcatctc cttctacgtc aagaccctca ctctacacca gttcccacct gctatggaca 2400
aatagatttt cagtccctga ttctgagagt tcagagacta ccacaaacac tacaactgca 2460
aaggaaatgg acaaaaatga gaatgaagaa gcagatttgg atgagcagtc ctctaagagg 2520
ctgtccatcc gagagaggag gcggcccaag gaacgacgaa gaggcacagg catcaatttc 2580
tggacaaagg atgaggatga aactgatggc tctgaagagg tcaaagaaac gtggcatgaa 2640
agactttcta ggttggaatc gggaggtagt aatcctacaa ccagtgattc ttacggtgac 2700
cgggcttcag caagagcccg tcgggaggcc cgggaggccc gcctagccac cctgaccagc 2760
cgtgtagaag aagacagcaa cagagattat aaaaaactct atgagagtgc tctgactgaa 2820
aaccaaaaac tgaaaacaaa acttcaggaa gcccagctag agctagcaga tataaagtcc 2880
aagcttgaga aggtggccca gcagaaacaa gaaaagacct ctgaccgatc atcagtgctg 2940
gagatggaga aacgggagag gcgagccttg gagcgcaaaa tgtcagaaat ggaggaagaa 3000
atgaaggtgt taacagaact gaaatccgac aaccagaggc tgaaagatga aaatggtgcc 3060
ctcatcagag tcatcagcaa actgtccaag taggctaggc tccagattta tgaggaaaga 3120
aagggacagc atttgctgcc cccacccctc ttttccagtc cttgccttcc aaccaaaaga 3180
aatggatgtt ttggtggaag gacacttctt tctatcaccc tcttcagtca cctctataca 3240
ctctacattt tctctgcact ttcaatgccc tgttcttcca aacccctatc ccaagtttta 3300
tgacagtttt aattgaagca tgattgtggt aattcgagcc atctggagaa tgctctgggg 3360
agtacaccag gctcagctgt ggacccctca acttcctgct gctcagctac tttgtccaca 3420
ttggatttgg tccaaacatg taagacttct accctaatca gtatccttca gctttttaca 3480
ttaacccagt gtcctctgat ataggtgagt cttgtggtag ccactccagg atcctgattg 3540
gggtgccaag agaaacagca ggatgttgaa ttgatcatca gatgccctct ggaatggtta 3600
gcatccaagg tgacagtgac tgcattgagg cgctctattc ttcttcacct ctcaggaact 3660
gacttttatt ttttctatca acacccagta atctccctaa ctagttttaa cccttattcc 3720
tccctcatac ctagccattt ctccaaggcg caaatggccc tggcttcatt tattcctttc 3780
ctttctatcc ttttatattt ttcccttccc caccccctca ctcaatggta taaaagctag 3840
gacagagcac ctgacctcag ttgtctttgg ccattgtggg aagtcattat tctggagaca 3900
agaaaatcat cactctggtg ccttggtggc agcaccactg cctgctccct ggaaggtaga 3960
ctatggcata agtgtcagct cattgttttc gtctcccact tatcttcccc cttcaaagtg 4020
attttcttta taagaaaatt tgggctctga atactccatt ctgcctcact tccttgacct 4080
tgttttcctc tcttcgccct gacacctgct tctaatgaga aatgaagtca gtgcaaggag 4140
gaatgagagg tggggagggg agattgttga gagatggact aggaagtgga agcaggctgg 4200

aaagacacat atggcgaaag tcaagtatct gaggccactt gcaacattgt gacaaagaag 4260
atggaagata gcagcctact gggctgtgag aatagtggtg aatttgatta tttccagtgg 4320
ttagatttag cagagagatt tactttctta gaaattaata ggggaaatgg cacttaatag 4380
aggtttaatg gaatgaagta ctttcaaccc attgatgaga gtcatggaag tttagagtgg 4440
caagacccag aggtcattag gtccagcttt ctattattgt gtcacaggga cccctgctac 4500
cacagctttc acccatgggc atggtcaggt agcctctggt ggctatgcat ttattactca 4560
aaagttgggc aactctaata acgagagagt ttttcctcat atcagctgaa gtctgcctgt 4620
ggattctcct tgtagtccag atctactatc tgcaactctt taacggcact tgagatatgt 4680
gcagtgggtg gtctccccct cagtcttact ttgagctgct gttactctag tcctttaagt 4740
cattcctcat atggcatgct tttcttacct gtcacatctt ggccatcctc ctctagacaa 4800
aaccagcctt taaaatatca tgtccaggcc tggaaagaat acaaatccag tgcaaaatta 4860
aaccattagt tcttgatgat aacctagcat taatacttac agtattttct ttttgtagga 4920
gttttatccc ttggttcact tgagtgtgca agtcattaaa tcctcatcat tttagggttg 4980
ccagtgatat tcagtgttta gagtggttca ttcccttaat atatactacc tctgtctgtt 5040
aattctttcc taacatgaca tgtagctctc catggtcctc cctctgaaac accacatgct 5100
gtttaaacta gaagctgtaa ttcaagatcc caattccacc aggtgcagtg gctcatgcct 5160
gtaatcccag cacttcagga ggccgaggtg gacagatcac gaggtcagga gttcgagacc 5220
atcctggcta acacggtgaa acccaatctc tactaaaaat acaaaaaatt agccaggcgt 5280
ggtggcaggc gcctgtagtc ccagctactc aggaggctga ggcaggagaa tggcgtgaac 5340
ccgggaggcg gagcttgcag tgagccttga tcacgccact gcactccagc ctgggtgaca 5400
gagcaagact ccatctcaaa aaaaataata ataataaata aataaaaaat cccaattaca 5460
gaacaggaga tatgtttcct ctgacaaaac cccattttta gagtgtataa aattctgtct 5520
ttcatttcca accatttttc aagagaagct gctactatct tgagacttcg tattatcatg 5580
ggagtactgt aagttcagtt ctcagggaat gctaaattag agtaataagt tttatatcct 5640
tcattctggg agaggaaatc aacataaggt gttgactaat tgattatcct ggaacatttt 5700
gaatattggg gaaggaagat gatacttctt tctcaagaaa agaaaattgg attattagga 5760
atacatcttg acccttttcc actcaataga ccagggaaag gggttacaga gagcctcctc 5820
agatctgttt gtgcctttca aaagtactgg gggcagattc tcaaaactct tagattggtg 5880
ttgcatccct gccacttcac atgacccttc catgttaaag ctgacttttc ttgccagaaa 5940
gttttccttg tgacaagaaa tttgcaagct tttcaatctc aggaatactg gattctatag 6000
ccaagaactc atgaaagtaa gtgtaaacta tcctaggtaa tttcaggtac tttctcttat 6060
gcaattaatt ttattaactg atttctggtc tatctccaga gataaaatac cattccttca 6120
caactgtatt gtctggaagc cccatgagat ggtaattata ggccttctac tagccagcag 6180
ttctctctgg atactggcat cagcaggtct cctttttctt gtgctgcttg gacaggtacc 6240

tactgctggc ctcttcagtg gccacaatcc tcagtcctca cctgctggtt tggagactca 6300
tgagcacccc aggcagctca agctgaataa atagctgcag acatggccaa caacatgggg 6360
ctcaggtctg tcacttaact tagagtcttt acatgaaata tcagaatggg aaagagaatg 6420
ttcctggtaa aactctgttt tcattaattt gttatttttc aaaaaccgag ccacaccttt 6480
ggaagtaatg atttggccat tccagagatt ctggggctaa agtttgtttt tccagggctg 6540
ctgatccttt tccagatagc tccagagtat gatgacccca gccccatccc aatctctagc 6600
gaaagggtta tgcaaatctc agggtgttgc ctctaaagaa tctaaagaat agacatgctt 6660
tgggcttact cagtgtacca gattctctag tgggccattc tcatttgatg ttccctagaa 6720
cctccctgtc ttccaactgc aggatgtgtt tacttctctt ctgggcttcc cctggtttct 6780
gtcctagttc ccaccccaag tttaagaaga cttagattca tttattgcta tctgaggcca 6840
ctagtgagat ttcagatctc tcttttgcaa cattttaccc atttcccttc cccaaaccat 6900
gtgcttatta gatggttcca ctcaggaagc aaggaagcta aaactcaagt taatggtctg 6960
tggatcccac gaaagtgtgt gtaaactact gactgtttct ccactagagg catttaattg 7020
agccaaagat gatggaagaa gaaactctca aagccatgta ctttgttagg tgtctggatt 7080
tttggcctgt ttgcagcctc tcctagccac agacagacac tgtctcctct cagagaaagg 7140
ccagtgtttc ggtccaggcc actggtgagt gtcggcaccc ttgaggtggg catggagtga 7200
gcagaagaag cctggagaag agagaactat ttaaaagtgt tgccagttat tcagcttcca 7260
ggcggctttg cacctccaaa ggtgcaaaaa ggaaaaagaa agcagaaagg attcagcatt 7320
taagcagaaa gaaaagggaa attctactct accagatgag ccactggttc cactggagct 7380
tatcagtgaa gtaacactgc aggagccctg gtatccttcc ctcagagata tcatcagggg 7440
ccctcaaacc ccattgttta tgccagcact gaccgcactt taagcacaca cttaccaatc 7500
agaggtcgtg taattggcct aaatctaggc ccagttatta ctacaaatct tagtcaccat 7560
actctctcgc ccaggctgga gtgcagtggt gtgatctcag ctcactgcaa cctccacctc 7620
ccaggctcaa gggatcctct cacctcagct cccgagcagc taggactgca ggtgtgcacc 7680
accatgccca gctaattttt gaattttttt gtagagatgg ggttttgcca tgttgcccag 7740
gctggtctga agctgctggg ctcaagcaat ccacctggct gggcctccca aagcgatagg 7800
attacaggcg taagggccca cgcccagcct accatactga ttttcaaggc agggtctaac 7860
ccttcataag atgtgagttc ttccaaccca ataagcaggc agctttgagg acaaaaaggc 7920
aacattaaaa gataagaccc tagaagtcag aacatctgta ttccaaggcc accccttctc 7980
cttttagtca tgtgactttg ggcaggtcac cctccctgtc tgtaaaatga ggttggactg 8040
gataatcttt atgtgccctt tccttttggc ttaaaattct gtgatttatt cattcttagg 8100
aagatcagca tatttattgg gatttctgct tcaaaaattc tttctaacct caaaaatcca 8160
agtccagaat tttttgtttt cttctggatt taaactgttt aatatgagca catcaaggtt 8220
gacttctcat cctttttggg aagtgaattt aatattgagg gtaattgaat gatgttgcaa 8280

tggctttccc tcctgctggc ttggtaagaa tattaaagcc tgcagaggtg gaaattggag 8340
ctcatggaga aatggataga aagcaatttg ttgcaggcag cctttggaca agttgtttta 8400
ataggaaata gacctgctgc ttcataggtt tcctcaacca cctttcctca gctttcttaa 8460
aatgggatct acattggctc ttcacaccca aatagcagac taatcgtttt tctgcttagc 8520
accgtctggt tcattgtctt gaactctgcc ttacagcagc aagaaaattt tcctcgacaa 8580
gaacctcaat ctttagttcc attgagctcc ccctctggat tttggactta ccagaagtag 8640
gaggttctga taccattcaa gatggtcttt ccttcaaagc aggtctgaag aggagactac 8700
caaagcagtg tttacaaacc cagagtccac acaaccatat tgcatagaac agcacttggc 8760
tttcacaagc ctcctacagg acctggtgta attggagtga aagggcagag accctggaag 8820
tggaggtggc tgtgtgctgc gatgggaaga aggcagaagg cccaggggct ttggacatag 8880
agcagggtgg aagctgcaag tactgggaag gaagagagtt tcacagaaac aaagctttgt 8940
cacacagaaa tgagttctgt ctcactggtg acttcatccc tcaggctcca gctgagcaga 9000
gattttaatc agcttcctta atgggtattg acactgctca ggaagcagta gaccctgtca 9060
gggacagcta ttgatctttt gtgttctgat tagattggaa aatagatcaa cttcattgta 9120
gtccaggaac tgttggtcac agctactagg aatgaggtga tttctgaggg ctgagaaaaa 9180
acacagaatc ttggccagca gccagcagct gcatggtgaa agatgcattc acttctcctt 9240
tgagagttgg ggttgagggc aaacatagaa cccaggtttg gcttacaacc cagtgtcccg 9300
gaagccctcc ttcgggagaa ctgtaagtaa gaggtgggtg tgtctaaaga caataccatt 9360
aatgaatgtt ctggccttac ctaaaaaggt ttagcaattt ggggataact cttggatcta 9420
gcttatgtgc gttcacatgc acatttgcta gcccagagct tttaaaatga ggtctggcat 9480
atacttgatt acaaatgaaa actcagaaac caattttatt tattaaatca tatcttttgt 9540
ttttccccct cccttctaat cccccaaagg acctatttga gctgttcccc aattcatctg 9600
cttattttgg accatgaatc tgccagagtg atattttctg ttatttctcc tccaaatttt 9660
tccctgatgt ttccaataaa gatttacttg ggtggcccct taaggtgaca tcaggatgct 9720
cttatgtcct tccagaataa gcatacactt cactcctctc cctttcatct ccctctgcat 9780
tcttaattcc ttgcttttct cacttggagc cgagggtgct ttagagaggt ggttttccat 9840
gaatcagcca agattcctgt agaagttggg tataccaacc accaccacca ccgcccccta 9900
ttccagtttc aaagctcctc ggctatgcta atgtcccctc agagatgagg tttgactttt 9960
aggcccgtat gactcctcca tagcctggcc aaggagacca tgagtagcca tgtctggttt 10020
actctttatc ctgagactgt ttatagctta aaacagaagt gtgtcttccc agcacaaacc 10080
taatcaatca gtgtatcagt gcatctggtg gcaacagctc agcccattca aagagcaagg 10140
attcaggaaa ggcacactga tggtggggag cctcttaaga gcctctaatg ttctcccaaa 10200
accagagttg agagtcggag tgccagtcgt cggggcccac tattcctgaa taagggacat 10260
gcaagggcca gaagtagctt gactctcgcc taaatatctg tgcctttgcc tgtcctttct 10320

cccactctac tgaaacccgg aacagattcc cgcttgcctt ctgatgaaga gaggttaggt 10380
aaagagagtt tggaggaaaa aagacaccag gaggcaggct gcggggtagg agagggttct 10440
gagaggaggc agcaatccag aatacctcct tttctagcca gcatcccttg aacttttgaa 10500
aggttgtgcc taccactggc tggcacacca gggcaatgat ttccctgcag aaggaaggaa 10560
agaatgtttc acccttgcat ccttcttgga gaagctacag cctgtgctca gttgagtggt 10620
tcacactcag actttggctt tatggttttc cttcctcctt gtctttgccc tgaccttgat 10680
caacaggggt gaaaagaacc accctgaggt ttccatgcct ctcccatttt agtggtagca 10740
ttttgtgtct ttactccacc cttcacccta gttccaccaa ggttcacaca ccagatgtaa 10800
ctgtttttca gctgagttgt atggattaac ttcagtccac tgtaaataca cctgggatgg 10860
ggtggggttg gggttgttta gggagaagca gccagacttg ctttgtgaac tgaatgtatt 10920
tttatgcaat tttgagtggc ctttcaaccc taagatgaat ggttttgttt tactggttgt 10980
tgttatatag ttttgagtat tctgtgtttg aaagtttggg aataataata ttcacttcta 11040
tatgatgcct gtaaacacaa cagtatttat aaatgagtaa ataaaactgt gttttaactt 11100
tg 11102

<210> 35
<211> 2616
<212> DNA
<213> Homo sapiens

<400> 35
agaggagcct gagaagaggc agaggaaggc gaaacatggc tgctctagga gtccagagta 60
tcaactggca gacggccttc aaccgacaag cgcatcacac agacaagttc tccagccagg 120
agctcatctt gcggagaggc caaaacttcc aggtcttaat gatcatgaac aaaggccttg 180
gctctaacga aagactggag ttcattgtct ccacagggcc ttacccctca gagtcggcca 240
tgacgaaggc tgtgtttcca ctctccaatg gcagtagtgg tggctggagt gcggtgcttc 300
aggccagcaa tggcaatact ctgactatca gcatctccag tcctgccagc gcacccatag 360
gacggtacac aatggccctc cagatcttct cccagggcgg catctcctct gtgaaacttg 420
ggacgttcat actgcttttt aacccctggc tgaatgtgga tagcgtcttt atgggtaacc 480
acgctgagag agaagagtat gttcaggaag atgccggcat catctttgtg ggaagcacaa 540
accgaattgg catgattggc tggaactttg gacagtttga agaagacatt ctcagcatct 600
gcctctcaat cttggatagg agtctgaatt tccgccgtga cgctgctact gatgtggcca 660
gcagaaatga ccccaaatac gttggccggg tgctgagtgc catgatcaat agcaatgatg 720
acaatggtgt gcttgctggg aattggagcg gcacttacac cggtggccgg gacccaagga 780
gctggaacgg cagcgtggag atcctcaaaa attggaaaaa atctggcttc agcccagtcc 840
gatatggcca gtgctgggtc tttgctggga ccctcaacac agcgctgcgg tctttgggga 900
ttccttcccg ggtgatcacc aacttcaact cagctcatga cacagaccga aatctcagtg 960
tggatgtgta ctacgacccc atgggaaacc ccctggacaa gggtagtgat agcgtatgga 1020

atttccatgt ctggaatgaa ggctggtttg tgaggtctga cctgggcccc tcgtacggtg 1080

gatggcaggt gttggatgct accccgcagg aaagaagcca aggggtgttc cagtgcggcc 1140

ccgcttcggt cattggtgtt cgagagggtg atgtgcagct gaacttcgac atgcccttta 1200

tcttcgcgga ggttaatgcc gaccgcatca cctggctgta cgacaacacc actggcaaac 1260

agtggaagaa ttccgtgaac agtcacacca ttggcaggta catcagcacc aaggcggtgg 1320

gcagcaatgc tcgcatggac gtcacggaca agtacaagta cccagaaggc tctgaccagg 1380

aaagacaagt gttccaaaag gctttgggga aacttaaacc caacacgcca tttgccgcga 1440

cgtcttcaat gggtttggaa acagaggaac aggagcccag catcatcggg aagctgaagg 1500

tcgctggcat gctggcagta ggcaaagaag tcaacctggt cctactgctc aaaaacctga 1560

gcagggatac gaagacagtg acagtgaaca tgacagcctg gaccatcatc tacaacggca 1620

cgcttgtaca tgaagtgtgg aaggactctg ccacaatgtc cctggaccct gaggaagagg 1680

cagaacatcc cataaagatc tcgtacgctc agtatgagaa gtacctgaag tcagacaaca 1740

tgatccggat cacagcggtg tgcaaggtcc cagatgagtc tgaggtggtg gtggagcggg 1800

acatcatcct ggacaacccc accttgaccc tggaggtgct gaacgaggct cgtgtgcgga 1860

agcctgtgaa cgtgcagatg ctcttctcca atccactgga tgagccggtg agggactgcg 1920

tgctgatggt ggagggaagc ggcctgctgt tgggtaacct gaagatcgac gtgccgaccc 1980

tagggcccaa ggagcggtcc cgggtccgtt ttgatatcct gccctcccgg agtggcacca 2040

agcaactgct cgccgacttc tcctgcaaca agttccctgc aatcaaggcc atgttgtcca 2100

tcgatgtagc cgaatgaagg gcgctggtgg cctcccgtac aaacttggac aacacggagc 2160

agggagagct caccatggaa tgaacccccc gcccatgctg tccggcctgg gaaaccctct 2220

ccatctccca aggctgccag acatggactc cgggctccag cacatccccc tctcctctcc 2280

cccaggttgg ggctgggtcc accctgtcct atgacttgat cacttttgca cattccctgg 2340

ccgcttctcc ccagagctgc ctgctctgtg agccccacag ccctgctcat tcctcacgcc 2400

cttcaatgct gcaggatgga ctggcccctg acccagggac tctccaaacg ggatacagga 2460

gagaagctgg tctagactgt ttgctgatcc ccaacctgca cggggcattc ctgcttctct 2520

ctcaggccac cacagagggc aggggatggt tagtcacctg ccccagcact cacaccctaa 2580

ctcaaaataa atgttaaata agtgcgatca cacaaa 2616

<210> 36
<211> 2831
<212> DNA
<213> Homo sapiens

<400> 36
   agcccgcgga gctgagcggc ggcggcggcg gcggcaggag cccgggaggc ggaggcggga 60
   ggcggcggcg gcgcgcggag acgcagcagc ggcagcggca gcatgtcggc cggcggagcg 120
   tcagtcccgc cgcccccgaa ccccgccgtg tccttcccgc cgccccgggt caccctgccc 180
   gccggccccg acatcctgcg gacctactcg ggcgccttcg tctgcctgga gattctgttc 240
   gggggtcttg tctggatttt ggttgcctcc tccaatgttc ctctacctct actacaagga 300
   tgggtcatgt ttgtgtccgt gacagcgttt ttcttttcgc tcctctttct gggcatgttc 360
   ctctctggca tggtggctca aattgatgct aactggaact tcctggattt tgcctaccat 420
   tttacagtat ttgtcttcta ttttggagcc tttttattgg aagcagcagc cacatccctg 480
   catgatttgc attgcaatac aaccataacc gggcagccac tcctgagtga taaccagtat 540
   aacataaacg tagcagcctc aatttttgcc tttatgacga cagcttgtta tggttgcagt 600
   ttgggtctgg ctttacgaag atggcgaccg taacactcct tagaaactgg cagtcgtatg 660
   ttagtttcac ttgtctactt tatatgtctg atcaatttgg ataccatttt gtccagatgc 720
   aaaaacattc caaaagtaat gtgtttagta gagagagact ctaagctcaa gttctggttt 780
   atttcatgga tggaatgtta attttattat gatattaaag aaatggcctt ttattttaca 840
   tctctcccct tttccctttt ccccctttat tttcctcctt ttctttctga aagtttcctt 900
   ttatgtccat aaaatacaaa tatattgttc ataaaaaatt agtatccctt ttgtttggtt 960
   gctgagtcac ctgaacctta attttaattg gtaattacag cccctaaaaa aaacacattt 1020
   caaataggct tcccactaaa ctctatattt tagtgtaaac caggaattgg cacacttttt 1080
   ttagaatggg ccagatggta aatatttatg cttcacggtc catacagtct ctgtcacaac 1140
   tattcagttc tgctagtata gcgtgaaagc agctatacac aatacagaaa tgaatgagtg 1200
   tggttatgtt ctaataaaac ttatttataa aaacaagggg aggctgggtt tagcctgtgg 1260
   gccatagttt gtcaaccact ggtgtaaaac cttagttata tatgatctgc attttcttga 1320
   actgatcatt gaaaacttat aaacctaaca gaaaagccac ataatattta gtgtcattat 1380
   gcaataatca cattgccttt gtgttaatag tcaaatactt acctttggag aatacttacc 1440
   tttggaggaa tgtataaaat ttctcaggca gagtcctgga tataggaaaa agtaatttat 1500
   gaagtaaact tcagttgctt aatcaaacta atgatagtct aacaactgag caagatcctc 1560
   atctgagagt gcttaaaatg ggatccccag agaccattaa ccaatactgg aactggtatc 1620
   tagctactga tgtcttactt tgagtttatt tatgcttcag aatacagttg tttgccctgt 1680
   gcatgaatat acccatattt gtgtgtggat atgtgaagct tttccaaata gagctctcag 1740
   aagaattaag tttttacttc taattatttt gcattacttt gagttaaatt tgaatagagt 1800
   attaaatata aagttgtaga ttcttatgtg tttttgtatt agcccagaca tctgtaatgt 1860
   ttttgcactg gtgacagaca aaatctgttt taaaatcata tccagcacaa aaactatttc 1920
   tggctgaata gcacagaaaa gtattttaac ctacctgtag agatcctcgt catggaaagg 1980
   tgccaaactg ttttgaatgg aaggacaagt aagagtgagg ccacagttcc caccacacga 2040
   gggcttttgt attgttctac tttttcagcc ctttactttc tggctgaagc atccccttgg 2100
   agtgccatgt ataagttggg ctattagagt tcatggaaca tagaacaacc atgaatgagt 2160
   ggcatgatcc gtgcttaatg atcaagtgtt acttatctaa taatcctcta gaaagaaccc 2220
   tgttagatct tggtttgtga taaaaatata aagacagaag acatgaggaa aaacaaaagg 2280
   tttgaggaaa tcaggcatat gactttatac ttaacatcag atcttttcta taatatccta 2340
   ctactttggt tttcctagct ccataccaca cacctaaacc tgtattatga attacatatt 2400
   acaaagtcat aaatgtgcca tatggatata cagtacattc tagttggaat cgtttactct 2460
   gctagaattt aggtgtgaga ttttttgttt cccaggtata gcaggcttat gtttggtggc 2520
   attaaattgg tttctttaaa atgctttggt ggcacttttg taaacagatt gcttctagat 2580
   tgttacaaac caagcctaag acacatctgt gaatacttag atttgtagct taatcacatt 2640
   ctagacttgt gagttgaatg acaaagcagt tgaacaaaaa ttatggcatt taagaattta 2700
   acatgtctta gctgtaaaaa tgagaaagtg ttggttggtt ttaaaatctg gtaactccat 2760
   gatgaaaaga aatttatttt atacgtgtta tgtctctaat aaagtattca tttgataaaa 2820
   aaaaaaaaaa a 2831

<210> 37
<211> 1749
<212> DNA
<213> Homo sapiens

<400> 37
gtggcctcga ggtggtggca gggccgcccc ctgcagtccg gagacgaacg cacggaccgg 60

gcctccggag gcaggttcgg ctggaaggaa ccgctctcgc ttcgtcctac acttgcgcaa 120

atgtctccga gcttactcac atagcatatt ggtatatcaa aatgaaatgc aaggaaccaa 180

aaataacata attgaaggca gtaaaagtga aattaaatag gaagatcatc agtcaaggaa 240

gacccactgg agaggacaga aaatgaagca gtgttttatc atgtgtattt cagcaggtct 300

tcttgaaatt taactaaaaa tatgactgct ctctcttcag agaactgctc ttttcagtac 360

cagttacgtc aaacaaacca gcccctagac gttaactatc tgctattctt gatcatactt 420

gggaaaatat tattaaatat ccttacacta ggaatgagaa gaaaaaacac ctgtcaaaat 480

tttatggaat atttttgcat ttcactagca ttcgttgatc ttttactttt ggtaaacatt 540

tccattatat tgtatttcag ggattttgta cttttaagca ttaggttcac taaataccac 600

atctgcctat ttactcaaat tatttccttt acttatggct ttttgcatta tccagttttc 660

ctgacagctt gtatagatta ttgcctgaat ttctctaaaa caaccaagct ttcatttaag 720

tgtcaaaaat tattttattt ctttacagta attttaattt ggatttcagt ccttgcttat 780

gttttgggag acccagccat ctaccaaagc ctgaaggcac agaatgctta ttctcgtcac 840

tgtcctttct atgtcagcat tcagagttac tggctgtcat ttttcatggt gatgatttta 900

tttgtagctt tcataacctg ttgggaagaa gttactactt tggtacaggc tatcaggata 960

acttcctata tgaatgaaac tatcttatat tttccttttt catcccactc cagttatact 1020

gtgagatcta aaaaaatatt cttatccaag ctcattgtct gttttctcag tacctggtta 1080

ccatttgtac tacttcaggt aatcattgtt ttacttaaag ttcagattcc agcatatatt 1140

gagatgaata ttccctggtt atactttgtc aatagttttc tcattgctac agtgtattgg 1200

tttaattgtc acaagcttaa tttaaaagac attggattac ctttggatcc atttgtcaac 1260

tggaagtgct gcttcattcc acttacaatt cctaatcttg agcaaattga aaagcctata 1320

tcaataatga tttgttaata ttattaatta aaagttacag ctgtcataag atcataattt 1380

tatgaacaga aagaactcag gacatattaa aaaataaact gaactaaaac aacttttgcc 1440

ccctgactga tagcatttca gaatgtgtct tttgaagggc tataccagtt attaaatagt 1500

gttttatttt aaaaacaaaa taattccaag aagtttttat agttattcag ggacactata 1560

ttacaaatat tactttgtta ttaacacaaa aagtgataag agttaacatt tggctatact 1620

gatgtttgtg ttactcaaaa aaactactgg atgcaaactg ttatgtaaat ctgagatttc 1680

actgacaact ttaagatatc aacctaaaca tttttattaa atgttcaaat gtaagcaaga 1740

aaaaaaaaa 1749

<210> 38
<211> 1638
<212> DNA
<213> Homo sapiens

<400> 38
gcggtgatga attgggacgc aggcgcggag cccagggacc actccccctg cacagacatg 60

agaccatagg ggacctgtct gggtggcctc agggataggc gctccccaag gtgtgaatga 120

ggcaggatga actggacagg tttgtacacc ttgctcagtg gcgtgaaccg gcattctact 180

gccattggcc gagtatggct ctcggtcatc ttcatcttca gaatcatggt gctggtggtg 240

gctgcagaga gtgtgtgggg tgatgagaaa tcttccttca tctgcaacac actccagcct 300

ggctgcaaca gcgtttgcta tgaccaattc ttccccatct cccatgtgcg gctgtggtcc 360

ctgcagctca tcctagtttc caccccagct ctcctcgtgg ccatgcacgt ggctcaccag 420

caacacatag agaagaaaat gctacggctt gagggccatg gggaccccct acacctggag 480

gaggtgaaga ggcacaaggt ccacatctca gggacactgt ggtggaccta tgtcatcagc 540

gtggtgttcc ggctgttgtt tgaggccgtc ttcatgtatg tcttttatct gctctaccct 600

ggctatgcca tggtgcggct ggtcaagtgc gacgtctacc cctgccccaa cacagtggac 660

tgcttcgtgt cccgccccac cgagaaaacc gtcttcaccg tcttcatgct agctgcctct 720

ggcatctgca tcatcctcaa tgtggccgag gtggtgtacc tcatcatccg ggcctgtgcc 780

cgccgagccc agcgccgctc caatccacct tcccgcaagg gctcgggctt cggccaccgc 840

ctctcacctg aatacaagca gaatgagatc aacaagctgc tgagtgagca ggatggctcc 900

ctgaaagaca tactgcgccg cagccctggc accggggctg ggctggctga aaagagcgac 960

cgctgctcgg cctgctgatg ccacatacca ggcaacctcc catcccaccc ccgaccctgc 1020

cctgggcgag cccctccttc tcccctgccg gtgcacaggc ctctgcctgc tggggattac 1080

tcgatcaaaa ccttccttcc ctggctactt cccttcctcc cggggccttc cttttgagga 1140

gctggagggg tggggagcta gaggccacct atgccagtgc tcaaggttac tgggagtgtg 1200

ggctgccctt gttgcctgca cccttccctc ttccctctcc ctctctctgg gaccactggg 1260

tacaagagat gggatgctcc gacagcgtct ccaattatga aactaatctt aaccctgtgc 1320

tgtcagatac cctgtttctg gagtcacatc agtgaggagg gatgtgggta agaggagcag 1380

agggcagggg tgctgtggac atgtgggtgg agaagggagg gtggccagca ctagtaaagg 1440

aggaatagtg cttgctggcc acaaggaaaa ggaggaggtg tctggggtga gggagttagg 1500

gagagagaag caggcagata agttggagca ggggttggtc aaggccacct ctgcctctag 1560

tccccaaggc ctctctctgc ctgaaatgtt acacattaaa caggatttta cagccaaaaa 1620

aaaaaaaaaa aaaaaaaa 1638

<210> 39
<211> 2534
<212> DNA
<213> Homo sapiens

<400> 39
gggattggga gggcttcttg caggctgctg ggctggggct aagggctgct cagtttcctt 60

cagcggggca ctgggaagcg ccatggcact gcagggcatc tcggtcgtgg agctgtccgg 120

cctggccccg ggcccgttct gtgctatggt cctggctgac ttcggggcgc gtgtggtacg 180

cgtggaccgg cccggctccc gctacgacgt gagccgcttg ggccggggca agcgctcgct 240

agtgctggac ctgaagcagc cgcggggagc cgccgtgctg cggcgtctgt gcaagcggtc 300

ggatgtgctg ctggagccct tccgccgcgg tgtcatggag aaactccagc tgggcccaga 360

gattctgcag cgggaaaatc caaggcttat ttatgccagg ctgagtggat ttggccagtc 420

aggaagcttc tgccggttag ctggccacga tatcaactat ttggctttgt caggtgttct 480

ctcaaaaatt ggcagaagtg gtgagaatcc gtatgccccg ctgaatctcc tggctgactt 540

tgctggtggt ggccttatgt gtgcactggg cattataatg gctctttttg accgcacacg 600

cactggcaag ggtcaggtca ttgatgcaaa tatggtggaa ggaacagcat atttaagttc 660

ttttctgtgg aaaactcaga aattgagtct gtgggaagca cctcgaggac agaacatgtt 720

ggatggtgga gcacctttct atacgactta caggacagca gatggggaat tcatggctgt 780

tggagcaata gaaccccagt tctacgagct gctgatcaaa ggacttggac taaagtctga 840

tgaacttccc aatcagatga gcatggatga ttggccagaa atgaagaaga agtttgcaga 900

tgtatttgca gagaagacga aggcagagtg gtgtcaaatc tttgacggca cagatgcctg 960

tgtgactccg gttctgactt ttgaggaggt tgttcatcat gatcacaaca aggaacgggg 1020

ctcgtttatc accagtgagg agcaggacgt gagcccccgc cctgcacctc tgctgttaaa 1080

caccccagcc atcccttctt tcaaaaggga tcctttcata ggagaacaca ctgaggagat 1140

acttgaagaa tttggattca gccgcgaaga gatttatcag cttaactcag ataaaatcat 1200

tgaaagtaat aaggtaaaag ctagtctcta acttccaggc ccacggctca agtgaatttg 1260

aatactgcat ttacagtgta gagtaacaca taacattgta tgcatggaaa catggaggaa 1320

cagtattaca gtgtcctacc actctaatca agaaaagaat tacagactct gattctacag 1380

tgatgattga attctaaaaa tggttatcat tagggctttt gatttataaa actttgggta 1440
cttatactaa attatggtag ttattctgcc ttccagtttg cttgatatat ttgttgatat 1500
taagattctt gacttatatt ttgaatgggt tctagtgaaa aaggaatgat atattcttga 1560
agacatcgat atacatttat ttacactctt gattctacaa tgtagaaaat gaggaaatgc 1620
cacaaattgt atggtgataa aagtcacgtg aaacagagtg attggttgca tccaggcctt 1680
ttgtcttggt gttcatgatc tccctctaag cacattccaa actttagcaa cagttatcac 1740
actttgtaat ttgcaaagaa aagtttcacc tgtattgaat cagaatgcct tcaactgaaa 1800
aaaacatatc caaaataatg aggaaatgtg ttggctcact acgtagagtc cagagggaca 1860
gtcagtttta gggttgcctg tatccagtaa ctcggggcct gtttccccgt gggtctctgg 1920
gctgtcagct ttcctttctc catgtgtttg atttctcctc aggctggtag caagttctgg 1980
atcttatacc caacacacag caacatccag aaataaagat ctcaggaccc cccagcaagt 2040
cgttttgtgt ctccttggac tgagttaagt tacaagcctt tcttatacct gtctttgaca 2100
aagaagacgg gattgtcttt acataaaacc agcctgctcc tggagcttcc ctggactcaa 2160
cttcctaaag gcatgtgagg aaggggtaga ttccacaatc taatccgggt gccatcagag 2220
tagagggagt agagaatgga tgttgggtag gccatcaata aggtccattc tgcgcagtat 2280
ctcaactgcc gttcaacaat cgcaagagga aggtggagca ggtttcttca tcttacagtt 2340
gagaaaacag agactcagaa gggcttctta gttcatgttt cccttagcgc ctcagtgatt 2400
ttttcatggt ggcttaggcc aaaagaaata tctaaccatt caatttataa ataattaggt 2460
ccccaacgaa ttaaatatta tgtcctacca acttattagc tgcttgaaaa atataataca 2520
cataaataaa aaaa 2534

## Description

The present invention refers to a method of monitoring, in particular prognosing and risk stratification of prostate cancer by the means of expression profiling.

Prostate cancer (PCA) is the most frequent tumor type in males and a major cause of death due to malignancy. It represents the cause of 6% of cancer deaths in men. Based on some estimates, 80% of men over the age of 80 suffer of some form of prostate disease (e.g. cancer, Benign Prostatic Hypertrophy, prostatitis, etc.).

Current methods for prostate cancer screening include a blood test for prostate specific antigen (PSA), digital rectal examination (DRE), and transrectal ultrasound (TRUS), whereas the latter is mainly used to image the prostate and to aid in guided needle biopsy. The widespread use of the prostate specific antigen (PSA) for the detection of PCA has resulted in an increasing number of men diagnosed with organ-confined, low Gleason-score PCA, which are potentially curable. However, the high sensitivity of the PSA test is accompanied by a low specificity, causing many patients suffering from unnecessary biopsy taking. Men with normal prostate pathology generally have a PSA level in blood below 4ng/ml. PSA levels between 4ng/ml and 10ng/ml ('grey zone') have a 25% chance of having prostate cancer. As a consequence, in 75% of the cases, men with an abnormal DRE and a PSA in this grey zone have a negative, or a seemingly unnecessary biopsy.

Thus, the object of the present invention is to find new molecular markers to improve early diagnosis, prediction of progression, and therapy of PCA. Because of their clinical importance, a better understanding of the molecular mechanisms of these mostly small tumors is essential in order to identify novel diagnostic and prognostic biomarkers for a tailored clinical management in the individual patient. Furthermore, in prostate cancer, heterogeneity is a common phenomenon which includes different histological grades within the same tumor focus and different genotypes among phenotypically similar foci in a single tumor. To better understand the molecular mechanisms of prostate cancer, it is essential to correlate gene expression with a specific cell type. Therefore, sub-populations of cells have to be selected to perform comparable molecular profiling experiments with the highest grade of information and comparability.

The WO2006/091776 discloses the identity of 41 genes the expression at the transcriptional and translational levels of which has been found to correlate with cancer progression. Furthermore, Schlomm et al. (International Journal of Oncology, 2005, Vol. 27, pages 713-720) have identified a set of genes which are shown to be either up- or down-regulated in prostate-derived tumor tissue as compared to healthy tissue. However, use of these genes as alternative or supplemental diagnostically or otherwise clinically useful markers in a commercial setting has not been enabled.

Thus, the problem of the present invention resides in the lack of a reliable method to clearly monitor the presence of a prostate tumor and to perform molecular classification of prostata cancer stages, which allows for an early determination of the potential aggressiveness of the tumor, the respective prognosis, and, thus, an optimal mode of therapy.

The problem is solved by the present invention, which provides
a method for monitoring the presence and/or progression of prostate tumor in an individual, the method comprising:
(a) determining in a sample the expression level of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39, and
(b) comparing the expression level of said at least one marker in a sample from an individual suffering from a prostate disease with the expression level in a sample from a healthy individual,
   wherein an altered expression of said at least one marker as compared to the healthy reference is indicative for the presence of prostate tumor.

According to the present invention, the term "monitoring" encompasses i) diagnosing whether an individual is suffering from a prostate disease, in particular prostate cancer; ii) prognosing and assessing the risk of the disease progression by determining up to which stage the tumor has already progressed; and iii) selecting specified treatments based on the prognosis provided to the patient through determining the expression levels of the markers of the present invention. Thus, the method of the present invention provides, through monitoring of the expression levels suitable means to evaluate which patients may benefit from a more aggressive treatment, and which patients could be spared from unnecessary treatments.

The term "prostate tumor progression" refers to the general classification of prostate tumor stages, which is well-known to the skilled artisan. In brief, the most commonly used staging method is the Tumour, Nodes, Metastasis (TNM) system, which recognises four stages of local tumour growth, from T1 (incidental) to T4 (invasion of neighbouring organs). Each stage describes the state of pathological development of the tumour. T1 represents an 'incidental' state, where the tumour is detected by chance following transurethral resection or by biopsy following PSA testing. At this stage, the tumour will be undetectable by palpation (DRE) or ultrasonography, but may be diagnosed by the method of the present invention. T4 represents advanced disease, where the tumour has invaded neighbouring organs. The nodal stage (N0-N1) and the metastatic stage (M0-M1C) reflect the clinical spread of the disease to lymph nodes and distant sites (metastasis), respectively. Grading systems can assess the degree of cell anaplasia (variation in size, shape and staining properties) and differentiation (how well differentiated the cells are) in the tumour. The Gleason grading system is based on the extent to which the tumour cells are arranged into recognisably glandular structures and the level of cell differentiation. The Gleason system identifies more than five levels of increasing disease aggressiveness, with Grade 1 being the least aggressive and over Grade 5 being the most aggressive cancer.

According to the present invention, a "sample" means any biological material containing genetic information in the form of nucleic acids or proteins obtainable or obtained from an individual. The sample includes e.g. tissue samples, cell samples, bone marrow and/or body fluids such as blood, serum, urin, saliva, semen. Preferably, the sample is tissue or cell samples. The person skilled in the art is aware of methods, how to isolate nucleic acids and proteins from a sample. A general method for isolating and preparing nucleic acids from a sample is outlined in Example 1.

According to the present invention, the term "expression" refers to the process by which mRNA or a polypeptide is produced based on the nucleic acid sequence of a gene, i.e. "expression" also includes the formation of mRNA upon transcription. In accordance with the present invention, the term "determining the expression level" preferably refers to the determination of the level of expression, namely of the markers.

Generally, "marker" refers to any genetically controlled difference which can be used in the genetic analysis of a test versus a control sample, for the purpose of assigning the sample to a defined genotype or phenotype. As used herein, "markers" refer to genes which are differentially expressed in, e.g., cancer patients with highly progressed prostate tumor stages accompanied with bad survival prognosis. The markers can be defined by their gene symbol name, their encoded protein name, their transcript identification number (cluster identification number), the data base accession number, public accession number or GenBank (NCBI, National Center for Biotechnology Information, http://www.ncbi.nlm.nih.gov) identifier or chromosomal location, UniGene accession number and cluster type, LocusLink accession number (see Examples and Tables).

Generally, the expression level of a marker is determined by the determining the expression of its corresponding "polynucleotide" or "polypeptide"as described hereinafter.

According to the present invention, the term "polynucleotide" refers, generally, to a DNA, in particular cDNA, or RNA, in particular a cRNA, or a portion thereof or a polypeptide or a portion thereof. In the case of RNA (or cDNA), the polynucleotide is formed upon transcription of a nucleotide sequence which is capable of expression. The polynucleotide fragments refer to fragments preferably of between at least 8, such as 10, 12, 15 or 18 nucleotides and at least 50, such as 60, 80, 100, 200 or 300 nucleotides in length, or a complementary sequence thereto, representing a consecutive stretch of nucleotides of a gene, cDNA or mRNA. In other terms, polynucleotides include also any fragment (or complementary sequence thereto) of a sequence derived from any of the markers defined above as long as these fragments unambiguously identify the marker.

The determination of the expression level may be effected at the transcriptional or translational level, i.e. at the level of mRNA or at the protein level. Protein fragments such as peptides or "polypeptides" advantageously comprise between at least 6 and at least 25, such as 30, 40, 80, 100 or 200 consecutive amino acids representative of the corresponding full length protein. Six amino acids are generally recognized as the lowest peptidic stretch giving rise to a linear epitope recognized by an antibody, fragment or derivative thereof. Alternatively, the proteins or fragments thereof may be analysed using nucleic acid molecules specifically binding to three-dimensional structures (aptamers).

Depending on the nature of the marker, i.e. whether it is in form of a polynucleotide or polypeptide, the determination of the expression levels may be effected by a variety of methods. For determining and detecting the expression level, it is preferred in the present invention that the marker is labelled.

The labelling of the marker, i.e. the polynucleotide or its corresponding polypeptide can occur by a variety of methods known to the skilled artisan. The label can be fluorescent, chemiluminescent, bioluminescent, radioactive (such as ³H or ³²P). The labelling compound can be any labelling compound being suitable for the labelling of polynucleotides and/or polypeptides. Examples include fluorescent dyes, such as fluorescein, dichlorofluorescein, hexachlorofluorescein, BODIPY variants, ROX, tetramethylrhodamin, rhodamin X, Cyanine-2, Cyanine-3, Cyanine-5, Cyanine-7, IRD40, FluorX, Oregon Green, Alexa variants (available e.g. from Molecular Probes or Amersham Biosciences) and the like, biotin or biotinylated nucleotides, digoxigenin, radioisotopes, antibodies, enzymes and receptors. Depending on the type of labelling, the detection is done via fluorescence measurements, conjugation to streptavidin and/or avidin, antigen-antibody- and/or antibody-antibody-interactions, radioactivity measurements, as well as catalytic and/or receptor/ligand interactions. Suitable methods include the direct labelling (incorporation) method, the amino-modified (amino-allyl) nucleotide method (available e.g. from Ambion), and the primer tagging method (DNA dendrimer labelling, as kit available e.g. from Genisphere). Particularly preferred for the present invention is the use of biotin or biotinylated nucleotides for labelling, with the latter being directly incorporated into, e.g. the cRNA polynucleotide by in vitro transcription.

If the polynucleotide is mRNA, cDNA may be prepared into which a detectable label, as exemplified above, is incorporated. Said detectably labelled cDNA, in single-stranded form, may then be hybridised, preferably under stringent or highly stringent conditions to a panel of single-stranded oligonucleotides representing different genes and affixed to a solid support such as a chip. Upon applying appropriate washing steps, those cDNAs will be detected or quantitatively detected that have a counterpart in the oligonucleotide panel. Various advantageous embodiments of this general method are feasible. For example, the mRNA or the cDNA may be amplified e.g. by polymerase chain reaction, wherein it is preferable, for quantitative assessments, that the number of amplified copies corresponds relative to further amplified mRNAs or cDNAs to the number of mRNAs originally present in the cell. In a preferred embodiment of the present invention, the cDNAs are transcribed into cRNAs prior to the hybridisation step wherein only in the transcription step a label is incorporated into the nucleic acid and wherein the cRNA is employed for hybridisation. Alternatively, the label may be attached subsequent to the transcription step.

Similarly, proteins from a cell or tissue under investigation may be contacted with a panel of aptamers or of antibodies or fragments or derivatives thereof. The antibodies etc. may be affixed to a solid support such as a chip. Binding of proteins indicative of disease or non-disease may be verified by binding to a detectably labelled secondary antibody or aptamer. For the labelling of antibodies, it is referred to Harlow and Lane, "Antibodies, a laboratory manual", CSH Press, 1988, Cold Spring Harbor. Specifically, a minimum set of proteins necessary for monitoring the presence and/or progression of prostate tumor may be selected for creation of a protein array system to make diagnosis on a protein lysate of a diagnostic tissue sample directly. Protein Array Systems for the detection of specific protein expression profiles already are available (for example: Bio-Plex, BIORAD, München, Germany). For this application preferably antibodies against the proteins have to be produced and immobilized on a platform e.g. glasslides or microtiterplates. The immobilized antibodies can be labelled with a reactant specific for the certain target proteins as discussed above. The reactants can include enzyme substrates, DNA, receptors, antigens or antibodies to create for example a capture sandwich immunoassay.

For reliably monitoring the presence and/or progression of prostate tumor it is useful that the expression of more than one of the above defined markers is determined. As a criterion for the choice of markers, the statistical significance of markers as expressed in q or p values based on the concept of the false discovery rate is determined. In doing so, a measure of statistical significance called the q value is associated with each tested feature. The q value is similar to the p value, except it is a measure of significance in terms of the false discovery rate rather than the false positive rate (Storey JD and Tibshirani R. Proc.Natl.Acad.Sci., 2003, Vol. 100:9440-5.

Of the above defined markers, the expression level of at least two, preferably of at least ten, more preferably of at least 25, most preferably of 39 of the markers in Table 1 is determined.

In another preferred embodiment, the expression level of at least 2, of at least 5, of at least 10 out of the markers having the SEQ ID NOs: 1 - 12, 1-20, 1-39, of Table 1 are measured.

The level of the expression of the "marker", i.e. the expression of the polynucleotide is indicative for the presence of prostate tumor of a cell or an organism. The level of expression of a marker or group of markers is measured and is compared with the level of expression of the same marker or the same group of markers from other cells or samples. The comparison may be effected in an actual experiment or in silico. When the expression level also referred to as expression pattern or expression signature (expression profile) is measurably different, there is according to the invention a meaningful difference in the level of expression. Preferably the difference at least is 5 %, 10% or 20%, more preferred at least 50% or may even be as high as 75% or 100%. More preferred the difference in the level of expression is at least 200%, i.e. two fold, at least 500%, i.e. five fold, or at least 1000%, i.e. 10 fold.

Accordingly, the expression level of markers expressed lower in a disease sample than in a healthy, normal sample is at least 5 %, 10% or 20%, more preferred at least 50% or may even be 75% or 100%, i.e. 2-fold lower, preferably at least 10-fold, more preferably at least 50-fold, and most preferably at least 100-fold lower in the disease sample. On the other hand, the expression level of markers expressed higher in a disease sample than in a healthy, normal sample is at least 5 %, 10% or 20%, more preferred at least 50% or may even be 75% or 100%, i.e. 2-fold higher, preferably at least 10-fold, more preferably at least 50-fold, and most preferably at least 100-fold higher in the disease sample.

For the method of the present invention it is preferred if the polynucleotide the expression level of which is determined is in form of a transcribed polynucleotide. A particularly preferred transcribed polynucleotide is an mRNA, a cDNA and/or a cRNA, with the latter being preferred. Transcribed polynucleotides are isolated from a sample, reverse transcribed and/or amplified, and labelled, by employing methods well-known the person skilled in the art (see Examples). In a preferred embodiment of the methods according to the invention, the step of determining the expression profile further comprises amplifying the transcribed polynucleotide.

In order to determine the expression level of the transcribed polynucleotide by the method of the present invention, it is preferred that the method comprises hybridizing the transcribed polynucleotide to a complementary polynucleotide, or a portion thereof, under stringent hybridization conditions, as described hereinafter.

The term "hybridizing" means hybridization under conventional hybridization conditions, preferably under stringent conditions as described, for example, in Sambrook, J., et al., in "Molecular Cloning: A Laboratory Manual" (1989), Eds. J. Sambrook, E. F. Fritsch and T. Maniatis, Cold Spring Harbour Laboratory Press, Cold Spring Harbour, NY and the further definitions provided above. Such conditions are, for example, hybridization in 6x SSC, pH 7.0/0.1 % SDS at about 40 to 45°, preferably 42°C for 16-23 hours, followed by a washing step with 2x SSC/0.1% SDS at 30 to 50°C. In order to select the stringency, the salt concentration in the washing step can for example be chosen between 2x down to 0.5x SSC/0.1% SDS at room temperature for low and medium stringency and 0.2x down to 0.05x SSC/0.1 % SDS at 30 to 50°C for high stringency. In addition, the temperature of the washing step can be varied between room temperature, ca. 22°C, for low stringency, and 65°C to 70° C for high stringency. Preferably the washing temperature is 36°C.

Also contemplated are polynucleotides that hybridize at lower stringency hybridization conditions. Changes in the stringency of hybridization and signal detection are primarily accomplished through the manipulation, preferably of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M NaH2PO4; 0.02M EDTA, pH 7.4), 0.5% SDS, 30% formamide, 100 mg/ml salmon sperm blocking DNA, followed by washes at 50°C with 1 X SSPE, 0.1% SDS. In addition, to achieve even lower stringency, washes performed following stringent hybridization can be done at higher salt concentrations (e.g. 5x SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

"Complementary" and "complementarity", respectively, can be described by the percentage, i.e. proportion, of nucleotides which can form base pairs between two polynucleotide strands or within a specific region or domain of the two strands. Generally, complementary nucleotides are, according to the base pairing rules, adenine and thymine (or adenine and uracil), and cytosine and guanine. Complementarity may be partial, in which only some of the nucleic acids' bases are matched according to the base pairing rules. Or, there may be a complete or total complementarity between the nucleic acids. The degree of complementarity between nucleic acid strands has effects on the efficiency and strength of hybridization between nucleic acid strands.

Two nucleic acid strands are considered to be 100% complementary to each other over a defined length if in a defined region all adenines of a first strand can pair with a thymine (or an uracil) of a second strand, all guanines of a first strand can pair with a cytosine of a second strand, all thymine (or uracils) of a first strand can pair with an adenine of a second strand, and all cytosines of a first strand can pair with a guanine of a second strand, and vice versa. According to the present invention, the degree of complementarity is determined over a stretch of 20, preferably 25, nucleotides, i.e. a 60% complementarity means that within a region of 20 nucleotides of two nucleic acid strands 12 nucleotides of the first strand can base pair with 12 nucleotides of the second strand according to the above ruling, either as a stretch of 12 contiguous nucleotides or interspersed by non-pairing nucleotides, when the two strands are attached to each other over said region of 20 nucleotides. The degree of complementarity can range from at least about 50% to full, i.e. 100% complementarity. Two single nucleic acid strands are said to be "substantially complementary" when they are at least about 80% complementary, preferably about 90% or higher. For carrying out the method of the present invention substantial complementarity is preferred.

Preferred methods for detection and quantification of the amount of polynucleotides, i.e. for the methods according to the invention allowing the determination of the level of expression of a marker, are those described by Sambrook et al. (1989) or real time methods known in the art as the TaqMan® method disclosed in WO92/02638 and the corresponding U.S. 5,210,015, U.S. 5,804,375, U.S. 5,487,972. This method exploits the exonuclease activity of a polymerase to generate a signal. In detail, the (at least one) target nucleic acid component is detected by a process comprising contacting the sample with an oligonucleotide containing a sequence complementary to a region of the target nucleic acid component and a labeled oligonucleotide containing a sequence complementary to a second region of the same target nucleic acid component sequence strand, but not including the nucleic acid sequence defined by the first oligonucleotide, to create a mixture of duplexes during hybridization conditions, wherein the duplexes comprise the target nucleic acid annealed to the first oligonucleotide and to the labeled oligonucleotide such that the 3'-end of the first oligonucleotide is adjacent to the 5'-end of the labeled oligonucleotide. Then this mixture is treated with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments. The signal generated by the hydrolysis of the labeled oligonucleotide is detected and/ or measured. TaqMan® technology eliminates the need for a solid phase bound reaction complex to be formed and made detectable. Other methods include e.g. fluorescence resonance energy transfer between two adjacenly hybridized probes as used in the LightCycler® format described in U.S. 6,174,670.

A preferred protocol if the marker, i.e. the polynucleotide, is in form of a transcribed nucleotide, is described in Examples 1 and 2, where total RNA is isolated, cDNA is synthesized and a fluorescent dye is incorporated during the reverse transcription reaction. The purified cDNA is applied to high-density cDNA arrays. The hybridized cDNA is detected according to the methods described in Example 2. The arrays can be produced by photolithography or other methods known to experts skilled in the art e.g. from U.S. 5,445,934, U.S. 5,744,305, U.S. 5,700,637, U.S. 5,945,334 and EP 0 619 321 or EP 0 373 203, or as decribed hereinafter in greater detail.

In another embodiment of the present invention, the marker is in form of a polypeptide. In another preferred embodiment, the expression level of the polynucleotides or polypeptides is detected using a compound which specifically binds to the polynucleotide of the polypeptide of the present invention.

As used herein, "specifically binding" means that the compound is capable of discriminating between two or more polynucleotides or polypeptides, i.e. it binds to the desired polynucleotide or polypeptide, but essentially does not bind unspecifically to a different polynucleotide or polypeptide.

The compound can be an antibody, or a fragment thereof, an enzyme, a so-called small molecule compound, a protein-scaffold, preferably an anticalin. In a preferred embodiment, the compound specifically binding to the polynucleotide or polypeptide is an antibody, or a fragment thereof.

As used herein, an "antibody" comprises monoclonal antibodies as first described by Köhler and Milstein in Nature 278 (1975), 495-497 as well as polyclonal antibodies, i.e. entibodies contained in a polyclonal antiserum. Monoclonal antibodies include those produced by transgenic mice. Fragments of antibodies include F(ab')₂, Fab and Fv fragments. Derivatives of antibodies include scFvs, chimeric and humanized antibodies. See, for example Harlow and Lane, loc. cit. For the detection of polypeptides using antibodies or fragments thereof, the person skilled in the art is aware of a variety of methods, all of which are included in the present invention. Examples include immunoprecipitation, Western blotting, Enzyme-linked immuno sorbent assay (ELISA), Enzyme-linked immuno sorbent assay (RIA), dissociation-enhanced lanthanide fluoro immuno assay (DELFIA), scintillation proximity assay (SPA). For detection, it is desirable if the antibody is labelled by one of the labelling compounds and methods described supra.

In another preferred embodiment of the present invention, the method for monitoring presence and/or progression of prostate tumor is carried out on an array.

In general, an "array" or "microarray" refers to a linear or two- or three dimensional arrangement of preferably discrete nucleic acid or polypeptide probes which comprises an intentionally created collection of nucleic acid or polypeptide probes of any length spotted onto a substrate/solid support. The person skilled in the art knows a collection of nucleic acids or polypeptide spotted onto a substrate/solid support also under the term "array". As known to the person skilled in the art, a microarray usually refers to a miniaturised array arrangement, with the probes being attached to a density of at least about 10, 20, 50, 100 nucleic acid molecules referring to different or the same genes per cm². Furthermore, where appropriate an array can be referred to as "gene chip". The array itself can have different formats, e.g. libraries of soluble probes or libraries of probes tethered to resin beads, silica chips, or other solid supports.

The process of array fabrication is well-known to the person skilled in the art. In the following, the process for preparing a nucleic acid array is described. Commonly, the process comprises preparing a glass (or other) slide (e.g. chemical treatment of the glass to enhance binding of the nucleic acid probes to the glass surface), obtaining DNA sequences representing genes of a genome of interest, and spotting sequences these sequences of interest onto glass slide. Sequences of interest can be obtained via creating a cDNA library from an mRNA source or by using publicly available databases, such as GeneBank, to annotate the sequence information of custom cDNA libraries or to identify cDNA clones from previously prepared libraries. Generally, it is recommendable to amplify obtained sequences by PCR in order to have sufficient amounts of DNA to print on the array. The liquid containing the amplified probes can be deposited on the array by using a set of microspotting pins. Ideally, the amount deposited should be uniform. The process can further include UV-crosslinking in order to enhance immobilization of the probes on the array.

The array can also be a high density oligonucleotide (oligo) array using a light-directed chemical synthesis process, employing the so-called photolithography technology. Unlike common cDNA arrays, oligo arrays use a single-dye technology. Given the sequence information of the markers, the sequence can be synthesized directly onto the array, thus, bypassing the need for physical intermediates, such as PCR products, required for making cDNA arrays. For this purpose, the marker, or partial sequences thereof, can be represented by 14 to 20 features, preferably by less than 14 features, more preferably less than 10 features, even more preferably by 6 features or less, with each feature being a short sequence of nucleotides (oligonucleotide), which is a perfect match (PM) to a segment of the respective gene. The PM oligonucleotide are paired with mismatch (MM) oligonucleotides which have a single mismatch at the central base of the nucleotide and are used as "controls". The chip exposure sites are defined by masks and are deprotected by the use of light, followed by a chemical coupling step resulting in the synthesis of one nucleotide. The masking, light deprotection, and coupling process can then be repeated to synthesize the next nucleotide, until the nucleotide chain is of the specified length.

Advantageously, the method of the present invention is carried out in a robotics system including robotic plating and a robotic liquid transfer system, e.g. using microfluidics, i.e. channelled structured.

A particular preferred method according to the present invention is as follows:
1. Obtaining a sample, e.g. tissue samples, from a patient having prostate cancer
2. Extracting RNA, preferably mRNA, from the sample
3. Reverse transcribing the RNA into cDNA
4. Hybridizing the cDNA on standard microarrays
5. Determining hybridization

In another embodiment, the present invention is directed to the use of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39, for the manufacturing of a diagnostic for monitoring presence and/or progression of prostate tumor.

The use of the present invention is particularly advantageous for monitoring tumor progression in an individual suffering from a prostate disease. Examples of prostate disease include prostatitis, benign prostatic hyperplasia, localized prostate cancer, hormone naïve metastatic prostate cancer, hormone refractory metastatic prostate cancer, or metastatic small cell prostate cancer.

In a preferred embodiment of the invention, the use is particularly advantageous for monitoring tumor progression in an individual suffering from prostate cancer.

The use of said markers for monitoring presence and/or progression of prostate tumor, preferably based on microarray technology, offers the following advantages: (1) more rapid and more precise diagnosis, (2) easy to use in laboratories without specialized experience, and (3) abolishes the requirement for analyzing viable cells for chromosome analysis.

Accordingly, the present invention refers to a diagnostic kit containing at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39, for monitoring the presence and/or progression of prostate tumor, in combination with suitable auxiliaries. Suitable auxiliaries, as used herein, include buffers, enzymes, labelling compounds, and the like. In a preferred embodiment, the marker contained in the kit is a nucleic acid molecule which is capable of hybridizing to the mRNA corresponding to at least one marker of the present invention. Preferably, the at least one nucleic acid molecule is attached to a solid support, e.g. a polystyrene microtiter dish, nitrocellulose membrane, glass surface or to non-immobilized particles in solution.

In another preferred embodiment, the diagnostic kit contains at least one reference for a disease and/or a healthy, normal sample. As used herein, the reference can be a biological sample or a data base.

In another embodiment, the present invention is directed to an apparatus for monitoring the presence and/or progression of prostate tumor in a sample, the apparatus containing a reference data base obtainable by
compiling a gene expression profile of a sample obtained from at least one healthy individual by determining the expression level of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39.

The values obtained from the determination are saved and maintained in a recallable format, in order to be used for comparison with an unknown sample. In brief, expression values of more than one marker are determined for the sample to be tested, comprising: receiving the gene expression values for more than one marker in the sample to be tested; means for providing a model generated by a supervised learning algorithm based on a dataset of expression values from known samples; comparing the gene expression values of the sample to that of the model, to thereby produce a classification of the sample; and providing an output indication of the classification. Thus, the apparatus can include: a source of expression values of more than one marker in the sample; means for providing a model generated by a trained algorithm based on a dataset of expression values from known biological samples; a processor routine executed by a digital processor, coupled to receive the expression values from the source, the processor routine determining classification of the sample by comparing the expression values of the sample to the model; and an output assembly, coupled to the digital processor, for providing an indication of the classification of the sample

The apparatus of the present invention containing a desired reference data base can be used in a way such that an unknown sample is, first, subjected to gene expression profiling, e.g. by microarray analysis in a manner as described supra or in the art, and the expression level data obtained by the analysis are, second, fed into the apparatus and compared with the data of the reference data base obtainable by the above method. The classifying of the gene expression profile whether it is derived from a disease or healthy individual occurs by means of a machine learning algorithm.

According to the present invention, the "machine learning algorithm" is a computational-based prediction methodology, also known to the person skilled in the art as "classifier", employed for characterizing a gene expression profile. The signals corresponding to a certain expression level which are obtained by the microarray hybridization are subjected to the algorithm in order to classify the expression profile. Supervised learning involves "training" a classifier to recognize the distinctions among classes and then "testing" the accuracy of the classifier on an independent test set. For new, unknown sample the classifier shall predict into which class - disease or healthy - the sample belongs.

Preferably, the machine learning algorithm is selected from the group consisting of Weighted Voting, K-Nearest Neighbors, Decision Tree Induction, Support Vector Machines (SVM) such as polynomial kernel and Gaussian Radial Basis Function-kernel SVM models, and Feed-Forward Neural Networks. Most preferably, the machine learning algorithm is K-Nearest Neighbors.

In a preferred embodiment, the reference data base is backed up on a computational data memory chip which can be inserted in as well as removed from the apparatus of the present invention, e.g. like an interchangeable module, in order to use another data memory chip containing a different reference data base.

The apparatus suitably contains a device for entering the expression level of the data, for example a control panel such as a keyboard. The results, whether and how the data of the unknown sample fit into the reference data base can be made visible on a provided monitor or display screen and, if desired, printed out on an incorporated of connected printer.

Alternatively, the apparatus of the present invention is equipped with particular appliances suitable for detecting and measuring the expression profile data and, subsequently, proceeding with the comparison with the reference data base. In this embodiment, the apparatus of the present invention can contain a gripper arm and/or a tray which takes up the microarray containing the hybridized nucleic acids.

In another embodiment, the present invention refers to a reference data base for monitoring the presence and/or progression of prostate tumor in an individual obtainable by comprising
compiling a gene expression profile of a sample obtained from at least one healthy individual by determining the expression level of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39,.

Preferably, the reference data bank is backed up and/or contained in a computational memory data chip.

The invention is further illustrated and exemplified by the following examples, without limiting the scope of the invention:

### Example 1: Sample preparation

Radical prostatectomy specimens are obtained from at least 20 patients. After removal, the prostate is macroscopically inspected. If a tumor is not visible or palpable, a single cut is made through the mid portion of the lateral surface of the gland from the basal to the apical section. Samples are taken with a 6 mm punch biopsy instrument (Biopsy Punch, Stiefel, Wächtersburg, Germany) from areas that are suspected to contain tumor foci based on information obtained from the preoperative systematic 10-location biopsies. Each individual sample is immediately stored in a kryo-tube filled with 1.5 ml RNAlater (Qiagen, Hilden, Germany). After storage overnight at ambient temperature, the specimens are transferred to a -20°C freezer. In the case of a palpable or visible tumor, the same procedure is performed in the suspected tumor area. Non-cancerous tissue is obtained by the same procedure from areas assumed to be tumor-free. After finishing all tissue-collecting procedures, the sliced surfaces are painted with indelible yellow ink (Wak Chemie, Steinbach, Germany). All materials and solutions are either purchased in RNase-free state or treated to deactivate RNases. Ethanol dilutions are made with RNase-free water. First, in the cryo-tubes, the specimens are thawed at room temperature. To elute most of the RNAlater from the tissue, two subsequent washing steps of 5 min each in 10 ml precooled (0°C) sterile PBS-buffer (PBS Dulbecco's, Invitrogen, Karlsruhe, Germany) are performed in an ice bath. These washings strongly facilitated downstream procedures, e.g. cryo cutting and laser microdissection as well as microscopic visualization, which are often influenced unfavorably by RNAlater. The washed specimens are directly applied to the holder of the cryo-microtome in a drop of Tissue-Tek® (OCT) at the optimal cutting temperature of -25°C. After thorough freezing, cryo sections are prepared and stained with haematoxylin and eosin. These sections are analyzed by a pathologist. If tumor cells are found, sections up to 15 µm thickness are prepared and transferred to P.A.L.M.® membrane slides (P.A.L.M.® Microlaser Technologies AG, Bernried, Germany). The membrane slides had previously been treated in dry heat (4 h at 180°C) to ensure an RNase-free surface. After transfer to the slides, the sections are air-dried for 1 min on ice and then fixed by incubation for 2 min in precooled (-20°C) 75% ethanol. After incubation in the staining solution (1% cresyl violet acetate in pure ethanol) for 20 sec, the slides are briefly dipped in 75% ethanol and then immediately transferred to 100% ethanol. After incubation for 30 sec, the sections are air-dried for about 5 min at room temperature. All ethanol and staining solutions are precooled and kept on ice during the entire procedure. Slides are then further processed immediately or stored at -80°C. To produce samples of the highest uniformity, tissue areas containing only tumor or normal prostate duct cells (1000-3000 µm2) are microdissected and collected employing a UV-laser based P.A.L.M.® MicroBeam system (P.A.L.M. Microlaser Technologies AG) according to the manufacturer's protocols. For RNA analysis the cells are collected in 10 µl of RNA-lysis buffer (RLT). From all tumors with Gleason 4 or 5 fractions, pure Gleason 3 tumor cell collectives are isolated by LMPC.

### Example 2: Extraction, preparation and amplification of nucleic acids

Collected tissue samples are processed applying the RNeasy Micro Kit (Qiagen, Hilden, Germany) according to the manufacturer's protocols. From each sample, one microliter of the final eluted RNA volume (14 µl) is applied to analysis in an Agilent Bioanalyzer microcapillary electrophoresis system (RNA 6000 Pico Kit, Agilent, Waldbronn, Germany). As an initial quality check and for further comparisons with processed tissue, a complete fresh section of each biopsy is directly transferred to lysis buffer and processed as described above before starting the microdissections. From each selected sample, RNA amounts of 20 ng (estimated on the basis of the Bioanalyzer analysis) are reverse transcribed to cDNA in a volume of 20 µl, applying random primers and the 1st strand cDNA synthesis kit for RT-PCR (AMV) (Roche, Mannheim, Germany) according to the manufacturer's protocol. For quantitative PCR-analyses in a LightCycler instrument, 3 µl of the cDNA-reactions described above are used as a template in each reaction with primers specific for human PSA mRNA (prostate specific antigen, KLK3, GenBank: NM_001648, fragment size: 154 bp). For normalization, identical cDNA-amounts are amplified with primers specific for the mRNA of the low abundant reference gene, huHPRT (Hypoxanthine phosphoribosyltransferase, GenBank: M31642, fragment size: 231 bp). Primers can be designed by TIB MOLBIOL (Berlin, Germany) to be mRNA-specific by spanning some introns and/or overlapping exon junctions. Quantification of the PCR-products is performed on the basis of double strand-specific fluorescence (LightCycler Fast Start DNA MasterPLUS SYBR Green I PCR Kit; Roche) and analyzed with LightCycler's software package. Briefly, the crossing points of the related PCR reactions, determined by the 'second derivative maximum' method of the software, are first used to normalize the PSA expression to the HPRT housekeeping gene, then the resulting values are used for pairwise comparison between the corresponding tumor and normal samples from each patient. The microarrays may contain 37,531 PCR-amplified products of human cDNA clones (Human Unigene Set RZPD3.1, German Resource Center for Genome Research, Berlin). PCR products from cDNA clones are purified by isopropanol precipitation, washed in 70% ethanol, and dissolved in 3X SSC/1.5 M betaine. The DNA is spotted on epoxy-coated glass slides (Quantifoil, Jena, Germany) using the ChipWriter Pro (Virtek Vision, Waterloo, Ontario) spotter and SMP3 pins (Telechem, Sunnyvale, CA, USA). After spotting, microarrays are rehydrated, and DNA is denatured with boiling water prior to washing with 0.2% SDS, water ethanol, and isopropanol. The arrays are dried with pressured air. Total RNA (200 ng) is amplified by T7 RNA polymerasebased *in vitro* transcription using the Ambion (Austin, TX) MessageAmp kit. Resultant aRNA is quality-checked with the Agilent 2100 Bioanalyzer. For primer annealing, 2 µg aRNA is mixed with 1 µg random hexamer primer, incubated at 70°C for 10 min and cooled on ice. The labelling reaction is performed in 12.5 µl containing 2.5 µl of 5X RT buffer (Invitrogen), 1.25 µl of 0.1 M DTT, 1 µl each of 5 mM dNTP mix (dGAT), 0.5 µl of 3 mM dCTP, 0.5 µl (20 U) of RNasin, 0.5 µl of 1 mM CY-3- or CY-5-labelled dCTP (Amersham Pharmacia Biotech) and 1 µl (100 U) of Superscript II reverse transcriptase (Invitrogen). The mixture is incubated for 1 h at 42°C, and the reaction is stopped by addition of 1.25 µl of 50 mM EDTA (pH 8.0). The RNA is removed by hydrolysis with 5 µl of 1 M NaOH at 65°C for 10 min, followed by neutralization with 1 µl of 5 M acetic acid. The samples are purified using Microcon YM-30 columns (Millipore, Billerica, MA). The samples are dissolved in 30 µl 1X DIG-Easy hybridization buffer (Roche Diagnostics, Mannheim, Germany), containing 5X Denhardt's solution and 10 ng/µl Cot1-DNA (Invitrogen), heat denatured (65°C, 2 min) and hybridized to the DNA on microarrays in a hybridization chamber (overnight, 37°C). The slides are washed with 1X SSC/0.1% SDS (15 min) and 0.1X SSC/ 0.1% SDS (10 min) and cleaned with 70 and 95% ethanol before drying with pressured air. Arrays are scanned with the GenePix 4000B microarray scanner (Axon Instruments Inc., Union City, CA, USA), and spots are quantified using GenePix Pro 4.1 software (Imaging Research Inc., St. Catharines, Ontario, Canada). Normalization and data analysis are performed with GenePix Pro 4.1 or GeneSpring (Silicon Genetics, Sunnyvale, CA) software. Statistically significant differential gene expression is calculated using "Significance Analysis of Microarrays" (SAM) SAM v1.21 (11).

### Example 3: Genes differentially expressed in normal versus tumor prostate samples

In a specified example, gene expression differences between tumor and normal tissues from ca 34 prostata cancer patients were analyzed In the comparative SAM analysis between tumor and normal tissue, 324 differentially expressed genes were found (q-value: < 0.358). Of the these samples, 3 were Gleason stage 5, 4 were Gleason 6, 23 were Gleason 7, 1 was Gleason 8. Of the 324 genes, 72 were expressed higher in the tumer, and 252 were expressed lower in the tumor than in the normal tissue. Based on these data, 39 selected genes were subjected to low-density arrays and validated with RNA samples of 10 tumor:normal pairs, which have been present in the microarray analysis as well. The genes of the gene signature, presented in table 1, of the present invention were selected pursuant the grade of differential expression.

**Table 1**

| **x-fold Expression (Tumor/Normal tissue; Low Density Arrays)** | **Name** | **Top candidates** | **Regulation in tumor vs normal** | **exact name** | **SEQ ID NO:** | **x-fold of normal control (Microarrays)** | **NCBI RefSeq** |
|---|---|---|---|---|---|---|---|
| 8,669902913 | LOC120224 | UPCA1 | up-regulated | hypothetical protein BC016153 | **1** | 1.45858 | NM_138788 |
| 1,729508197 | LOC116238 | UPCA2 | up-regulated | hypothetical protein BC014072 | **2** | 1.36247 | NM_138463 |
| 2,86 | TM4SF13 | UPCA3 | up-regulated | transmembrane 4 superfamily member 13 | **3** | 1.79628 | NM_014399 |
| 2,465116279 | SH3MD2 | UPCA4 | up-regulated | SH3 multiple domains 2 | **4** | 1.54467 | NM_020870 |
| 2,096153846 | RAP1GA1 | UPCA5 | up-regulated | RAP1, GTPase activating protein 1 | **5** | ? | NM 002885 |
| 1,6875 | RUVBL1 | UPCA6 | up-regulated | RUVB, E.coli, Homolog-like 1 | **6** | 1.41794 | NM_003707 |
| 0,130534351 | WFDC2 | DPCA1 | down-regulated | WAP four-disulfide core domain 2 | **7** | 0.46359 | NM_ 080736, 006103, 080735, 080734, 080733 |
| 0,173333333 | FHL1 | DPCA2 | down-regulated | four and a half LIM domains 1 | **8** | 0.53607 | NM_001449 |
| 0,229166667 | MEIS2 | DPCA3 | down-regulated | Meis1, myeloid ecotropic viral integration site 1 homolog 2 (mouse) | **9** | 0.67360 | NM_ 170674; 170675; 170676; 170677, 172316; 172315;020 149; 002399 |
| 0,231182796 | SMOC1 | DPCA4 | down-regulated | Secreted modular calcium-binding protein | **10** | 0,48798 | NM_ 001034852; 022137 |
| 0,237547893 | FHL2 | DPCA5 | down-regulated | four and a half LIM-domains 2 | **11** | 0.63231 | NM_001450 |
| 0,26519337 | PTGIS | DPCA6 | down-regulated | prostaglandin 12 (prostacyclin ) synthase | **12** | 0.80589 | NM_000961 |
| 0,113402062 | TRIM29 | | down-regulated | tripartite motif-containing 29 | **13** | 0.51847 | NM_012101 |
| 0,190184049 | HSPB8 | | down-regulated | heat shock 22kDa protein 8 | **14** | 0.44397 | NM_014365 |
| 0,254237288 | NTN1 | | down-regulated | netrin 1 | **15** | 0.67086 | NM_004822 |
| 0,268292683 | MAP1 B | | down-regulated | microtubule-associated protein 1B | **16** | 0.55387 | NM_018174 |
| 0,26993865 | MT1 K | | down-regulated | Metallothion ein 1K | **17** | 0,50218 | *156357 |
| 0,272727273 | CLU | | down-regulated | Clusterin | **18** | 0.62007 | NM_001831 ; 203339 |
| 0,276315789 | MT1X | | down-regulated | metallothion ein 1X | **19** | 0.45535 | NM_005952 |
| 0,277777778 | PTRF | | down-regulated | RNA-polymerase I / transcript release factor | **20** | 0,74078 | NM_012232 |
| 0,28 | DPYSL3 | | down-regulated | dihydropyrimidinase-like 3 | **21** | 0.58193 | NM_001387 |
| 0,280487805 | TGFB3 | | down-regulated | transforming growth factor beta 3 | **22** | 0.54370 | NM_003239 |
| 0,303370787 | CHST2 | | down-regulated | carbohydrate (N-acetylglucosamine-6-0) sulfotransferase 2 | **23** | 0.76145 | NM_004267 |
| 0,305555556 | CAV1 | | down-regulated | Caveolin 1 | **24** | 0.64887 | NM_001753 |
| 0,309352518 | LGALS3BP | | down-regulated | lectin, galactoside-binding, soluble, 3 binding protein | **25** | 0.61671 | NM_005567 |
| 0,320441989 | DKK3, RIG | | down-regulated | dickkopf homolog 3 | **26** | 0.66568 | NM_015881 ;013253; 001018057 |
| 0,341708543 | SCARCL1 | | down-regulated | SPARC-like 1 (mast9, hevin) | **27** | 0.48754 | NM_004684 |
| 0,428571429 | MT1 B | | down-regulated | Metallothion ein 1B | **28** | 0.50630 | NM_005947 |
| 0,585858586 | TP531NP2 | | down-regulated | tumor protein p53 inducible nuclear protein 2 | **29** | 0.66975 | NM_021202 |
| n.d. | ACTRT1 | | down-regulated | actin-related protein T1 | **30** | 0.70093 | NM_138289 |
| n.d. | ATP2A2 | | down-regulated | Sarcoplasmic/endoplasmic reticulum calcium ATPase 2 | **31** | 0.69880 | NM_001681 ; 170665 |
| n.d. | MT1H | | down-regulated | Metallothion ein 1H | **32** | 0.60492 | NM_005951 |
| n.d. | MYH11 | | down-regulated | Myosin heavy polypeptide 11 (smooth muscle) | **33** | 0,41368 | NM_001040 114 |
| n.d. | PPP1R12B | | down-regulated | protein phosphatase 1, regulatory (inhibitor) subunit 12B | **34** | 0.61352 | NM_002481 |
| 1,220588235 | TGM3 | | down-regulated | Transglutam inase 3 (E polypeptide, protein-glutamin-glutamyltransferase) | **35** | ? | NM_003245 |
| 1,64 | MAL2 | | up-regulated | mal, T-cell differentiation protein 2 | **36** | 1.57374 | NM_052886 |
| 2,107843137 | GPR160 | | up-regulated | G-protein coupled receptor 160 | **37** | 1.61250 | NM_014373 |
| 3,944827586 | GJB1 | | up-regulated | gap junction protein, beta 1, 32kDa (connexin 32) | **38** | 1.37505 | NM_000166 |
| 12,13128492 | AMACR | | up-regulated | alpha-methylacyl-CoA racemase | **39** | 3.93190 | NM_014324 |

**Table 2 Functional roles of verified genes**

| **Gene** | **Function** | **Gene** | **Function** |
|---|---|---|---|
| **ACTRT1** | **cytoskeleton** | **MT1X** | **metal binding** |
| **SPARCL1** | **Ca binding** | **MAP1 B** | **microtubulus formation** |
| **SMOC1** | **Ca binding** | **CLU** | **multiple** |
| **ATP2A2** | **Ca transport** | **MYH11** | **muscle contraction** |
| **NTN1** | **cell signaling, cell motility** | **PPP1R12B** | **muscle contraction** |
| **GJB1** | **channel** | **TGFB3** | **paracrine signaling** |
| **HSPB8** | **chaperone** | **WFDC2** | **protease inhibitor** |
| **TGM3** | **gamma-glutamyl transferase** | **CAV1** | **protein folding** |
| **GPR160** | **GPCR** | **LGALS3BP** | **receptor** |
| **RAP1GA1** | **GTPase** | **PTRF** | **transcription** |
| **PTGIS** | **isomerase, monooxygenase** | **FHL1** | **transcription factor** |
| **AMACR** | **lipid metabolism** | **FHL2** | **transcription factor** |
| **MAL2** | **membrane protein** | **MEIS2** | **transcription factor** |
| **TM4SF13** | **membrane protein** | **RUVBL1** | **transcription factor** |
| **LOC120224** | **membrane protein ?** | **TRIM29** | **transcription factor** |
| **CHST2** | **metabolism** | **TP53INP2** | **transcription factor ?** |
| **DPYSL3** | **metabolism** | **LOC116238** | **transporter ?** |
| **MT1B** | **metal binding** | **SH3MD2** | **ubiquitin-ligase** |
| **MT1H** | **metal binding** | **DKK3, RIG** | **wnt signaling** |
| **MT1K** | **metal binding** | | |

## Claims

1. A method for monitoring the presence and/or progression of prostate tumor in an individual, the method comprising:
(c) determining in a sample the expression level of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39, and
(d) comparing the expression level of said at least one marker in a sample from an individual suffering from a prostate disease with the expression level in a sample from a healthy individual,
wherein an altered expression of said at least one marker as compared to the healthy reference is indicative for the presence of prostate tumor.

2. The method according to claim 1, wherein the marker is labelled.

3. The method according to claim 1 or 2, wherein the label is a luminescent, preferably a fluorescent label, an enzymatic or a radioactive label.

4. The method according to at least one of the claims 1-3, wherein the expression level of at least 2, preferably of at 10, more preferably of at least 25, most preferably of 39 of the markers is determined.

5. The method according to claim 1, wherein the expression level of at least one marker selected from markers comprising the SEQ ID NO: 1, 2, 3, 4, 5, 6, 7, 8, 9, 10, 11, and/or 12 is determined.

6. The method according to at least one of the claims 1-5, wherein the expression level of markers expressed lower in a disease than in a healthy sample is at least 5 %, 10 % or 20 %, more preferred at least 50 % or may even be 75 % or 100 %, i.e. 2-fold lower, preferably at least 10-fold, more preferably at least 50-fold, and most preferably at least 100-fold lower in the disease sample.

7. The method according to at least one of the claims 1-5, wherein the expression level of markers expressed higher in a disease than in a healthy sample, is at least 5 %, 10 % or 20 %, more preferred at least 50 % or may even be 75 % or 100 %, i.e. 2-fold higher, preferably at least 10-fold, more preferably at least 50-fold and most preferably at least 100-fold higher in the disease sample.

8. The method according to at least one of the claims 1-7, wherein the disease sample is from an individual having prostate cancer.

9. The method according to at least one of the claims 1-8, wherein at least one marker is in the form of a transcribed polynucleotide, or a portion thereof.

10. The method according to claim 9, wherein the transcribed polynucleotide is a mRNA or a cDNA.

11. The method according to claim 9 or 10, wherein the determining of the expression level comprises hybridizing the transcribed polynucleotide to a complementary polynucleotide, or a portion thereof, under stringent hybridization conditions.

12. The method according to at least one of the claims 1-8, wherein at least one marker is in the form of a polypeptide, or a portion thereof.

13. The method according to claim 12, wherein the determining of the expression level comprises contacting the marker with a compound specifically binding to the marker.

14. The method according to claim 13, wherein the compound is an antibody, or a fragment thereof.

15. The method according to at least one of the claims 1-14, wherein the method is carried out on an array.

16. The method according to at least one of the claims 1-15, wherein the method is carried out in a robotics system.

17. The method according to at least one of the claims 1-16, wherein the method is carried out using microfluidics.

18. Use of at least one marker as defined in at least one of the claims 1-3 for the manufacturing of a diagnostic for monitoring the presence and/or progression of prostate tumor in an individual.

19. A diagnostic kit containing at least one marker as defined in at least one of the claims 1-3 for monitoring the presence and/or progression of prostate tumor in an individual, in combination with suitable auxiliaries.

20. The diagnostic kit according to claim 19, wherein the kit contains a reference for a disease and/or a healthy sample.

21. The diagnostic kit according to claim 20, wherein the reference is a biological sample or a database.

22. An apparatus for monitoring the presence and/or progression of prostate tumor in an individual in a sample, the apparatus containing a reference database.

23. The apparatus according to claim 22, wherein the reference database is obtainable by compiling a gene expression profile of a sample obtained from at least one healthy individual by determining in said sample the expression level of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39,.

24. A reference database for monitoring the presence and/or progression of prostate tumor in an individual in a sample obtainable by compiling a gene expression profile of a sample obtained from at least one healthy individual by determining the expression level of at least one marker selected from the markers comprising the sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and/or 39,.

25. The reference database according to claim 24, wherein the reference database is backed up and/or contained in a computational memory chip.
